# EUROPÄISCHE PATENTSCHRIFT

(11) **EP 1 549 750 B1**
(45) Veröffentlichungstag und Bekanntmachung des Hinweises auf die Patenterteilung: **29.04.2015**
(21) Anmeldenummer: 03808718.5
(22) Anmeldetag: 09.10.2003
(51) Int. Cl.: C12N 15/62, A61K 39/00

(54) **MODULÄRE ANTIGEN-TRANSPORTER MOLEKÜLE (MAT-MOLEKÜLE) ZUR MODULIERUNG VON IMMUNREAKTIONEN, ZUGEHÖRIGE KONSTRUKTE, VERFAHREN UND VERWENDUNGEN**
MODULAR ANTIGEN TRANSPORTER MOLECULES (MAT MOLECULES) FOR MODULATING IMMUNE REACTIONS, ASSOCIATED CONSTRUCTS, METHODS AND USES THEREOF
MOLECULES MODULAIRES DE TRANSPORT D'ANTIGENES (MOLECULES MAT) DESTINEES A LA MODULATION DE REACTIONS IMMUNITAIRES, ET CONSTRUCTIONS ASSOCIEES, PROCEDES ET UTILISATIONS CORRESPONDANTS

(30) Priorität: 11.10.2002 EP 02022774
(43) Veröffentlichungstag der Anmeldung: 06.07.2005
(73) Patentinhaber: Boehringer Ingelheim Vetmedica GmbH, 55216 Ingelheim am Rhein (DE)
(72) Erfinder: LAMPING, Norbert, 30175 Hannover (DE); RETO, Crameri, CH-7220 Davos-Platz (CH); FLÜCKIGER, Sabine, CH-6312 Steinhausen (CH); DAIGLE, Isabelle, CH-7270 Davos-Platz (CH)
(74) Vertreter: Kröncke, Rolf
(86) Internationale Anmeldenummer: PCT/EP2003/011190
(87) Internationale Veröffentlichungsnummer: WO 2004/035793

(56) Entgegenhaltungen:
- US-A- 5 633 234
- VAN BERGEN J ET AL: "Get into the groove! Targeting antigens to MHC class II." IMMUNOLOGICAL REVIEWS. DENMARK DEC 1999, Bd. 172, Dezember 1999 (1999-12), Seiten 87-96, XP001145539 ISSN: 0105-2896
- BONINI C ET AL: "Targeting antigen in mature dendritic cells for simultaneous stimulation of CD4+ and CD8+ T cells." JOURNAL OF IMMUNOLOGY (BALTIMORE, MD.: 1950) UNITED STATES 15 APR 2001, Bd. 166, Nr. 8, 15. April 2001 (2001-04-15), Seiten 5250-5257, XP002231368 ISSN: 0022-1767
- THOMSON S A ET AL: "Targeting a polyepitope protein incorporating multiple class II-restricted viral epitopes to the secretory/endocytic pathway facilitates immune recognition by CD4+ cytotoxic T lymphocytes: a novel approach to vaccine design." JOURNAL OF VIROLOGY. UNITED STATES MAR 1998, Bd. 72, Nr. 3, März 1998 (1998-03), Seiten 2246-2252, XP002231369 ISSN: 0022-538X
- RODRIGUEZ F ET AL: "CD4(+) T cells induced by a DNA vaccine: immunological consequences of epitope-specific lysosomal targeting." JOURNAL OF VIROLOGY. UNITED STATES NOV 2001, Bd. 75, Nr. 21, November 2001 (2001-11), Seiten 10421-10430, XP002231370 ISSN: 0022-538X
- SHELDON K ET AL: "Loligomers: design of de novo peptide-based intracellular vehicles." PROCEEDINGS OF THE NATIONAL ACADEMY OF SCIENCES OF THE UNITED STATES OF AMERICA. UNITED STATES 14 MAR 1995, Bd. 92, Nr. 6, 14. März 1995 (1995-03-14), Seiten 2056-2060, XP002231371 ISSN: 0027-8424 in der Anmeldung erwähnt
- FORD K G ET AL: "PROTEIN TRANSDUCTION: AN ALTERNATIVE TO GENETIC INTERVENTION?" GENE THERAPY, MACMILLAN PRESS LTD., BASINGSTOKE, GB, Bd. 8, Nr. 1, Januar 2001 (2001-01), Seiten 1-4, XP001098109 ISSN: 0969-7128

## Beschreibung

### Technisches Gebiet der Erfindung

Die Erfindung befasst sich mit der Stimulierung und der Inhibierung des Immunsystems zur Prophylaxe, Therapie oder Diagnose von Erkrankungen, die mit einem nicht stark genug stimulierten oder mit einem zu stark stimuliertem Immunsystem einhergehen. Zu diesen Erkrankungen zählen unter anderem Infektionserkrankungen, Tumorerkrankungen, Allergien, Autoimmunerkrankungen, Transplantatabstoßungsreaktionen usw. Der Kern der Erfindung ist ein neuartiges Verfahren, bei dem das Immunsystem durch Gabe eines MAT-Moleküls beeinflusst wird, dass aus zumindest den drei folgenden Bestandteilen besteht:
1. Einem Translokations-Modul, das bewirkt, dass das MAT-Molekül von Außen in Zellen eindringen kann,
2. einem intrazellulärem Targeting-Modul, das bewirkt, dass das MAT-Molekül innerhalb der Zelle derart verarbeitet wird, dass es zu einer veränderten Immunantwort bzw. einer veränderten Präsentation des Antigens kommt und
3. einem Antigen-Modul, das die Spezifität der modulierten Immunantwort bestimmt.

Die Kombination dieser drei Elemente zu einem MAT-Molekül ermöglicht die gezielte, spezifische Modulation des Immunsystems des behandelten Individuums, bzw. ermöglicht eine veränderte Präsentation des Antigens durch die antigenpräsentierende Zelle.

### Stand der Technik

### Verarbeitung von Antigenen durch antigenpräsentierende Zellen

Die Verarbeitung von Antigenen durch Antigen Präsentierende Zellen (APC) erfolgt über zwei verschiedene Wege. Intrazellulär auftretende Antigene werden von MHC I (Major Histocompatibility Complex class I, MHC class I) Molekülen auf der Zelloberfläche präsentiert, während extrazelluläre Antigene über MHC II (Major Histocompatibility Complex class II, MHC class II) Moleküle auf der Zelloberfläche präsentiert werden. Beide Mechanismen initiieren eine Immunreaktion des Wirts auf das Antigen. Der Weg, den das Antigen von der Aufnahme in die Zelle bis zur Präsentation auf der Zelloberfläche in Form eines MHC II-Antigen-Komplexes nimmt, verläuft über verschiedene Zellorganellen, unter anderem über das endoplasmatische Retikulum, den Golgi Apparat, das trans-Golgi Netzwerk, Lysosomen, Endosomen und über "MHC class II compartments" (MIIC). Bei der MHC II vermittelten Antigenpräsentation spielen die MIICs eine wichtige Rolle. In diesen Organellen der Zelle werden die MHC II-Moleküle mit niedermolekularen Antigenen oder mit proteolytischen Fragmenten von Proteinen beladen. Bei diesem Vorgang wird die anfangs am MHC II-Molekül gebundene "Invariant Chain" (auch MHC II gamma-Kette oder li genannt) proteolytisch abgebaut und unter der Regulation verschiedener Proteine, die direkt oder indirekt an MHC II binden, das Antigen an das MHC II-Molekül gebunden [1]. Zu diesen Regulatorischen Molekülen zählen unter anderem HLA-DM, HLA-DO, LAMP-1, LAMP-2, CD63, CD83, usw. Die genaue Funktion dieser Proteine ist z.T. bisher ungeklärt, aber viele von ihnen weisen Signalsequenzen auf, die ihren Transport zu den Lysosomen, zu den Endosomen, zum trans-Golgi Netzwerk, zu den MIICs usw. fördern [2-4]. Bei den proteolytischen Reaktionen, die notwendig sind, damit das Antigen auf MHC II Molekülen präsentiert werden kann, spielen eine Reihe von Proteasen eine Rolle. Zu den Proteasen, die in MIICs vorhanden sind, gehören unter anderem verschiedene Mitglieder der Cathepsin-Familie, wie z.B. Cathepsin S und Cathepsin L [1].

### Targeting und Targetingsequenzen

Aminosäuresequenzen, die die Eigenschaft haben, sich innerhalb oder außerhalb einer Zelle gezielt an einen bestimmten Ort oder in einer bestimmten Zellorganelle anzureichern, werden häufig als Targetingsequenzen bezeichnet. Dabei ist hervorzuheben, dass verschiedene Arten von Targeting unterschieden werden können. Insbesondere unterscheiden sich intrazelluläres und extrazelluläres Targeting. Zum extrazellulärem Targeting werden z.B. Antikörper verwendet, die von außen an direkt zugänglichen Strukturen auf der Zelloberfläche, z.B. an den extrazellulären Anteil von Membranproteinen, binden. Ein Antikörper, der ein Protein auf der Zelloberfläche von Tumorzellen bindet, kann z.B. mit einem Zellgift gekoppelt werden. Dieser Antikörper bewirkt dann ein extrazelluläres Targeting des Zellgifts zur Tumorzelle, wodurch diese gezielt abgetötet werden kann. Diese Art von Targeting ist grundsätzlich verschieden von intrazellulärem Targeting, bei dem oft intrazelluläre Membranen überwunden werden müssen oder bei dem die Targetingsequenz von intrazellulären Rezeptoren gebunden wird und so z.B. in bestimmte Zellorganellen eindringt, oder bei dem die Targetingsequenz durch spezielle Kanäle, ausgebildet von speziellen Proteinen, in das Zellorganell eindringen kann, für das die Targetingsequenz spezifisch ist. Wird nachfolgend in der vorliegenden Patentanmeldung von einer Targetingsequenz oder von einem Targeting-Modul gesprochen, so ist damit grundsätzlich immer intrazelluläres Targeting und nicht extrazelluläres Targeting gemeint. Ausserdem ist in der vorliegenden Patentanmeldung, mit Targeting nicht jede Art von intrazellulärem Targeting gemeint, sondern nur intrazelluläres Targeting, das dazu dient, Moleküle zu Organellen oder intrazellulären Regionen zu transportieren, die an der Verarbeitung, Prozessierung und/oder Bindung von Antigenen an MHC-Moleküle beteiligt sind. Solche Organellen oder intrazellulären Regionen sind z. B. das endoplasmatische Retikulum, der Golgi Apparat, das trans-Golgi Netzwerk, Endosomen, Lysosomen und MIICs. Targetingsequenzen, abstammend von verschiedenen Proteinen sind in der Literatur beschrieben [1- 5].

### Translokation und Translokationssequenzen

Weiterhin sind aus der Literatur zahlreiche Aminosäuresequenzen, insbesondere abstammend von Viren, z. B. HIV-tat oder das vom Herpes Simplex Virus stammende Protein VP22 bekannt, die den Transport von Proteinen, Peptiden und anderen Substanzklassen, wie z. B. Nukleinsäuren oder pharmazeutisch wirksamen Substanzen, ins Zellinnere fördern. Dazu werden diese sogenannten Translokationssequenzen an die zu transportierenden Moleküle (auch Cargo-Moleküle genannt) entweder über kovalente oder über nicht kovalente Bindungen gebunden. Die resultierenden Verbindungen aus Translokationssequenz und Cargo-Molekül können dann extrazellulär zu Zellen hinzu gegeben werden. Die Translokationssequenz bewirkt dann, dass das Cargo-Molekül ins Zellinnere gelangt. Dieses Prinzip ist ebenfalls in zahlreichen Arbeiten, insbesondere für die HIV-Tat-Sequenz, beschrieben [6-8].

Obwohl es bereits möglich und vorgesehen war, als Cargo-Molekül ein Antigen zu verwenden, war es mit Hilfe der bekannten Translokationsmittel allein noch nicht möglich, eine Immunantwort in einem Individuum so gezielt und dosiert zu erzeugen, wie dies für bestimmte Aufgaben-beispielsweise die Allergie-Desensibilisierungwünschenswert erscheint. Es besteht daher ein Bedarf für weitere Verfahren zur gezielten Immunmodulation.

Von Bergen J. et. al., Immunol. Rev., 1999, 172, 87-96, fasst das Wissen auf dem Gebiet des Targetings von Antigenen bis zum Jahr 1999 zusammen. Danach werden zum Targeting der Antigene zu den MHC II-Molekülen zwei unterschiedliche Strategien angewendet, die Verwendung von DANN-Konstrukten und die gezielte rezeptorvermittelte Aufnahme von Antigenen durch APC, die entsprechende Rezeptoren exprimieren.

Sheldon K. et.al., PNAS, 1995, 92(6), 2056-2060, betrifft die Verwendung von Loligomeren. Die dort beschriebenen Moleküle erlauben einen Import bestimmter Sequenzen aus dem extrazellulären Bereich in dem Zellkern.

### Aufgabe der Erfindung

Es ist daher die Aufgabe der Erfindung, Mittel zur Verfügung zu stellen, um verschiedenste und beliebige Antigene so gezielt ihrem Prozessierungsort zuzuführen, dass dies therapeutisch und diagnostisch noch besser nutzbar ist.

### Lösung der Aufgabe

Die Erfindung stellt die Verwendung von modularen Antigen-Transport-Molekülen zur Verfügung, die es ermöglichen, Antigene sehr gezielt Zellen zuzuführen, um eine effiziente, spezifische Immunreaktion zu erzielen. Diese Moleküle ermöglichen es erstens, Antigene effizient vom extrazellulären Raum in den intrazellulären Raum der Zelle zu befördern und ermöglichen es zweitens, dass die Antigene, wenn sie im Inneren der Zelle angekommen sind, effizient zu den Zellorganellen gelangen, in denen sie für die Antigenpräsentation weiter verarbeitet werden. Dieser zweistufige Prozess kann ganz allgemein zur gezielten, effizienten Modulation der Immunreaktion eines Individuums genutzt werden.

Als Werkzeug zum Erzielen dieser Wirkungen wurden spezielle Moleküle entwickelt, die nachfolgend in dieser Patentanmeldung als "modulare Antigen-Transporter"-Moleküle oder MAT-Moleküle bezeichnet werden. Diese MAT- Moleküle, zugehörige Nukleinsäuren, Vektoren, Zellen, Zelllinien, Vesikel, Immunglobuline sowie zur Erfindung gehörige Verwendungen und Verfahren, die sich auf diese Bestandteile beziehen oder mit ihnen arbeiten, sind in den Ansprüchen näher gekennzeichnet.

Für die Lösung der Aufgabe ist eine bisher nie beschriebene Kombination von zumindest drei Modulen zu einer neuartigen Klasse von Molekülen, die als MAT-Moleküle (moduläre Antigen-Transport-Moleküle) bezeichnet werden, vorgesehen. Diese drei Module umfassen zumindest ein Translokations-Modul, zumindest ein Targeting-Modul und zumindest ein Antigen-Modul. Die drei verschiedenen Module werden über kovalente oder nicht kovalente Bindungen aneinander gekoppelt. Das so hergestellte MAT-Molekül kann direkt einem Individuum, dessen Immunreaktion gegen die in dem MAT-Molekül enthaltenen Antigene beeinflusst werden soll, verabreicht werden. Alternativ können auch in vitro Zellen mit MAT-Molekülen behandelt werden und die so behandelten Zellen anschließend dem Individuum verabreicht werden. Bei diesem Verfahren bewirken die Translokations-Module, dass das MAT-Molekül in die Zelle eindringen kann, die Targeting-Module bewirken, dass das MAT-Molekül intrazellulär derart verarbeitet wird, dass es zu einer Immunantwort kommt und die Identität der Antigene in den Antigen-Modulen bestimmt, gegen welches Antigen die Immunreaktion gerichtet ist. Ein wesentlicher Vorteil dieses neuen Verfahrens zur Modulation der Immunantwort eines Individuums ist vor allem seine universelle Anwendbarkeit, d.h. es kann mit verschiedensten Antigenen, verschiedenen Translokations-Modulen und verschiedenen Targeting-Modulen gearbeitet werden. Außerdem hat die Verwendung eines Translokations-Moduls zur Folge, dass das Verfahren nicht gewebe- oder zellspezifisch ist, sondern universell zur Immunmodulation nahezu aller Arten von Zellen geeignet ist. Ein weiterer Vorteil ist der modulare Aufbau des MAT-Moleküls. Der modulare Aufbau erlaubt es, das MAT-Molekül schnell an die jeweiligen medizinischen Erfordernisse anzupassen. Auch die genaue Anordnung der drei Bestandteile des MAT-Moleküls und die Art ihrer Verknüpfung miteinander kann variiert werden, solange zumindest je ein Modul aller drei Arten der Module in dem MAT-Molekül vorhanden ist. Bezüglich der Antigene gibt es aufgrund des Verfahrens keine Einschränkungen. Das Verfahren kann z.B. zur Aktivierung des Immunsystems eines Individuums gegen Krankheitserreger, wie z.B. gegen Viren, Bakterien, Parasiten usw., d.h. ganz allgemein als Impfstoff verwendet werden. Außerdem kann das Verfahren zur Aktivierung des Immunsystems gegen entartete Zellen, wie z.B. Tumorzellen, usw., verwendet werden. Es kann aber auf der anderen Seite auch zu Desensibilisierung des Immunsystems eines Individuums gegen Allergene wie z.B. Pollen, Tierhaare, Hausstaubmilben, Insektengifte, usw. oder zur gezielten Unterdrückung des Immunsystems z.B. beim Vorliegen von Autoimmunreaktionen wie z.B. Arthritis, Rheuma, Diabetes, SLE ("systemic lupus erythematosus"), usw. und zur Unterdrückung von TransplantatAbstoßungsreaktionen, verwendet werden. Weitere nicht ausdrücklich genannte Erkrankungen, die mit einer zu starken oder einer zu schwachen Immunreaktion einhergehen, können ebenfalls mit den erfindungsgemäßen MAT-Molekülen behandelt werden.

Im Stand der Technik wurde es bisher häufig als sehr nachteilig angesehen, dass Translokationssequenzen nicht spezifisch für bestimmte Zellarten sind, sondern bei allen Arten von Zellen gleichermaßen wirksam sind [9]. Die universelle Funktionalität von Translokations-Modulen ist in der vorliegenden Erfindung jedoch ein großer Vorteil, der im bisherigen Stand der Technik nicht erkannt wurde. Im bisherigen Stand der Technik wurde oftmals angestrebt, "Cargo-Moleküle", die unter Zuhilfenahme von Translokationssequenzen in Zellen eingeschleust werden, vor proteolytischen Abbau in der Zelle zu schützen [9]. In der vorliegenden Erfindung ist genau das Gegenteil ausdrücklich gewünscht und vorteilhaft für die Wirkungsweise der Erfindung. Ein proteolytischer Abbau der Antigen-Module in der Zelle ist für die Wirkung der Erfindung, d.h. für eine effiziente Antigenpräsentation, von Vorteil. Daher werden in der vorliegenden Erfindung Targeting-Module eingesetzt, die gezielt den Transport der Antigen-Module in Zellkompartimente fördern, wo sie proteolytisch abgebaut werden.

Die bekanntesten Aminosäuresequenzen, die als Translokations-Modul im Sinne der Erfindung verwendet werden können, sind die HIV-Tat und die VP22 Sequenz. Für diese Sequenzen ist beschrieben, dass sie sowohl eine Translokation durch die Zellmembran bewirken, als auch einen Transport in den Zellkern [7]. Dieser Transport in den Zellkern ist im Zusammenhang mit der Vorliegenden Erfindung unerwünscht, da im Zellkern keine Antigenprozessierung stattfindet und es somit nicht zu einer effizienten Antigenpräsentation kommt. Dieses bisher ungelöste Problem wird mit der vorliegenden Erfindung durch die Verwendung eines Targeting-Moduls gelöst, dass bewirkt, dass das MAT-Molekül intrazellulär nicht in den Zellkern transportiert wird, sondern gezielt in diejenigen Organellen transportiert wird, in denen Antigenprozessierung oder die Beladung von MHC-Molekülen mit Antigen stattfinden. Das intrazelluläre Translokations-Modul der erfindungsgemäßen MAT-Moleküle beseitigt daher einen wesentlichen Nachteil, den bisher im Stand der Technik bekannte Tat-Antigen Fusionsproteine aufweisen.

Im bisherigen Stand der Technik sind auch Fusionsproteine, bestehend aus einer Targetingsequenz, z.B. der Invarianten Kette des MHC II Moleküls, und einem Antigen bekannt. Diese Fusionsproteine sind jedoch nicht in der Lage, effizient in Antigen-präsentierende Zellen ein zu dringen. Dazu ist, bei ihrer Verwendung zur Immunisierung eines Individuums, zusätzlich ein Adjuvans notwendig, dass die Aufnahme des Fusionsproteins in die Zelle fördert. Diese Adjuvantien, wie z.B. Mineralöl, Mycobakterienextrakte oder Freund'sches Adjuvans haben jedoch, unerwünschte Nebenwirkungen, wie z.B. lokale Entzündungsreaktionen. Die in der nun vorliegenden Erfindung verwendeten MAT-Moleküle haben gegenüber herkömmlichen Impfstoffen den Vorteil, dass sie direkt mit einem physiologisch sehr verträglichen Translokations-Modul gekoppelt sind, dass die Aufnahme in Zellen sehr wirksam fördert. Dadurch kann unter Umständen auf zusätzliche Adjuvantien ganz oder teilweise verzichtet werden. Dieses hat zur Folge, dass bei Immunisierungen mit MAT-Molekülen deutlich weniger unerwünschte Nebenwirkungen auftreten werden.

Es ist seit langem bekannt, dass extra- oder intrazelluläre MHC I präsentierte Antigene zu einer zytoxischen Immunantwort führen, nicht jedoch zu einer starken projektiven humoralen Immunantwort. Durch die Verwendung von MAT-Molekülen können die in dem Antigen-Modul enthaltenen Antigene jedoch extrazellulär zugegeben werden, wirken aber wie intrazelluläre Antigene, da das Translokations-Modul das Antigen in den intrazellulären Raum transportiert und das Targeting-Modul intrazellulär den Transport des Antigen derart beeinflusst, dass es zu einer humoralen Immunantwort kommt. Durch dieses neuartige Verfahren lässt sich die seit vielen Jahren angestrebte starke Induktion einer humoralen Immunantwort mit extrazellulär zugegebenen Antigenen (MAT-Molekülen) erreichen.

In dem Überblicksartikel von van Bergen, J., et al, 1999, Immunological Reviews, 172, 87-96 werden verschiedene Strategien zum Targeting von Antigenen in MHC Klasse II Moleküle beschrieben. Diese Strategien beruhen einerseits auf rezeptorvermittelte Endozytose der Antigene. Andererseits werden molekularbiologische Möglichkeiten zum endogenen Targeting der Antigene diskutiert. In dem Artikel von Sheldon, K., et al, PNAS, 1995, 92, 2056-2060, werden neue Moleküle, die als Loligomere bezeichnet werden beschrieben. Hierbei handelt es sich um Moleküle, die Translokationssignale fusioniert mit einem Markermolekül, beschreiben. Diese Konstrukte werden als Möglichkeit angesehen, gezieltes interzelluläres Targeting zu ermöglichen.

Dazu werden im Gegensatz zum bisherigen Stand der Technik, in dieser Erfindung zwei an sich bekannte Mechanismen in neuartiger Weise kombiniert, so dass es zu einer wesentlich verbesserten Immunisierung mit Antigenen kommt. Bei diesen Mechanismen handelt es sich in beiden Fällen um gezielte Antigen-Transportmechanismen, die in der Summe zu einer sehr effizienten Immunantwort führen. Jeder dieser beiden Transportmechanismen wird durch ein definiertes Modul des MAT-Moleküls bewirkt. Dabei handelt es sich um:
1. Transport des Antigens vom extrazellulären Raum in den intrazellulären Raum (Translokations-Modul)
   in Kombination mit
2. Transport des Antigens innerhalb der Zelle spezifisch zu den Organzellen, die für die Antigenprozessierung zuständig sind (Targeting-Modul).

Dies führt zu dem Ergebnis: sehr effiziente Immunisierung, nur IgG, kein IgE.

Diese neuartige Kombination von 2 bekannten Transportmechanismen führt dazu, das z. B. mit viel geringeren Antigen-Konzentrationen immunisiert werden kann und hat außerdem den sehr großen und überraschenden Vorteil, dass vorwiegend eine Immunantwort vom Th1-Typus auftritt, d. h. eine Immunantwort in Form von IgG-Antikörpern und nicht in Form von Allergien verursachenden IgE-Antikörpern.

### Beschreibung der Erfindung im Einzelnen

### Translokationssequenzen / Translokations-Module

Die Begriffe Translokationssequenz und Translokations-Modul werden im Text der vorliegenden Anmeldung gleichwertig und mit gleicher Bedeutung nebeneinander verwendet. Der Begriff Translokations-Modul ist eingeführt worden, um klar zu stellen, dass Translokations-Module nur ein Teil eines MAT-Moleküls im Sinne der Erfindung sind. Außerdem stellen Translokations-Module nicht nur in der Natur vorkommende Translokations-Peptidsequenzen wie z.B. HIV-tat dar, sondern auch z.B. Peptidomimetics oder andere Strukturen, die die gleiche Funktion wie die in der Natur vorkommende Translokations-Peptidsequenzen übernehmen können.

Die Erfindung umfasst die Verwendung verschiedener Translokations-Module zur Herstellung von MAT-Molekülen, die zumindest aus einem Translokations-Modul, zumindest einem Targeting-Modul und zumindest einem Antigen-Modul bestehen. Generell sind alle derzeit bekannten und zukünftig bekannt werdenden Translokationssequenzen geeignet, im Sinne der vorliegenden Erfindung verwendet zu werden. In der Literatur sind zahlreiche geeignete Translokationssequenzen beschrieben. Zu diesen Translokationssequenzen gehören virale Sequenzen, Homeoprotein-Sequenzen, "Leucine Zipper"-Sequenzen, arginin- und lysin-reiche Sequenzen sowie verschiedene andere Sequenzen von Proteinen, die trotz fehlender Sekretions-Signalsequenz sezerniert werden, usw.

### Virale Peptidsequenzen, die als Translokations-Module geeignet sind

Zu den im Rahmen dieser Erfindung als Translokations-Module geeigneten Peptidsequenzen zählen unter anderem virale Proteine oder Teilsequenzen von viralen Proteinen wie z.B. das Protein "HIV transcriptional activator protein" (HIV-tat). Zu den geeigneten Tat-Proteinen zählen neben dem Tat-Protein des HIV-1 Virus auch die Tat-Proteine von anderen Lentiviren [9]. Zahlreiche modifizierte Tat-Peptide sind als Sequenzen beschrieben worden, die Translokation bewirken können. Darunter befinden sich Tat-Peptide, die nur Teilsequenzen des Tat-Proteins darstellen [10], Tat-Peptide, die Punktmutationen enthalten [10], Tat-Peptide, die in umgekehrter Sequenzreihenfolge (invers) vorliegen [10], oder Tat-Peptide, die ungewöhnliche Aminosäuren, wie z.B. D-Isomere von Aminosäuren enthalten [10], usw. Alle diese Variationen von Peptidsequenzen sind daher generell als Translokations-Module geeignet. Weiterhin sind im Sinne der vorliegenden Erfindung Peptide als Translokations-Module geeignet, die von anderen Viren stammen, wie z.B. VP22 (Herpes Simplex Virus-1 VP22 Tegument Protein) [9]. Es sind derzeit auch kommerzielle Expressionsvektoren erhältlich, die eine VP22-Sequenz enthalten, die zur Translokation geeignet ist. Diese Expressionsvektoren erlauben daher die Herstellung von VP22-Fusionsproteinen (Voyager™-VP22-System, Invitrogen, Breda, The Netherlands). Bei Verwendung dieses Expressionssystems ist jedoch kein Targeting-Modul in dem Fusionsprotein enthalten. Auch andere Viren, z.B. dem "Marek's Disease Virus-1", ein Virus, das Lymphoma bei Hühner verursacht, expremieren ein Protein, das mit VP22 verwandt ist und das ebenfalls als Translokations-Modul geeignet ist [11]. Diese Proteine und Teilsequenzen dieser Proteine sind nur exemplarisch genannt, und es sind derzeit zahlreiche weitere Peptide bekannt, bzw. werden zukünftig noch bekannt werden, die als Translokations-Module im Sinne der Erfindung geeignet sind.

### Homeoproteine, die als Translokations-Module geeignet sind

Eine weitere Gruppe von im Sinne der vorliegenden Erfindung geeigneten Translokations-Modulen sind Peptide, die vom "Drosophila homeotic protein Antennapedia" (ANTp) abgeleitet sind [9]. Unter anderem sind auch ANTp-Peptide, die eine invertierte Sequenz von ANTp beinhalten [7], die D-Isomere von Aminosäuren enthalten [7], oder die Punktmutationen in ihrer Sequenz enthalten [7] als Translokations-Modul geeignet. Es wird außerdem erwartet, dass noch zahlreiche weitere Sequenzmodifikationen von ANTp möglich sind, die vermutlich Translokation ermöglichen werden [7]. ANTp-Peptid Varianten werden auch als "Transportan"-Peptide bezeichnet. Weitere Homeoproteine wie z.B. Engrailed 1 (En1), Engrailed 2 (En2), Hoxa-5, Hoxc-8, Hoxb-4 und KNOTTED-1 [7] beinhalten ebenfalls Sequenzen, die im Sinne der vorliegenden Erfindung als Translokations-Modul verwendet werden können. KNOTTED-1 ist sogar ein pflanzliches Protein, das jedoch ebenfalls in tierischen Zellen als Translokations-Modul geeignet ist. Diese Peptide sind nur exemplarisch genannt und es sind zahlreiche weitere Homeoproteine bekannt [12], die Peptidsequenzen enthalten, die als Translokations-Modul im Sinne der Erfindung geeignet sein können. Auch weitere, bisher noch nicht bekannte Homeoproteine können Sequenzen enthalten, die als Translokations-Modul geeignet sind.

### Leucine Zipper Proteine, die als Translokations-Modul geeignet sind

Eine weitere Gruppe von im Sinne der vorliegenden Erfindung als Translokations-Modul geeigneten Sequenzen sind Peptide, die eine "leucine zipper" Domäne enthalten. Beispiele für Proteine, deren "Leucine Zipper" Domänen als Translokationssequenz verwendet werden können sind z.B. human cFos-(139-164), human cJun-(252-279), oder der Hefe-Transkriptionsfaktor yeast GCN4-(231-252) [8]. Weitere derzeit schon bekannte oder zukünftig bekannt werdende "Leucine Zipper" Proteine sind ebenfalls im Sinne der Erfindung als Translokations-Modul geeignet.

Arginin- oder lysin-reiche Peptide, die als Translokations-Modul geeignet sind Arginin-reiche Peptide, häufig abstammend von RNA- und DNA-bindenden Proteinen, stellen weitere Peptidsequenzen dar, die im Sinne der vorliegenden Erfindung als Translokations-Module verwendet werden können. Beispiele für solche Sequenzen sind HIV-1 Rev-(34-50), "flock house virus coat protein" FHV coat-(35-49), BMV Gag-(7-25), HTLV-II Rex-(4-15), CCMV Gag-(7-25), P22 N-(14-30), lambda N-(1-22), phi 21 N-(12-29) und PRP6-(129-144) aus der Hefe [8]. Ebenfalls im Sinne der Erfindung können Polyarginin-Peptide mit 4 bis 16 [8] oder auch mit mehr als 16 Argininresten verwendet werden. Zusätzlich zu Polyarginin-Peptiden können auch Peptide als Translokations-Module verwendet werden, die neben Arginin noch weitere Aminosäuren beinhalten, z.B. das W/R-Peptid (RRWRRWWRRWWRRWRR [9], oder das R9-Tat-Peptid, bei dem die 9 zentralen Aminosäurereste der insgesamt 11 Aminosäuren eines Tat-Peptides durch Argininreste ersetzt wurden (GRRRRRRRRRQ) [8]. Außerdem konnte gezeigt werden, das auch Peptide, die z.B. aus neun Lysinresten bestehen, die Fähigkeit haben, als Translokations-Modul im Sinne der Erfindung zu wirken [13]. Diese Peptide sind nur exemplarisch genannt und es sind zahlreiche weitere Arginin- oder Lysin-reiche Peptide geeignet, als Translokations-Modul im Sinne der Erfindung verwendet zu werden [8, 13]. Weitere derzeit schon bekannte oder in der Zukunft noch bekannt werdende arginin- oder lysin-reiche Peptide sind vermutlich auch als Translokations-Modul geeignet. Auch Sequenzen, die Guanidino- oder Amidino-Gruppen enthalten, sind im Sinne der vorliegenden Erfindung als Translokations-Modul geeignet [14].

### Proteine ohne Signalsequenz, die als Translokations-Modul geeignet sind

Eine Reihe weiterer Proteine haben die Fähigkeit, ohne das eine Sekretions-Signalsequenz vorhanden ist, die Zellmembran von innen nach außen zu penetrieren, d.h. sekretiert zu werden. Diese Proteine können häufig auch umgekehrt von außen ins Zellinnere eindringen. Diese Proteine oder Teilsequenzen dieser Proteine können somit auch als Translokations-Module im Sinne der vorliegenden Erfindung verwendet werden. Einige exemplarische Beispiele für solche Proteine sind "fibroblast growth factor 1" (FGF-1), "fibroblast growth factor 2" (FGF-2), Caveolin-1, Lactoferrin, Thioredoxin, Interleukin 1 beta und"ciliary neurotrophic factor" (CNTF) [7], oder Interleukin 1 alpha,"vas deferens protein", platelet-derived endothelial cell growth factor" (PD-ECGF), Thymosin, Para-Thymosin, 14.5 kDa lectin (L14), Transglutaminase, "thioredoxin-like protein" (ADF),"sciatic nerve growth-promoting activity", Faktor XIIIa, "mammary-derived growth inhibitor", Galectin, Rhodanase [15].

Diese Peptide sind nur exemplarisch genannt und es sind zahlreiche weitere Peptide bekannt oder werden in der Zukunft noch bekannt werden, die als Translokations-Modul im Sinne der Erfindung geeignet sind.

Toxine, die als Translokations-Modul geeignet sind Viele Toxine oder Teilsequenzen von Toxinen haben die Eigenschaft, als Translokations-Modul zu wirken, wie z. B. folgende Toxine : komplettes Abrin, komplettes Ricin, komplettes Modeccin, komplettes Pseudomonas Exotoxin A, komplettes Diphtheria Toxin, komplettes Pertussis Toxin, komplettes Shiga Toxin, die A-Kette von Ricin, die A-Kette von Abrin, die A-Kette von Modeccin, die enzymatisch aktive Domäne von Pseudomonas Exotoxin, die A-Kette von Diphtheria Toxin A, die enzymatisch aktive Domäne von Pertussis Toxin, die enzymatisch aktive Domäne von Shiga Toxin, Gelonin, Pokeweed Antiviral Protein, Saporin, Tritin, Barley Toxin und Schlangengift-Peptide [16]. Diese exemplarisch genannten Toxine und viele weitere nicht ausdrücklich genannte andere Toxine oder zukünftig bekannt werdende Toxine können im Sinne der vorliegenden Erfindung als Translokations-Modul verwendet werden.

Steuerung der Effizienz von Translokations-Modulen Durch Variation der Länge von z. B. einer poly-Arginin-Kette oder durch gezielte Auswahl von z. B. nur einer Teilsequenz des HIV-Tat Sequenz kann die Effizienz der Translokation gesteuert werden, so dass je nach den jeweiligen Erfordernissen eine sehr effiziente Translokation des entsprechenden MAT- Moleküls erfolgt oder eine weniger effiziente Translokation [8,13]. Eine sehr effiziente Translokation kann den Vorteil haben, dass die Wirksamkeit des MAT- Moleküls erhöht ist und/oder dass die notwendige Dosis an MAT-Molekül verringert werden kann, was wiederum Kosten für Impfstoff spart. Eine verringerte Dosis an MAT-Molekül wiederum hat den Vorteil, das geringere Nebenwirkungen auftreten. Andererseits ermöglicht eine verringerte Effizienz der Translokation es, dass die MAT-Moleküle sich im behandelten Individuum z. B. nach intravenöser Injektion, weiträumig verteilen, da sie nicht sofort lokal nahezu quantitativ in alle Zellen eindringen, die sich in der Nähe befinden.

### Beispielhafte minimale Sequenzen, die als Translokations-Modul wirken

Alle beispielhaft genannten Translokationssequenzen müssen nicht in Form des gesamten Proteins Bestandteil des MAT-Moleküls sein, um als Translokations-Modul für das MAT-Molekül im Sinne der Erfindung wirksam zu sein. Vielmehr ist für viele der genannten Proteine ein minimaler Sequenzbereich bekannt, die als Translokationssequenz verwendet werden kann. Dieser Sequenzbereich umfasst z.B. für HIV-Tat z.B. die folgende Sequenz: Tyr-Gly-Arg-Lys-Lys-Arg-Arg-Gln-Arg-Arg-Arg, für VP22 die folgende Sequenz: Asp-Ala-Ala-Thr-Ala-Thr-Arg-Gly-Arg-Ser-Ala-Ala-Ser-Arg-Pro-Thr-Glu-Arg-Pro-Arg-Ala-Pro-Ala-Arg-Ser-Ala-Ser-Arg-Pro-Arg-Arg-Pro-Val-Glu und für Antennapedia die folgende Sequenz: Arg-Gln-Iso-Lys-Iso-Trp-Phe-Gln-Asn-Arg-Arg-Met-LysTrp-Lys-Lys [17]. Außerdem können die Sequenzen auch in Form von Fragmenten verwendet werden, die nicht den derzeit bekannten minimalen funktionellen Sequenzabschnitten entsprechen, solange die resultierende Sequenz noch funktionell im Sinne eines Translokations-Moduls ist.

Translokations-Module müssen daher nicht in Form des gesamten Proteins oder des gesamten Moleküls Bestandteil des MAT-Moleküls sein, um als Translokations-Modul für das MAT-Molekül im Sinne der Erfindung wirksam zu sein. Vielmehr ist z.B. für einige der genannten Proteine ein Sequenzbereich bekannt, der als Translokations-Modul verwendet werden kann. Außerdem können die Proteinsequenzen auch in Form von Fragmenten verwendet werden, die nicht den bisher bekannten funktionellen Sequenzabschnitten entsprechen, solange die resultierende Sequenz noch als Translokations-Modul funktionell ist. Die Prüfung der Funktionalität eines Translokations-Moduls kann z.B. unter Verwendung von fluoreszenzmarkierten Translokations-Modulen oder unter Verwendung von enzymmarkierten Translokations-Modulen oder unter Verwendung von mit Metallpartikeln markierten Translokations-Modulen ermittelt werden. Die so markierten Translokations-Module werden einem Versuchstier oder in vitro kultivierten Zellen verabreicht und der Verbleib der Translokations-Module mit Methoden wie FACS ("fluoresence activated cell sorting"), Mikroskopie, konfokaler Fluoreszenzmikroskopie, Elektronenmikroskopie usw. verfolgt. Diese Techniken zur Überprüfung der Funktionalität von Translokations-Modulen sind in der Literatur beschrieben und z.T. schon zur Ermittlung der Funktionalität von Sequenzen zur Translokation verwendet worden [8, 18].

### Targetingsequenzen/Targeting-Module

Die Begriffe Targetingsequenz und Targeting-Modul werden im Text der vorliegenden Anmeldung gleichwertig und mit gleicher Bedeutung nebeneinander verwendet. Unter Targeting ist in dieser Patentanmeldung grundsätzlich immer intrazelluläres Targeting gemeint. Der Begriff Targeting-Modul ist eingeführt worden, um klar zu stellen, dass Translokations-Module nur ein Teil eines MAT-Moleküls im Sinne der Erfindung sind.

Die Erfindung umfasst die Verwendung verschiedener Sequenzen als Targeting-Module zur Herstellung von MAT-Molekülen die zumindest aus einem Translokations-Modul, zumindest einem Targeting-Modul und zumindest einem Antigen-Modul bestehen. Generell sind alle derzeit bekannten und zukünftig bekannt werdenden Aminosäuresequenzen und Moleküle, die Targeting vermitteln können, geeignet, im Sinne der vorliegenden Erfindung als TargetingModule verwendet zu werden. In der Literatur sind zahlreiche geeignete Sequenzen beschrieben. Zu diesen als Targeting-Modul geeigneten Sequenzen gehören alle Sequenzen, die bewirken, dass das MAT-Molekül intrazellulär zu den Stellen oder Organellen innerhalb einer Zelle transportiert wird, an denen Vorgänge stattfinden, die an der Präsentierung der in dem MAT-Molekül enthaltenen Antigen-Module beteiligt sind. Zu diesen Stellen und Organellen innerhalb der Zelle gehören insbesondere"MHC class 11 compartments" (MIICs), Endosomen, Lysosomen, der Golgi Apparat, das trans-Golgi Netzwerk und das endoplasmatische Retikulum. Diese intrazellulären Organellen sind an Vorgängen wie z. B. dem Transport oder der Prozessierung von Antigenen, der Vorbereitung und Beladung der MHC II Moleküle mit Antigenen oder prozessierten Antigenen, und dem Transport der mit Antigenen beladenen MHC II Moleküle an die Zelloberfläche usw. beteiligt.

MHC-Moleküle, die Sequenzen enthalten, die als Targeting-Modul geeignet sind. Im Sinne der Erfindung sind insbesondere eine Reihe von Sequenzen als Targeting-Module geeignet. Die Invariante Kette des MHC II Moleküls (Ii, invariant chain, MHC II gamma chain) ist die in der Literatur am häufigsten beschriebene Sequenz, die Targeting vermitteln kann. Beim Menschen sind verschiedene Varianten der Invarianten Kette beschrieben, die auch als IiP33, IiP41, IiP35 und IiP43 bezeichnet werden [1] und die als Targeting-Module geeignet sind. Weitere im Sinne der Erfindung als Targeting-Modul geeignete

Sequenzen sind die beta-Kette des MHC II Moleküls [19]. Auch Fragmente der genannten Sequenzen sind als Targeting-Modul geeignet.

### Lysosomale Membranproteine, die Sequenzen enthalten, die als Targeting-Modul geeignet sind

Eine Reihe von Membranproteinen, die in Lysosomen vorkommen und dort einer der Haupt-Proteinbestandteile sind, weisen Sequenzmotive auf, die ein Targeting zum Lysosom bewirken. Zu dieser Gruppe von Proteinen gehören unter anderem Lamp 1 ("lysosomal-associated membrane protein-1"), Lamp 2, Lamp 3, Limp II ("Lysosomal integrated membrane protein II") und LAP ("Lysosomal acid phosphatase") [4]. Diese und andere derzeit bekannte oder zukünftig bekannt werdende lysosomale Proteine, die Targeting-Sequenzmotive aufweisen, können im Sinne der Erfindung als Targeting-Module verwendet werden, wobei die gesamte Proteinsequenz oder Teilsequenzen davon als Targeting-Modul verwendet werden können.

### Tetraspan-Proteine, die Sequenzen enthalten, die als Targeting-Modul geeignet sind

Die Mitglieder einer Familie von Proteinen, die vier membrandurchspannende Domänen aufweisen ("tetraspan proteins"), sind ebenfalls in Sinne der Erfindung als Targeting-Module geeignet, da diese Proteine sehr effizient in MIICs gelangen. Der Mechanismus, über den "Tetraspan" Proteine in MIICs gelangen ist unklar, da sie keine bekannten Aminosäuresequenzen enthalten, die ein Targeting ermöglichen. Trotzdem gibt es Mechanismen, die diese Proteine in die MIICs transportieren, das heißt, sie können im Sinne der vorliegenden Erfindung als Targeting-Module verwendet werden. Zur Familie der "Tetraspan" Proteine zählen CD37, CD53, CD63, CD81, CD82 und CD86 [20]. Für CD63 und CD82 ist bekannt, dass sie mit MHC II, HLA-DO und HLA-DM Molekülen assoziieren [20]. "Tetraspan" Proteine sind ebenfalls in den Membranen von Exosomen vorhanden. Exosomen sind Vesikel, die nach der Fusionierung von MIICs mit der Plasmamembran entstehen und so von der antigenpräsentierenden Zelle freigesetzt werden. Exosomen enthalten MHC II Moleküle und können Antigene präsentieren und so T-Zellen stimulieren [20]. Weitere Proteine, die sehr ähnlich zu der "Tetraspan" Proteinfamilie sind, und die daher ebenfalls im Sinne dieser Erfindung als Targeting-Modul geeignet sind, sind das Schistosoma-mansoni-Membranprotein SM23 und das tumorassoziierte Antigen CO-029 [21]. Weitere nicht ausdrücklich genannte oder derzeit noch nicht bekannte Tetraspan-Proteine oder Teilsequenzen von Tetraspan-Proteinen können ebenfalls Sequenzen enthalten, die als Targeting-Module geeignet sind. Weitere Proteine mit Sequenzen, die als Targeting-Module geeignet sind Zahlreiche weitere Proteine sind im endosomalen/lysosomalen Kompartiment verschiedener Zelltypen zu finden und gelangen daher über bestimmte Mechanismen, die derzeit nur teilweise bekannt sind, dort hin. Diese Proteine oder Teilsequenzen dieser Proteine sind folglich auch im Sinne der vorliegenden Erfindung geeignet, als Targeting-Module verwendet zu werden. Zu diesen im endosomalen/lysosomalen Kompartiment vorhandenen Proteinen zählen unter anderem das Lipoprotein geringer Dichte ("low density lipoprotein", LDL), Insulin, Epidermis-Wachstumsfaktor (epidermal growth factor, EGF), polymeres Immunglobulin, Transferrin, der kationenabhängige Mannose-6-Phosphat Rezeptor, der kationenunabhängige Mannose-6-Phosphat Rezeptor, CD3, usw. [21], sowie CD1b [22], und viele weitere derzeit bekannte oder zukünftig bekannt werdende Proteine oder Protein-Sequenzen, die ein Targeting in das endosomale/lysosomale Kompartiment erlauben. Diese Sequenzen können im Sinne der Erfindung als Targeting-Modul verwendet werden. Verschiedene kommerzielle Anbieter bieten Expressions-Vektoren an, in denen Nukleinsäuresequenzen enthalten sind, die für Sequenzen kodieren, die zum Targeting geeignet sind. Zum Beispiel bieten sowohl die Firma BD Bioscience Clontech (Palo Alto, CA, USA) und die Firma Stratagene (La Jolla, CA, USA) Expressions-Vektoren mit Targeting-Modulen an, die das resultierende Fusionsprotein zum Golgi Apparat oder zu den Peroxisomen leiten. Diese Expressionsvektoren erlauben es jedoch nicht, Fusionsproteine herzustellen, die ein Translokations-Modul beinhalten, so dass sie verschieden zu den erfindungsgemäßen MAT-Molekülen sind. In den Vektoren von Clontech wird dazu z.B. eine Sequenz von Calreticulin (KDEL "retrieval sequence") zum Targeting des Fusionsproteins zum Golgi Apparat verwendet. Weitere nicht ausdrücklich genannte oder derzeit noch nicht bekannte Proteine können ebenfalls Sequenzen enthalten, die als Targeting-Module im Sinne der Erfindung geeignet sind.

### Sequenzmotive, die in bestimmten Gruppen von Targetingsequenzen vorkommen

Für verschiedene Proteinsequenzen wurden bestimmte Sequenzmotive ermittelt, die für ihre Funktion als Targeting-Modul wichtig sind. Für die beta-Kette von HLA-DM wurde z.B. das Sequenzmotiv Tyrosin-Threonin-Prolin-Leucin als Targeting-Motiv identifiziert, wobei der Tyrosin- und der Leucinrest von besonderer funktioneller Bedeutung zu sein scheinen [2]. Für verschiedene lysosomale Membranproteine wie LAP ("Lysosomal acid phosphatase"), Lamp 1 ("Lysosomal associated membrane protein 1 "), Lamp 2 und Lamp 3 [3, 4] und für CD 1 b [22] wurden Tyrosin-Motive identifiziert, die für das intrazelluläre Targeting von Bedeutung sind. Für ein weiteres lysosomales Protein, Limp II ("Lysosomal integrated membrane protein II") konnte ein Leucin-Motiv identifiziert werden, das für intrazelluläres Targeting wichtig ist [4]. Für Ii konnte gezeigt werden, dass zwei Sequenzbereiche unabhängig voneinander für das intrazelluläre Targeting von Bedeutung sind (Aminosäureposition 1 bis 11 und Position 12 bis 29) [23]. Beide Targetingsequenzen für sich allein sind schon funktionell und die Sequenz 1 bis 11 enthält an Position 7 und 8 ein funktionell essentielles Leucin-Isoleucin-Motiv [23]. Die beta-Kette des MHC II Moleküls enthält ebenfalls ein Sequenz-Motiv, das ein oder zwei funktionell wichtige Leucinreste beinhaltet und unmittelbar vor diesem Leucin-Motiv befindet sich N-terminal ein konservierter Glycinrest [19]. Zusammenfassend haben demnach insbesondere Leucin- und Tyrosinreste eine wichtige Funktion in Targetingsequenzen und es können daher entsprechende Aminosäuresequenzen als Targeting-Module gezielt entworfen werden. Diese können als Targeting-Module im Sinne der Erfindung verwendet werden.

### "Nicht-Aminosäure-Strukturen" die als Targeting-Module verwendet werden können

Auch Moleküle, die nicht einer Aminosäuresequenz oder einer Aminosäure entsprechen, können im Sinne der Erfindung als Targeting-Module für MAT-Moleküle eingesetzt werden. Ein seit langem bekanntes Beispiel für eine Struktur, die zum Targeting in Lysosomen geeignet ist, ist z.B. Mannose-6-Phosphat [24]. Proteine, die Mannose-6-Phosphat Reste beinhalten, werden von verschiedenen Mannose-6-Phosphat Rezeptoren zu den Lysosomen transportiert. Diesen Mechanismus kann man im Sinne der Erfindung dazu nutzen, das MAT-Molekül in die Lysosomen zu transportieren, um so eine effiziente Präsentation des Antigens zu erreichen. Dazu können Mannose-6-Phosphat Reste allein oder als Bestandteil komplexerer Zuckerstrukturen an das MAT-Molekül kovalent oder nicht kovalent gekoppelt werden. Generell können alle Liganden von Mannose-6-Phosphat Rezeptoren in Sinne der vorliegenden Erfindung als Targeting-Module für MAT-Moleküle verwendet werden. Weitere derzeit bekannte Strukturen oder zukünftig bekannt werdende Strukturen, die ein Targeting zu MIICs, Endosomen, Lysosomen, dem Golgi Apparat, dem trans-Golgi Netzwerk oder dem endoplasmatischen Retikulum ermöglichen, können als Targeting-Modul im Sinne der Erfindung verwendet werden.

Alle beispielhaft genannten Targeting-Module müssen nicht in Form des gesamten Proteins oder des gesamten Moleküls Bestandteil des MAT-Moleküls sein, um als Targeting-Modul für das MAT-Molekül im Sinne der Erfindung wirksam zu sein. Vielmehr ist z.B. für einige der genannten Proteine ein Sequenzbereich bekannt, der als Targeting-Modul verwendet werden kann. Außerdem können die beispielhaft genannten Proteinsequenzen auch in Form von Fragmenten verwendet werden, die nicht den bisher bekannten funktionellen Sequenzabschnitten entsprechen, solange die resultierende Sequenz noch als Targeting-Modul funktionell ist. Die Prüfung der Funktionalität eines Targeting-Moduls kann z.B. unter Verwendung von fluoreszenzmarkierten Targeting-Modulen oder unter Verwendung von enzymmarkierten Targeting-Modulen oder unter Verwendung von mit Metallpartikeln markierten Targeting-Modulen ermittelt werden. Die so markierten Targeting-Module werden einem Versuchstier oder in vitro kultivierten Zellen verabreicht und der Verbleib der Targeting-Module mit Methoden wie FACS ("fluoresence activated cell sorting"), Mikroskopie, konfokaler Fluoreszenzmikroskopie, Elektronenmikroskopie usw. verfolgt.

### Antigen-Module

Als Antigen-Module können im Sinne der Erfindung grundsätzlich alle Arten von Antigenen eingesetzt werden, die eine Immunantwort modulieren können. Geeignet sind sowohl Antigene, die derzeit schon bekannt sind, als auch Antigene, die zukünftig entdeckt werden. Unter Umständen kann es sich auch um Antigene handeln, die bei herkömmlichen, derzeit im Stand der Technik bekannten, Immunisierungsmethoden nicht zu einer Immunantwort führen, die aber bei Anwendung der neuartigen, in der vorliegenden Patentanmeldung beschriebenen Verfahren zu einer Immunantwort des Individuums führen. Es können nicht nur Proteine und Peptide, sondern auch Zuckerstrukturen, Lipide, z.B. Lipopolysaccharide, Lipoteichonsäuren und andere Bestandteile bakterieller Membranen (CD1b bindet z.B. Zuckerstrukturen und Lipide), Nukleinsäuren wie z.B. CpG-Motive enthaltende DNA, organische Substanzen, wie z.B. Latex oder pharmazeutisch wirksame Substanzen als Antigen im Sinne der Erfindung verwendet werden. Das Antigen kann von Menschen, Tieren, Pflanzen, Pilzen, Parasiten, ein- oder mehrzelligen Mikroorganismen, Viren und anderen Lebensformen stammen. Die Antigene können aus biologischen Material isoliert worden sein, rekombinant hergestellt worden sein oder synthetisch, z.B. durch Peptid-Synthese, hergestellt worden sein. Bei synthetisch hergestellten Antigenen kann es sich um Substanzen handeln, die in der Natur vorkommen oder die in der Natur nicht vorkommen, jedoch durch chemische Synthese erhältlich sind. Beispiele für nicht in der Natur vorkommende, unter Umständen aber als Antigen geeignete Substanzen sind z. B. synthetisch hergestellte Stoffe, die in Medikamenten vorhanden sind, oder synthetische Peptide mit Aminosäuresequenzen, die in der Natur nicht vorkommen, oder Peptidomimetika, usw. In der Natur vorkommende oder synthetisch oder rekombinant hergestellte Antigene können durch molekularbiologische, enzymatische, chemische und andere Methoden modifiziert werden, um ihnen für die jeweilige Anwendung vorteilhaftere Eigenschaften zu verleihen. Diese vorteilhaften Eigenschaften können unter anderem eine höhere oder niedrigere Wirksamkeit als Antigen, eine breitere oder eine spezifischere Wirkung als Antigen, eine bessere Löslichkeit in hydrophilen oder hydrophoben Lösungsmitteln, eine höhere Permeabilität der Antigen-Module für Zellmembranen, für Membranen von Organellen, für die Blut-Hirn-Schranke, für die Blut-Liquor-Schranke usw., eine höhere oder geringere in vivo oder in vitro Halbwertszeit, eine geringere oder höhere Toxizität, eine bessere in vivo oder in vitro Nachweisbarkeit des Antigens nach Anwendung des Antigens in Form eines MAT-Moleküls usw. sein. Außerdem können im Sinne der Erfindung mehrere Antigene in einem Antigen-Modul kombiniert werden [25]. Es können dazu identische Antigene in mehrfacher Kopie im Antigen-Modul enthalten sein, oder es können z. B. verschiedene Varianten des gleichen Antigens in einem Antigen-Modul kombiniert werden. Auch können Antigene z. B. von Antigen 1 und andere Antigene z. B. vom Antigen 2 in einem Antigen-Modul kombiniert werden usw. Auch weitere Kombinationen, wie z. B. Antigen 1 in mehrfacher Kopie und Antigen 2 in einfacher Kopie können in einem Antigen-Modul kombiniert werden usw. Außerdem können auch ein oder mehrere verschiedene und/oder ein oder mehrere identische Antigen-Module in einem MAT-Molekül vorhanden sein. Prinzipiell sind alle möglichen Kombinationen von einfach und mehrfach vorhandenen identischen oder veränderten Kopien von Antigenen, abstammend von einem oder mehreren verschiedenen Antigenen im Sinne der Erfindung kombinierbar.

Antigene und Allergene, die als Antigen-Modul verwendet werden können In der Literatur sind bisher schon zahlreiche Antigene, insbesondere Allergene beschrieben. Die nachfolgend konkret bekannten Allergene können im Sinne der Erfindung als Antigen-Modul verwendet werden. Weitere Allergene und Varianten von Allergenen, die ebenfalls im Sinne der Erfindung als Antigen- Modul verwendet werden können, sind im Stand der Technik bekannt [26,27].

Die Allergene sind nach Gruppen wie Allergene von Kräutern und Gräsern, von Bäumen, von Milben, von Pilzen, von Insekten, von Lebensmitteln und von sonstigen Allergene wie z. B. Latex-Allergene geordnet. In den Aufzählungen sind wie folgt aufgelistet: wissenschaftliche Name des Organismus, eine gebräuchliche Abkürzung des Allergens direkt gefolgt von der GeneBank Accession-Nr. des Allergens (in Klammern geschrieben), sofern bekannt.
Allergene von Kräutern und Gräsern:
Ambrosia artemisiifolia, Amb a 1 und Amb a 2; Mercurialis annua, Mer a 1 (Y13271); Parietaria judaica, Par j 1 (X77414), Par j 2 (X95865; X95866); Cynodon dactylon, Cyn d 1(S83343); Dactylis glomerata, Dac g 3 (U25343); Holcus lanatus, Hol I 1 (Z27084, Z68893); Lolium perenne, Lol p 1 (M57474), Lol p 2 (X73363) Lol p 5 (M59163); Phalaris aquatica, Pha a 1(S80654); Phleum pratense, Phl p 1 (X78813), Phl p 2 (X75925),Phl p 3, Phl p 5 (X74735)
Allergene von Bäumen:
Alnus glutinosa, Aln g 1 (S50892); Betula verrucosa, Bet v 1 (X15877), Bet v 2, Bet v 1 d; Carpinus betulus, Car b 1 (X66932, X66918) ; Corylus avellana, Cor a 1 (X70999, X71000, X70997, X70998, Z72439, Z72440, AF136945, AF323973, AF323974, AF323975); Ligustrum vulgare, Lig v 1 (X77787, X77788); Olea europea, Ole e 1 (S75766), Ole e 9 (AF249675); Syringa vulgaris, Syr v 1 (X76541); Cryptomeria japonica, Cry j 1, Cry j 2 (D29772, D37765); Cupressus arizonica, Cup a 1 (AJ278498); Cupressus sempervirens, Cup s 1 (AF257491); Juniperus ashei, Jun a 2 (AJ404653)
Allergene von Milben:
Blomia tropicalis, Blo t 5 (U59102); Dermatophagoides farinae, Der f 1, Der f 2, Der f 11; Dermatophagoides pteronyssinus, Der p 1, Der p 2, Der p 5, Der p 7; Lepidoglyphus destructor, Lep d 2 (X81399); P. americana, Cra-A; T. putrescentiae, Tyr p2
Allergene von Tieren:
Bos domesticus, Bos d 2 (L42867) ; Equus caballus, Equ c 1 (U70823); Felis domesticus, Fel d 1 (M74952, M74953)
Allergene von Pilzen:
Alternaria alternata, Alt a 1 (U82633), Alt a 2 (U62442); Aspergillus flavus, Asp fl 1 (AF137272); Aspergillus fumigatus, Asp f 1 (M83781, S39330), Asp fl/a, Asp f 2 (U56938), Asp f 3 (U20722, U58050), Asp f 4, Asp f 6, Asp f 8; Aspergillus niger, Asp n 18; Aspergillus oryzae, Asp o 13 (X17561); C. comatus, Cop c 1; Penicillium chrysogenum, Pen ch 13 (AF193420), Pen ch 20 (S77837); Penicillium oxalicum, Pen o 18 (AAG44478); Malassezia sympodialis, Mal s 1 (X96486)
Allergene von Insekten:
Apis mellifera, Api m 1 (X16709), Api m 2 (L10710), Api m 4 (X02007); PLA2 (X16709); Blattella germanica, Bla g 1(AF072219, L47595, AF072221, AF072220), Bla g 2 (U28863), Bla g 4 (U40767), Bla g 5 (U92412); Periplaneta americana, Per a 1 (AF072222), Per a 3 (L40819); Dolichovespula maculata, Dol m 1 (X66869), Dol m 2 (L34548) Dol m 5 (J03601); Dolichovespula arenaria, Dol a 5 (M98859), Polistes annularies, Pol a 5 (M98857); Vespula vulgaris, Ves v 1 (L43561), Ves v 2 (L43562), Ves v 5 (M98858); Myrmecia pilosula, Myr p 1 (X70256), Myr p 2 (S81785)
Allergene aus Lebensmitteln:
Salmo salar, Sal s 1 (X97824); Bos domesticus, Bos d 4 (M18780), Bos d 5 (X14712); Gallus domesticus, Gal d 1 (J00902), Gal d 2 (J00992); Metapenaeus ensis, Met s 1 (U08008); Hordeum vulgare, Hor v 15 (X63517); Oryza sativa, Ory s 1 (U31771); Apium graveolens, Api g 1 (Z48967); Daucus carota, Dau c 1 (U47087, D88388); Malus domestica, Mal d 1 (X83672); Pyrus communis, Pyr c 1 (AF057030); Persea americana, Pers a 1 (Z78202); Prunus armeniaca, Pru ar 1 (U93165); Prunus avium, Pru av 1 (U66076); Arachis hypogaea, Ara h 1 (L34402), Ara h 2 (L77197); Bertholletia excelsa, Ber e 1 (M 17146); Juglans regia, Jug r 1 (U66866), Jug r 2 (AF066055); Ricinus communis, Ric c 1 (X54158); Sesamum indicum, Ses i 1 (AF240005) Weitere Allergene (Latex):
   Hevea brasiliensis, Hev b 1 (X56535),Hev b 2, Hev b 3, Hev b 5 (U42640), Hev b 6 (M36986), Hev b 7, Hev b 8

Diese bekannten Allergene sind lediglich beispielhaft genannte und es sind weitere Alltergene aus dem Stand der Technik bekannt, die ebenfalls im Sinne der Erfindung in Antigen-Modulen verwendet werden können.

Neben Allergenen sind eine Reihe von Krankheitserreger bekannt, gegen die derzeit noch keine wirksame oder dauerhafte Immunisierung zur Verfügung steht. Da das erfindungsgemäße Verfahren auf einer neuartigen Immunisierungsstrategie basiert, ist es möglich, dass zur Immunisierung gegen diese bisher durch Immunisierungen nicht befriedigend prophylaktisch behandelbaren Erkrankungen, Wirkung zeigt. Zu diesen Erkrankungen zählen insbesondere Infektionen mit HIV-Viren, mit Hepatitis C-Viren, mit Erregern von Tuberkulose (Mycobakterium tuberculosis), Lepra (Mycobacterium leprae), Pest (Yersinia pestis) sowie mit Malariaerregern (Plasmodium species, z.B. falciparum).

### Weitere Module, die in MAT-Molekülen vorhanden sein können

Neben den bereits beschriebenen drei Modulen Translokations-Modul, Targeting-Modul und Antigen-Modul, die mindestens in dem MAT-Molekül vorhanden sein müssen, können noch weitere optionale Module in dem MAT-Molekül vorhanden sein. Zu diesen optionalen Modulen gehören z.B. Module, die die Isolierung oder den Nachweis der MAT-Moleküle ermöglichen. Solche Module werden im Stand der Technik oftmals auch als "tags" bezeichnet und werden daher nachfolgend in dieser Patentanmeldung als Tag-Module bezeichnet. Weitere optional in den MAT-Molekülen enthaltende Module können Spacer-Module sein, d.h. Module, die zwischen den anderen Modulen angeordnet sind und deren Aufgabe es ist, diese Module aneinander zu koppeln. Nachfolgend in dieser Patentanmeldung werden diese Module Spacer-Module genannt. Es ist auch möglich, das bestimmte Module gleichzeitig die Funktion von zwei oder mehr Modulen übernehmen. Zum Beispiel können viele Tag-Module gleichzeitig zur Isolierung und zur Detektion des MAT-Moleküls verwendet werden, oder ein in einem MAT-Molekül enthaltendes Antigen-Modul könnte auch zur Detektion und/oder Isolierung des MAT-Moleküls verwendet werden, wenn z.B. ein Antikörper gegen das Antigen-Modul zur Verfügung steht, usw.

### Tag-Module, die in MAT-Molekülen vorhanden sein können

Im Sinne der Erfindung können ein oder mehrere verschiedene und/oder ein oder mehrere gleiche Tag-Module Bestandteil eines MAT-Moleküls sein. Tag-Module können kurze, häufig aus nicht mehr als 20 Aminosäureresten bestehende Peptide sein, können aber auch kompletten Proteinsequenzen oder bestimmten Domänen von Proteinen entsprechen. Tag-Module können auch Funktionelle Gruppen sein, die nicht aus Aminosäuren aufgebaut sind, wie z.B. Biotin oder Digoxigenin. Fast alle Tag-Module können auf zwei verschiedene Art und Weisen verwendet werden. Zum einen können sie zur Isolierung des MAT-Moleküls verwendet werden, zum anderen können sie zum Nachwies der Anwesenheit des MAT-Moleküls verwendet werden. Generell sind alle derzeit bekannten und alle zukünftig noch bekannt werdenden Tag-Module geeignet, im Sinne der Erfindung verwendet zu werden. Beispiele für geeignete Tag-Moleküle, die im Sinne der vorliegenden Erfindung verwendet werden können sind: Histidinsequenzen aus 4 bis 12 oder mehr, vorzugsweise unmittelbar aufeinander folgenden Histidinresten, auch His-tag, His6-tag, HIS6-tag, Penta-His™, Tetra-His™, RGS-His™, usw. genannt (Qiagen, Hilden, Germany), Myc oder c-Myc-tag, PinPoint™-tag (eine Signalsequenz, die bewirkt, dass das entsprechende Protein in vivo von Bakterien mit einer Biotingruppe versehen wird), HA-tag, 6xHN-tag (Promega Biosciences Inc., San Louis Obispo, CA, USA), Xpress™-tag, myc-tag, V5-tag (Invitrogen, Breda, The Netherlands), S-tag, CBD-tag, GST-tag, HSV-tag, T7-tag (Novagen Inc., Madison, WI, USA), FLAG-tag, HA-tag, c-myc-tag, "calmodulin-binding peptide tag" (CBP) tag (Stratagene, La Jolla, CA, USA), His-tag, Protein A-tag, Glutathion S-Transferase (GST) tag (Amersham Biosciences, Uppsala, Sweden), Strep-tagll (IBA GmbH, Göttingen, Germany), His-tag (Roche Applied Science, Rotkreuz, Switzerland), FLAG-tag, GST-tag, Protein A-tag (Sigma, St. Louis, MO, USA), "Maltose Binding Protein" (MBP), "chitin-binding tag", (New England Biolabs, Beverly, MA, USA), His-tag (BD Biosciences Clontech, Palo Alto, CA, USA). Für einige dieser Tag-Module sind auch Anwendungen beschrieben, bei denen mehr als ein Tag-Modul in einem Molekül verwendet werden. Zum Beispiel können zwei Tag-Module am N- oder am C-terminus eines Proteins gekoppelt sein oder es kann je ein Tag-Modul am N- und eines am C-terminus gekoppelt werden (Qiagen, Hilden, Germany und Stratagene, La Jolla, CA, USA). Tag-Module können auch intern in der Sequenz von anderen Proteinen, z.B. zwischen zwei Domänen eines Proteins, eingefügt werden (Strep-tagll, IBA, Göttingen, Germany).

Weitere Tag-Module werden primär zum Nachweis des Moleküls, an das sie gekoppelt sind verwendet. Jedoch können diese Tag-Moleküle prinzipiell auch zur Isolierung von Proteinen, z.B. unter Verwendung von Affinitätschromatographie, verwendet werden. Dazu können z.B. Chromatographiematerialien verwendet werden, an die Antikörper gegen diese Tag-Module gekoppelt sind. Im Sinne der vorliegenden Erfindung können auch Tag-Module verwendet werden, wie z.B. das "green fluorescent protein" (GFP), das "enhanced green fluorescent protein" (EGFP), das "enhanced cyan fluorescent protein" (ECFP), das "enhanced yellow fluorescent protein" (EYFP), das "red fluorescent protein" (DsRed2) (BD Bioscience Clontech, Palo Alto, CA, USA), das "renilla green fluorescent protein" (hrGFP) (Stratagene, La Jolla, CA, USA), usw. Diese Tag-Module können sowohl am N- als auch am C-terminus z.B. eines Fusionsproteins lokalisiert sein. Neben fluoreszierenden Tag-Modulen können auch Enzyme als Tag-Modul verwendet werden. Häufig verwendete Enzyme sind z.B. Luciferease, beta-Galactosidase, alkalische Phosphatase, Meerrettichperoxidase, usw. Diese Enzyme können über ihre jeweilige Enzymaktivität, d.h. aufgrund der Umsetzung von Substraten dieser Enzyme, nachgewiesen werden. Dazu sind verschiedene Arten von Substraten geeignet, wie z.B. Substrate, die Licht im sichtbaren Bereich des Spektrums absorbieren, fluoreszierende Substrate, Substrate, deren Umsetzung zur Emittierung von Licht führt, oder Substrate, deren enzymatische Umsetzung durch die Abnahme der Konzentration des Substrates oder die Zunahme des Produktes unter Verwendung verschiedener Nachweisverfahren bestimmt werden kann usw.

Eine weitere Verwendungsmöglichkeit von Tag-Modulen im Sinne der vorliegenden Erfindung ist die Verwendung von z.B. Peptidsequenzen, die als Kinasesubstrate geeignet sind. Diese Peptidsequenzen können dann unter Zugabe von radioaktivem Phosphor und Zugabe von Kinasen radioaktiv markiert werden. Beispiele für Tag-Module, die in diesem Sinne für die vorliegende Erfindung Verwendung finden können sind : das "kemptide-tag" (ein Peptid, das von Proteinkinase A phosphoryliert werden kann), das"calmodulin-binding peptide tag" (CBP), das ebenfalls mit Proteinkinase A phosphoryliert werden kann (Stratagene, La Jolla, CA, USA) usw.

Eine weitere Verwendungsmöglichkeit von Tag-Modulen im Sinne der vorliegenden Erfindung ist die Verwendung von z. B. Proteinen, Protein- Domänen, oder Peptidsequenzen, die spezifisch andere Proteine oder andere Strukturen binden. Aus der Literatur bekannte Beispiele für solche Tag-Module sind : Protein A, Protein G, Protein A/G, Protein L, wobei alle diese Proteine an Antikörperstrukturen binden (Pierce, Rockford, IL, USA), Glutathion-S-Transferase, die an Glutathion bindet, das"maltose binding protein" (MBP), dass an Amylose bindet, Streptavidin oder Avidin, die beide an Biotin binden, das"calmodulin-binding peptide"das an Calmodulin bindet, das"chitin-binding tag"das an Chitin bindet, usw. Generell können alle Arten an Molekülen, die jeweils spezifisch an andere Moleküle binden, als Tag-Modul im Sinne der Erfindung verwendet werden, d. h. Rezeptor-Ligand, Antikörper-Antigen, Lektin- Zuckerstruktur, Protein-Lipid, Protein-Nukleinsäure, Protein-Protein, usw., sowie zahlreiche weitere Beispiele, die in der Literatur beschrieben sind [28].

### Spacer-Module

Als Spacer-Module im Sinne der Erfindung können alle Arten von Molekülen verwendet werden, die geeignet sind, andere Module, die Bestandteil des MAT-Moleküls sind, aneinander zu koppeln. Die Kopplung kann sowohl durch kovalente, als auch durch nicht kovalente Bindungen erfolgen. Die Spacer- Module haben unter anderem die Aufgabe, die verschiedenen Module des MAT- Moleküls räumlich derart voneinander zu trennen, dass sie sich nicht gegenseitig in ihrer Funktionalität negativ beeinflussen. Die Kopplung von Modulen des MAT-Moleküls im Sinne der Erfindung kann durch Spacer-Module erfolgen, die durch chemische oder enzymatische Reaktionen, z, B. durch Proteasen, zu einem späteren Zeitpunkt wieder gespalten werden können.

Dadurch können die durch die Spacer-Module verbundenen Module des MAT-Moleküls bei Bedarf wieder voneinander getrennt werden.

Dazu können generell alle derzeit bekannten oder zukünftig bekannt werdenden Proteasen verwendete werden [29, 30]. Derzeit häufig verwendete Proteasen sind Thrombin, Faktor Xa, Enterokinase oder das TAGZyme-System (Qiagen, Hilden, Germany), usw. Verschiedene chemische Reaktion, die geeignet sind zum Spalten von Spacer-Modulen sind dem Fachmann bekannt oder aus Angaben der Hersteller von Spacer-Molekülen, z.B. von der Firma Pierce, zu entnehmen.

Bei den Spacer-Modulen kann es sich um insbesondere um Peptidsequenzen oder um organische Moleküle handeln. Im Stand der Technik sind zahlreiche Spacermoleküle bekannt, die im Sinne der Erfindung verwendet werden können. Außerdem können auch Spacermoleküle im Sinne der Erfindung verwendet werden, die zukünftig entwickelt oder entdeckt werden. Als Spacer-Module sind unter anderem Peptidspacer, Crosslinker, natürliche oder synthetische Polymere wie z.B. Nukleinsäuren, substituierte oder unsubstituierte Kohlenwasserstoffe, usw. geeignet. Außerdem können Kombinationen von Molekülen als Spacer-Module verwendet werden, die über nicht kovalente Wechselwirkungen miteinander Komplexe bilden können und so zwei oder mehr Module zu einem MAT-Molekül zusammenfügen können. Ein bekanntes Beispiel für eine solche Kombination an Molekülen, die aneinander binden ist, Biotin / Streptavidin.

### Peptidsequenzen als Spacer-Module

Viele Proteine, die aus mehreren Domänen bestehen, haben in ihrer Aminosäuresequenz kurze Sequenzbereiche, die in der Literatur auch als Spacer bezeichnet werden. Diese Spacer haben die Aufgabe, die verschiedenen Domänen des Proteins räumlich derart voneinander zu trennen, dass sie sich nicht gegenseitig in ihrer Funktionalität negativ beeinflussen. Dazu muss insbesondere gewährleistet sein, dass die Spacersequenz so flexibel ist, dass die beiden Domänen sich nicht gegenseitig sterisch in ihrer Funktion behindern.

Derartige Peptidsequenzen können im Sinne der vorliegenden Erfindung als Spacer-Module verwendet werden. Eine große Anzahl an verschieden Spacer-Peptidsequenzen sind in der Literatur beschrieben. Vorzugsweise haben diese Spacer eine Länge zwischen 2 und 60 Aminosäuren, können aber auch längere Sequenzen aufweisen. Spacer können auch aus nur einer Aminosäure bestehen. Viele kommerziell erhältliche Expressions-Vektoren enthalten bereits Sequenzbereiche, die für Peptidspacer kodieren, die z.B. eine Tag-Sequenz mit der in den Expressions-Vektor einzufügenden Proteinsequenz verbinden. Oft werden sehr kurze Peptidspacer, bestehend aus nur zwei Aminosäuren wie z.B. Leucin-Glycin, Glycin-Alanin oder Serin-Alanin (IBA GmbH, Göttingen, Germany) verwendet, oder kurze Aminosäuresequenzen von 4 bis 6 Aminosäuren Länge bestehend aus Glycin und/oder Alanin (Qbiogene Inc., Carlsbad, CA, USA). Zahlreiche weitere Spacersequenzen sind in der Literatur beschrieben und können im Sinne der vorliegenden Erfindung als Spacer-Module verwendet werden. Prinzipiell können alle derzeit bekannten Spacermoleküle und zukünftig bekannt werdende Spacermoleküle als Spacer-Modul in den erfindungsgemäßen MAT-Molekülen eingesetzt werden. Eine Methode, um als Spacer-Modul geeignete Aminosäuresequenzen zu identifizieren, ist die Verwendung von Datenbanken, die Aminosäuresequenzen nach Protein-Domänen durchsuchen. Kurze Aminosäuresequenzen von vorzugsweise 2 bis 60 Aminosäuren Länge, die zwischen zwei so identifizierten Protein-Domänen in einer Aminosäuresequenz vorhanden sind, können als Spacer-Modul im Sinne der Erfindung verwendet werden. Eine der derzeit verfügbaren Datenbanken zur Identifizierung von Protein-Domänen und damit auch von Peptidsequenzen, die als Spacer-Modul geeignet sind, ist die "SBASE protein domain library" [31].

### Crosslinker als Spacer-Module

Im Sinne der Erfindung können Spacer-Module auch in Form von Crosslinkern in das MAT-Molekül eingefügt werden. Dazu werden die einzelnen Module des MAT-Moleküls hergestellt und anschließend über chemische Reaktionen mit Crosslinkern kovalent aneinander gekoppelt. Dazu sind zahlreiche Crosslinker kommerziell erhältlich. Zum Beispiel bietet die Firma Pierce (Pierce Biotechnology, Inc., Rockford, IL, USA) zahlreiche verschiedene Crosslinker an. Derzeit kann man z. B. bei Pierce zwischen Crosslinkern auswählen, die mit Aminogruppen, Sulfhydrolgruppen, Zuckerstrukturen, Carboxylgruppen, Hydroxylgruppen oder nicht selektiv mit den Modulen, die zu einem MAT- Molekül kombiniert werden sollen, reagieren. Außerdem stehen derzeit z. B. bei Pierce Biotechnology Inc. zur Herstellung von MAT-Molekülen Crosslinker zur Verfügung, die durch bestimmte chemische Reaktionen wieder getrennt werden können, z. B. durch Thiole, Basen, Perjodat, Hydroxylamin, durch Einwirkung von Licht oder durch unspezifische Reaktionen. Durch die gezielte Auswahl von Crosslinkern kann außerdem der Abstand zwischen den einzelnen Modulen des MAT-Moleküls gezielt festgelegt werden. Derzeit bietet z. B. die Firma Pierce Crosslinker an, die einen Abstand von 1,4 Angström (N-Succinimidyl iodoacetate) bis 34,7 Angström (Bis- (beta- (4-azidosalicylamido) ethyl) disulfide) einfügen, je nachdem welchen Crosslinker man verwendet. Als weitere Variationsmöglichkeit bei der Verwendung von Crosslinkern zum Koppeln von verschiedenen Modulen zu MAT-Molekülen im Sinne der vorliegenden Erfindung kann man den Crosslinker Sulfo-SBED der Firma Pierce Biotechnology Inc. verwenden. Sulfo-SBED koppelt zum einen zwei Module durch kovalente Reaktion und enthält zusätzlich am eingefügten Spacermolekül eine Biotingruppe. An diese Biotingruppe kann dann ein weiteres Modul des MAT-Moleküls über nicht kovalente Bindungen angefügt werden. Dazu kann man das einzufügende Modul z.B. mit Avidin oder Streptavidin koppeln. Das so entstandene Streptavidin-gekoppelte Modul kann dann über die in dem Crosslinker vorhandene Biotingruppe an die anderen Module gekoppelt werden. Im Sinne der Erfindung können prinzipiell alle derzeit bekannten Crosslinker sowie zukünftig bekannt werdenden Crosslinker zum Verknüpfen von Modulen zu einem MAT-Molekül verwendet werden.

### Weitere Spacer-Module

Spacer-Module im Sinne der Erfindung können z.B. aus L-Isomeren oder aus D-Isomeren Aminosäuren, aus ungewöhnlichen Aminosäuren, aus Aminosäuren mit postranslationalen Modifikationen, aus Nukleinsäuren, aus PNAs ("Peptide Nucleic Acids"), aus Lipiden, aus Zuckerstrukturen, oder anderen natürlichen oder synthetischen Polymeren, wie z.B. substituierten oder unsubstituierten Kohlenwasserstoffen, Polyacetat, Polyethylenglykol, Cyclodextrin, Polymethacrylat, Gelatin, Oligoharnstoff usw. bestehen, oder aus anderen Substanzen oder aus Kombinationen der genannten oder weiterer Substanzen. Im Sinne der Erfindung können prinzipiell alle derzeit bekannten Substanzen, die geeignet sind, Module zu einem MAT-Molekül zusammen zu fügen, sowie Moleküle, die zukünftig bekannt werden und entsprechende Eigenschaften haben, als Spacer-Modul zum Verknüpfen von Modulen zu einem MAT-Molekül verwendet werden.

### Spacer-Module, die durch nicht-kovalente Wechselwirkungen aneinander binden

Für diese Klasse an Spacermolekülen gibt es in der Literatur eine große Anzahl an Beispielen. Beispiele für solche Kombinationen an Molekülen, die über nicht kovalente Wechselwirkungen aneinander binden und die kommerziell erhältlich sind: Biotin / Streptavidin oder Avidin oder Strep-tagll (IBA GmbH, Göttingen, Germany) oder PinPoint™-tag (Stratagene, La Jolla, CA, USA), Glutathion S-Transferase / Glutathion und Protein A / konstanter Anteil von Antikörpern (FC-Teil) (Pharmacia Amersham Biosciences, Uppsala, Sweden, Sigma, St. Louis, MO, USA), Maltose-Binding Protein (MBP) / Amylose (New England Biolabs, Beverly, MA, USA), Histidin-tag / Ni-Chelat (Qiagen, Hilden, Germany, BD Bioscience Clontech, Palo Alto, Ca, USA, Invitrogen, Breda, The Netherlands, Novagen Inc., Madison, WI, USA, Roche Applied Science, Rotkreutz, Switzerland), "chitin-binding tag" / Chitin (New England Biolabs, Beverly, MA, USA), "calmodulin-binding protein" / Calmodulin (Stratagene, La Jolla, CA, USA). Außerdem gibt es noch eine Reihe weiterer Molekül-Kombinationen, wie z.B. Rezeptor / Ligand Kombinationen, Antikörper / Antigen Kombinationen, Lektin / Zuckerstruktur Kombinationen, usw. Zahlreiche derzeit schon bekannte Protein-Protein Interaktionen sind in Datenbanken zu finden und können im Sinne der Erfindung als Spacer-Module verwendet werden [28]. Alle derzeit bekannten und in der Zukunft bekannt werdenden Kombinationen von Molekülen, die miteinander nicht-kovalente Bindungen eingehen können, können prinzipiell im Sinne der Erfindung als Spacer-Modul verwendet werden. Eine weitere Methode, Spacer-Module in MAT-Moleküle einzufügen ist die Verwendung von bi-spezifischen Molekülen, die zwei unterschiedliche Bindungsstellen in einem Molekül vereinen. Beispiele für solche Moleküle wären Biotin-markierte Lektine (Pierce Biotechnology, Inc., Rockford, IL, USA), die z.B. ein Streptavidin-markiertes Modul und ein weiteres Modul, das eine Zuckerstruktur trägt, die von dem Lektin gebunden wird, aneinander binden können. Ein weiteres Beispiel für eine derartige Kopplungsmöglichkeit sind bispezifische Antikörper, die zwei verschiedene Epitope erkennen usw.

Eine weitere Variante zum Einfügen von Spacer-Modulen in MAT-Moleküle ist folgende: zunächst werden zumindest zwei Module über ein nicht kovalent bindendes Spacer-Modul aneinander gekoppelt und anschließend wird der Komplex mit chemischen Crosslinkern behandelt, die kovalente Bindungen zwischen Modulen einfügen, die sich in räumlicher Nähe befinden. Dieses hat den Vorteil, das im ersten Schritt gezielt bestimmte Module in definierter Art und Weise aneinander gekoppelt werden und anschließend die nicht kovalente Kopplung in eine stabilere kovalente Kopplung umgewandelt wird. Werden die Module direkt mit Crosslinkern behandelt, die kovalente Bindungen herstellen, so ist in der Regel die Art und Weise, wie die Module aneinander gekoppelt werden, zufällig und nicht spezifisch.

### Aufbau der MAT-Moleküle

Generell ist jede beliebige Anordnung der einzelnen Module des MAT-Moleküls möglich. Jedes Modul kann einmal oder mehrfach im MAT-Molekül vorhanden sein. Als Mindestvoraussetzung muss mindestens ein Translokations-Modul, mindestens ein Targeting-Modul und mindestens ein Antigen-Modul vorhanden sein. Zusätzliche Module wie Tag-Module, Spacer-Module, usw. können optional vorhanden sein, müssen aber nicht vorhanden sein. Alle Module können einmal oder mehrfach im MAT-Molekül vorhanden sein. Sind Module mehrfach vorhanden, so können sie in Form identischer Kopien mehrfach vorhanden sein, oder es können verschiedene Versionen eines Moduls jeweeils in einfacher Kopie oder in mehrfacher Kopie vorhanden sein. Es können auch völlig verschiedene Module der gleichen Klasse von Modulen, z.B. ein His-Tag-Modul und ein Biotin-Tag-Modul, in einem MAT-Molekül vorhanden sein. Beide Module übernehmen funktionell die selbe Aufgabe (Tag-Modul) in dem MAT-Molekül, müssen jedoch hinsichtlich ihrer molekularen Struktur keine Gemeinsamkeiten aufweisen.

Zwei oder mehr identische Kopien eines Antigen-Moduls in einem MAT-Molekül können z.B. dazu dienen, eine verstärkte Immunantwort gegen das betreffende Antigen zu bewirken. Zwei oder mehr verschiedene Antigen-Module können z.B. in einem MAT-Molekül kombiniert werden, um die Immunreaktion gegen zwei oder mehr verschieden Antigene gleichzeitig zu modulieren. Zwei oder mehr verschiedene Translokations-Module in einem MAT-Molekül verwendet werden. Zum Beispiel eine Tat-Sequenz und eine VP22-Sequenz können dazu dienen, die Translokation effizienter zu machen, indem die Translokation des MAT-Moleküls dann bei einem breiteren Spektrum an verschieden Zellarten oder Gewebearten effizient statt findet. Es können auch z.B. zwei oder mehr Tag-Module in einem MAT-Molekül verwendet werden, z. B. ein His-Tag und ein FLAG-Tag, wobei z.B. das His-Tag zur Isolierung des MAT-Moleküls verwendet wird und z.B. das FLAG-Tag zum Nachweis des MAT-Moleküls dient. Es können zwei oder mehr verschiede Targeting-Module in einem MAT-Molekül verwendet werden, z.B eine Sequenz aus der Invarianten Kette des MHC II-Moleküls und als weiteres Targeting-Modul eine Mannose-6-Phosphatgruppe, von denen z.B. die Invariante Kette als Targeting-Modul in die MIICs wirkt und die Mannose-6-Phosphatgruppe ein Targeting ins Lysosom bewirkt, wodurch insgesamt die Effizienz der Antigenpräsentation oder die Anzahl der verschieden Epitope des Antigens, die von den Antigenpräsentierenden Zellen präsentiert werden, erhöht werden kann.

Auch die Position der einzelnen Module innerhalb des MAT-Moleküls kann beliebig variiert werden, solange zumindest ein Translokations-Modul, zumindest ein Targeting-Modul und zumindest ein Antigen-Modul vorhanden ist. Alle oder einige Module des MAT-Moleküls können z.B. auch, anstatt in Form einer linearen Aneinanderreihung von Modulen, als zirkuläre oder als verzweigte Modul-Struktur oder auch in Form von Dendrimeren, oder als eine Kombination aus linearen und/oder verzweigten und/oder zirkulären und/oder dendrimeren Molekülanteilen vorliegen. Zirkuläre Modul-Strukturen des MAT-Moleküls können z.B. durch Reaktion von zwei Cysteinresten miteinander oder durch Reaktion eines Cysteinrestes mit einer Thiolestergruppe innerhalb einer ursprünglich linear aufgebauten Kette an Modulen erzeugt werden. Es gibt kommerzielle Anbieter von Expressionsvektoren, die spezielle Vektoren anbieten, die es ermöglichen, über diese Mechanismen zirkuläre Fusionsproteine herzustellen, wie z.B. das IMPACT™ -TWIN System der Firma New England Biolabs, Beverly, MA, USA. Verzweigte Module könnten z.B. durch Synthese von Peptiden hergestellt werden, bei denen, ausgehend von poly L-Lysin, jeweils an nachfolgenden Lysinresten an beide freien Aminogruppen ein neuer Lysinrest angefügt wird. Dadurch kann eine nahezu beliebig verzweigte Peptidstruktur geschaffen werden. Anschließend können dann z.B. Translokations-Module und/oder Targeting-Module an das verzweigte Peptid Grundgerüst synthetisiert werden werden [32]. Auch durch Protein-Ligation können an ein lineares, zirkuläres oder verzweigtes Peptid-Grundgerüst weitere Module gekoppelt werden [33, 34]. Außerdem können bei der Peptidsynthese z.B. Biotin-Gruppen in das Peptid-Grundgerüst eingefügt werden und an diese Biotin-Gruppen können dann Module über z.B. Streptavidin, das Strep-tag System oder über das PinPoint™-System (IBA GmbH, Göttingen, Germany bzw. Promega Biosciences Inc., San Louis Obispo, CA, USA) an das Peptid-Grundgerüst angefügt werden. So angefügte Module sind dann über nicht kovalente Bindungen an das Peptid-Grundgerüst gekoppelt.

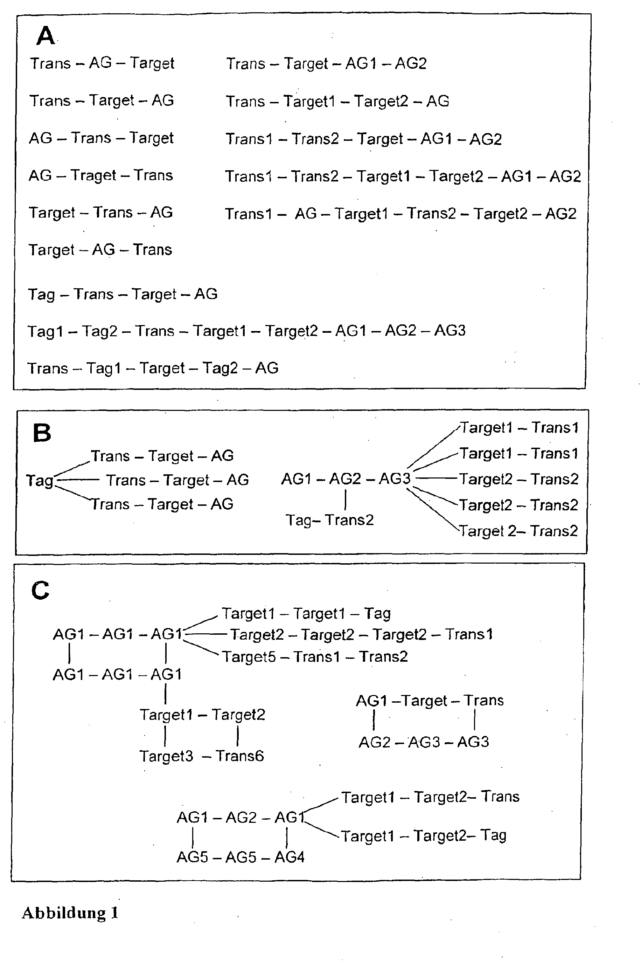

Abbildung 1 zeigt exemplarisch einige Beispiele, wie MAT-Moleküle hinsichtlich ihrer Zusammensetzung aus verschiedenen Modulen und hinsichtlich der Anordung der Module innerhalb des MAT-Moleküls aufgebaut sein können.

### Aufbau der Module von MAT-Molekülen

### Peptide, Proteine, Aminosäuren, ungewöhnliche Aminosäuren, Postranslationale Modifikationen usw.

Die Begriffe Peptid und Protein werden in der vorliegenden Patentanmeldung gleichwertig nebeneinander verwendet. Unter einem Peptid oder einem Protein im Sinne der Erfindung wird eine kovalente Verbindung von zumindest zwei Aminosäuren über eine Peptidbindung verstanden. Der Begriff "Aminosäure" und der Begriff "Aminosäurerest" werden in der vorliegenden Anmeldung gleichwertig benutzt, d.h. die Bedeutung beider Begriffe ist identisch. Die Begriffe Aminosäure/Aminosäurerest und Peptid/Protein werden in der vorliegenden Anmeldung in Form der am weitesten möglichen Definition verwendet.

Unter Aminosäuren im Sinne der Erfindung werden neben den 20 durch den genetischen Code bestimmten Aminosäuren auch die Aminosäuren, die durch Stop-Codons kodiert werden können, wie z.B. Seleno-Cystein oder Pyrro-Lysin verstanden. Außerdem sind alle bekannten Aminosäure- und Peptidderivate wie z.B. glykosylierte, phosphorylierte, sulphatierte Aminosäuren/Peptide, sowie L-isomere und D-isomere Aminosäuren eingeschlossen, sowie Aminosäure- und Peptidderivate, die zukünftig noch bekannt werden. Aminosäure- und Peptidderivate können durch posttranslationale Modifikationen, durch chemische Modifikationen, durch enzymatische Modifikationen oder aufgrund anderer Mechanismen entstehen, bzw. gezielt hergestellt werden. Die resultierenden Peptide können Modifikationen enthalten, die in allen Bereichen des Peptidmoleküls auftreten können. Zum Beispiel können Modifizierungen im Peptid-Rückgrat ("peptide backbone"), in den Aminosäure-Seitenketten, an N-terminalen Enden des Peptids oder an C-terminalen Enden des Peptids auftreten. Die Modifizierungen können bei einzelnen Aminosäuren, bei mehreren Aminosäuren oder bei allen Aminosäuren vorhanden sein und es können keine, eine oder mehrere Arten von Modifizierungen in beliebigen Kombinationen in einem Peptid vorhanden sein. Die Peptide können verzweigt sein, es können zyklische Peptide vorliegen und es können beliebige Kombinationen von verzweigten und zyklischen Peptiden vorliegen. Verzweigte und/oder zyklische Peptide können über natürliche biologische Prozesse entstehen oder synthetisch hergestellt werden. Als Beispiele für ungewöhnliche Aminosäuren sind unter anderem exemplarisch genannt: alpha-Aminobuttersäure, beta-Aminobuttersäure, beta-Aminoisobuttersäure, beta-Alanin, gamma-Aminobuttersäure, alpha-Aminoadipinsäure, 4-Aminobenzoesäure, Aminoethylcystein, alpha-Aminopenicillansäure, Allysin, 4-Carboxyglutaminsäure, Cystathionin, Carboxyglutaminsäure, Carboxyamidomethylcystein, Carboxymethylcystein, Cysteinsäure, Citrolin, Dehydroalanin, Diaminobuttersäure, Dehydroamino-2-Buttersäure, Ethionin, Glycin-Prolin Dipeptid, 4-Hydroxyprolin, Hydroxylysin, Hydroxyprolin, Homoserin, Homocystein, Histamin, Isovalin, Lysinoalanin, Lanthionin, Norvalin, Norleucin, Ornithin, 2-Piperidinecarboxylsäure, Pyroglutaminsäure, Pyrrolysin, Prolin-Hydorxyprolin Dipeptid, Sarcosin, 4-Selenocystein, Syndesine, Thioprolin, usw. Alle genannten Aminosäuren können in Form ihrer L-Isomere oder in Form ihrer D-Isomere vorhanden sein, sofern ihre Struktur dieses zuläst. Generell sind alle derzeit bekannten natürlich vorkommenden oder enzymatisch oder chemisch oder auf andere Art und Weise entstandenen oder herstellbaren Aminosäuren und Aminosäure-Derivate sowie zukünftig bekannt werdende Modifizierungen von Aminosäuren unter den Begriff "Aminosäure" zusammengefasst und können im Sinne der Erfindung Bestandteil von MAT-Molekülen sein.

Als postranslationale oder chemische Modifikationen, die im Sinne der Erfindung in einzelnen oder mehreren Modulen des MAT-Moleküls enthalten sein können, sind unter anderem Modifizierungen der Aminosäuresequenzen durch folgende Strukturen exemplarisch genannt: Bindung von freiem Cystein an ein Cystein in der Peptidsequenz, Bildung von Disulfidbindungen zwischen zwei Cysteinresten, Methylierungen, Acetylierungen, Acylierungen, Farnesylierungen, Formylierungen, Geranylgeranylisierungen, Biotinylierungen, Stearoylierungen, Palmitylierungen, Lipoylierungen, C-Mannosylierungen, Myristoylierungen, Phosphorylierungen, Sulfatylierungen, N-Glykosilierungen, O-Glykosilierungen, Amidierungen, Deamidierungen, Demethylierungen, Cysteinylierungen, Carboxylierungen, Hydroxylierungen, lodierungen, Oxidierungen, Pegylierungen, Prenylierungen, ADP-ribosylierungen, 5'-Adenosylierungen, 4'-Phosphopantheinierungen, Glutathionylierungen, kovalente Bindung: von Flavin, von Häm-Gruppen (oder anderer Porphyrine), von Nukleinsäuren oder von Nukleinsäure-Derivaten, von Lipiden oder von Lipidderivaten, von Phosphatidylinosistol, von Glycosylphospatidylinositol-Ankern (GPI-Ankern), von Pyridoxalphosphat, von Mannose-6-Phosphat, Modifizierungen von Cystein zu Carboxyamidomethylcystein oder Carboxymethylcystein oder Pyridylethylcystein, Modifizierung von Lysin zu Liponsäure, Modifizierung von Glutamin zu Pyroglutaminsäure, Addition von Aminosäuren an Peptide durch tRNAs, Ubiquitinmarkierung von Peptiden, Verzweigungen von Peptiden z.B. in Form von Poly-L-Lysin, Zyklisierungen von Peptiden z.B. durch Ausbildung von Disulfidbindungen zwischen 2 Cysteninresten, usw. möglich. In der Literatur sind zahlreiche weitere Modifizierungen von Proteinen beschrieben, die unter anderen auch in Datenbanken archiviert sind [35]. Generell sind alle derzeit bekannten natürlich vorkommenden oder enzymatisch oder chemisch oder auf andere Art und Weise entstandenen oder herstellbaren Modifizierungen von Peptiden sowie zukünftig bekannt werdende Modifizierungen von Peptiden unter den Begriff "Peptid" zusammengefasst und können im Sinne der Erfindung Bestandteil von MAT-Molekülen sein.

### Peptidomimetics

Außerdem können im Sinne der Erfindung einzelne oder mehrere Aminosäuren von Modulen oder das gesamte Modul oder alle Module des MAT-Moleküls durch Strukturen, bestehend aus Peptidomimetics, ersetzt werden. Der Begriff Peptidomimetics wird in der vorliegenden Anmeldung in Form der am weitesten möglichen Definition verwendet. Ein Peptidomimetikum ist eine Substanz, die nichtpeptidische Strukturelemente enthält und in der Lage ist, die biologische Wirkung des natürlichen Muttermoleküls zu imitieren oder zu antagonisieren. In Stand der Technik sind zahlreiche Arbeiten bekannt, die ausführlich auf Möglichkeiten der Nutzung von Peptidomimetics als Ersatz für herkömmliche Peptidstrukturen eingehen. Generell können ein oder mehrere Module des MAT- Moleküls ganz oder in Teilen aus Peptidomimetics aufgebaut sein [36-38]. Dieses kann verschiedene Vorteile haben. Translokations-Module können dadurch eventuell effizienter in Zellen eindringen, Targeting-Module können das MAT-Molekül effizienter oder weniger effizienter und/oder spezifischer in das gewünschte intrazelluläre Organell transportieren, Antigen-Module können zu einer verstärkten oder verminderten Immunantwort relativ zur Immunantwort gegen das herkömmliche Antigen führen oder Tag-Module können bessere physikochemische Eigenschaften haben, was ihre Eignung zur Isolierung und/oder Detektion des MAT-Moleküls verbessert, usw. Außerdem kann durch den Einsatz von Peptidomimetika unter Umständen die in vivo Stabilität des MAT-Moleküls vermindert oder erhöht, seine Toxizität vermindert oder erhöht, seine Löslichkeit in hydrophilen oder hydrophoben Medien verbessert und seine in vitro Haltbarkeit verlängert und eventuell die Synthesekosten für das Peptidomimetikum relativ zu den Synthesekosten des entsprechenden herkömmlichen Peptids gesenkt werden. Ein Beispiel für Peptidomimetics sind Spiegelmeree der Firma NOXXON Pharma AG, Berlin, Germany. Diese Art von Peptidomimetics hat z. B. den Vorteil, dass sie keine Immunarntwort hervorrufen und daher z. B. in Translolcations-Modulen, Targeting-Modulen, Tag-Modulen, Spacer-Modulen, usw. von MAT-Molekülen sinnvoll eingesetzt werden könnten. Als Antigen-Modul wären Spiegelmeres jedoch nicht geeignet.

### Herstellung und Isolierung von MAT-Molekülen

Die Gewinnung von MAT- Molekülen unter Verwendung von rekombinanten Expressionssystemen, Chromatographiemethoden und chemischen Syntheseprotokollen ist dem Fachmann bekannt. Die so gewonnenen MAT-Moleküle können unter anderem zur Herstellung von Medikamenten und Diagnostika sowie zur Herstellung von Antikörpern in Versuchstieren und in in vitro Systemen Verwendung finden.

### Herstellung von MAT-Molekülen

Zu den dem Fachmann bekannten Methoden zur Herstellung von MAT-Molekülen gehört die rekombinante Expression von Pepiden. Zur Expression der Peptide können unter anderem Zellsysteme wie z.B. Bakterien wie Escherichia coli, Hefezellen wie Saccharomyces cerevisiae, Insektenzellen wie z.B. Spodoptera frugiperda (Sf-9) Zellen, oder Säugerzellen wie "Chinese Hamster Ovary" (CHO) Zellen verwendet werden. Diese Zellen sind von der "American Tissue Culture Collection" (ATCC) erhältlich. Zur rekombinanten Expression von Peptiden werden z.B. Nukleinsäuresequenzen, die für ganze MAT-Moleküle oder für einzelne Module von MAT-Molekülen kodieren in Kombination mit geeigneten regulatorischen Nukleinsäuresequenzen wie z.B. Selektionsmarkern, Promotoren, usw. mit molekularbiologischen Methoden in einen Expressionsvektor eingefügt. Geeignete Selektionsmarker sind z.B. Resistenzen gegen Antibiotika wie Ampicillin, Kanamycin, Neomycin, Puromycin oder Stoffwechseldefekte, z.B. Hefezellen, die kein Alanin, Leucin, Tryptophan usw. herstellen können oder Säugerzellen, denen das Enzym "hypoxanthine- guanine phosphoribosyltransferase" fehlt und die daher in HAT-Medium (Hypoxantine, Aminopetrin, Thymidin-Medium) nicht überleben können usw. Geeignete Promotoren sind z.B. der "cytomegalovirus immediate early promoter" (CMV-Promoter), der SP1-Minimal Promoter oder der Thymidin-Kinase Promoter (TK-Promoter). Bei der Auswahl der Promotoren müssen für das jeweilige Zellsystem geeignete Promotoren ausgewählt werden. Für Bakterien ist z.B. der T7 oder der T7/lacO Promoter geeignet, während für z.B. Hefezellen der nmt1 Promotor geeignet ist. Bei toxischen MAT-Molekülen oder Modulen von MAT-Molekülen kann es vorteilhaft oder erforderlich sein, Expressionsvektoren zu verwenden, die es ermöglichen die Expression dieser Moleküle von außen zu steuern, z.B. durch das Tet-On™ und das Tet-Off™ Expressionssystem (Promega Biosciences, San Louis, CA, USA). Bei diesem System wird die Aktivität des Promoters der Expressionsvektoren durch die Zugabe von Tetrazyklin zum Wachstumsmedium der Zellen reguliert. Weitere Beispiele für Methoden, die zur extern Regulierung der Expression von MAT-Molekülen oder von Modulen von MAT-Molekülen verwendet werden können ist die Induktion der T7 Polymerase durch IPTG oder die Verwendung von Ecdysone-Induzierbaren Expressionssystemen wie z.B. das "Complete Control^{®} Inducible Mammalian Expression System" (Stratagene, La Jolla, MO, USA). Bei Verwendung von Vektoren, die eine IRES ("internal ribosome entry site") Sequenz enthalten können auch mehrere Moleküle durch die Verwendung von nur einem Expressionsvektor gleichzeitig hergestellt werden (z.B. pLP-IRESneo-Vektor, Promega Biosciences, San Louis; CA, USA). Auf diese Weise können so z.B. zwei oder mehr Module eines MAT-Moleküls, die durch nicht-kovalente Bindungen miteinander interagieren sollen in passenden stöchiometischen Mengenverhältnissen zueinander parallel expremiert und eventuell auch parallel aufgereinigt werden. Verschiedene Firmen bieten kommerziell erhältliche Expressionvektoren für verschiedene Zellsysteme an, z.B. die Firmen Invitrogen, Qiagen, Stratagen, Clontech, Novagen, New England Biolabs, Pharmingen, Promega, Pharmacia, usw. Die so gewonnenen Expressionsvektoren können dann in geeignete Zellen, z.B. durch Elektroporation, Calziumphosphat-Co-Präzipitation, Liposomen-vermittelte Transfektion, usw., in einer dem Fachmann bekannten Art und Weise eingefügt werden. Alternativ können auch durch molekularbiologische Methoden hergestellte rekombinante Viren verwendet werden, die dann wiederum Zellen infizieren und bewirken, dass die infizierten Zellen MAT-Moleküle oder Module von MAT-Molekülen expremieren. Geeignete virale Expressionssysteme sind z.B. das Bacculovirus System, z.B. BacculoGold (BD Bioscience Pharmingen, Palo Alto, CA, USA), adenovirale Expressionssysteme wie z.B. ViraPort™ (Stratagene, La Jolla, CA, USA), retrovirale Expressionssysteme wie z.B. AdEasy (Stratagene, La Jolla, CA, USA) usw.

Alternativ zur Transfektion von Expressionsvektoren und zu viralen Expressionssystemen können auch in vitro-Translationssysteme verwendet werden, bei denen z.B. Kaninchen Retikulozytenlysate oder E. coli S30 Extrakte oder Weizenkeim Extrakte zur Synthese von MAT-Molekülen oder zur in vitro Synthese von Modulen von MAT-Molekülen verwendet werden, ohne dass lebende Zellen zur Expression notwendig sind.

### Zellsysteme zur Herstellung von MAT-Molekülen

Als Ausgangsmaterial zur Herstellung von MAT-Molekülen oder zur Herstellung von einzelnen Modulen von MAT-Molekülen können Zelllysaten oder Zellkulturüberstände verwendet werden. Zellsysteme können z.B. aus Bakterien wie z.B. E. coli, Bazillus, caulobacter, Pseudomonas oder Streptomyceten oder Hefen, wie z.B. Saccharomyces, Pichia oder Hansenula, oder Insektenzellen wie z.B. Sf-9, Sf-21 oder High Five, oder Säugerzellen, wie z.B. CHO-Zellen, COS-Zellen, 3T3-Zellen, BHK-Zellen, 293-Zellen usw., gewonnen werden. Durch die Verwendung von Signalsequenzen, die den Export von Proteinen aus dem Zellinneren in den extrazellulären Raum bewirken, kann gezielt das zu exprimierende Protein im Zellkulturmedium oder im periplasmatischen Raum von z.B. Bakterien angereichert werden. Eine weitere Quelle für Ausgangsmaterial zur Herstellung von MAT-Molekülen oder von Modulen von MAT-Molekülen können nicht-menschliche transgene Tiere, transgene Pflanzen, transgene Pilze oder transgene Mikroorganismen sein, in die Nukleinsäuren stabil oder transient eingebrancht wurden, die für MAT-Moleküle oder Module von MAT-Molekülen kodieren. Die entsprechenden Nukleinsäuren können dabei sowohl direkt ins Genom des jeweiligen Organismus integriert werden, als auch z.B. in Form von Plasmiden oder in Form von anderen DNA oder RNA-Molekülen in die Organismen eingebracht werden. Die entsprechenden MAT-Moleküle oder Module von MAT-Molekülen können dann z.B. aus der Milch, den Eiern, aus Serum, aus Urin, aus Gewebe usw. der transgenen Tiere, aus z.B. Speicherknollen, Samen, Blättern usw. von transgenen Pflanzen, aus z.B. dem Mycel, dem Fruchtkörper usw. der transgenen Pilze oder aus in vitro kultivierten Zellen oder anderen Organismen oder aus den entsprechenden Zellkulturmedien gewonnen werden. Generell sind alle Arten von Organismen geeignet, als Expressionssystem zur Herstellung von MAT-Molekülen oder von Modulen von MAT-Molekülen verwendet zu werden.

### Isolierung von MAT-Moleküle

Die so hergestellten MAT-Moleküle oder Module von MAT-Molekülen können mit Techniken, die dem Fachmann bekannt sind isoliert werden. Dazu können zahlreiche dem Fachmann bekannte Methoden zur Isolierung von Proteinen verwendet werden, wie z.B. Präzipitations-Methoden, Flüssigphasen-Trennmethoden, chromatographische Methoden usw. Zu den geeigneten Präzipitationsmethoden gehören u.a. Immunpräzipitation, Ammoniumsulfat-Fällung, Polyethylenglykol-Fällung, Ethanol-Fällung, Tri-Chlor-Essigsäure-Fällung (TCA-Fällung), Hitze-Fällung, usw. Zu den Flüssigphasen-Trennungsmethoden gehören z.B. die Extraktion mit organischen Lösungsmitteln wie z.B. Alkoholen, Aceton, Chloroform, Acetonitril, usw., zu den chromatographischen Methoden gehören z.B. Kationenaustauscher-Chromatographie, Anionenaustauscher-Chromatographie, isoelektrische Fokussierung, Reverse Phase Chromatographie, Gelfiltration, immobilisierte Metall-Ionenaffinitäts-Chromatographie (IMAC), wobei verschiedene Metallionen, wie z.B. Nickel, Zink, Kobalt usw. verwendet werden können, Hydroxyl-Apatit-Chromatographie, zahlreiche verschiedene Affinitäts-Chromatographie-Methoden wie z.B. Immunaffinitäts-Chromatographie, Affinitätschromatographie unter Verwendung von immobilisierten Nukleinsäuren, oder immobilisierten Proteaseinhibitoren, usw. Die verwendeten Chromatographie-Medien können auf Basis einer Agarosematrix, auf Basis von magnetischen Partikeln, in Form von Membranen, in Form von Hohlfasern, auf Basis verschiedener Polymere wie z.B. Polystyrol usw. aufgebaut sein. Chromatographiemethoden können generell in verschiedensten Maßstäben durchgeführt werden ausgehend von Chromatographiesäulen mit wenigen *µ*l Volumen bis zu großen Chromatographiesäulen mit mehreren 100 Liter Volumen. Außerdem können Chromatographien bei normalen atmosphärischen Druck, unter mittleren Drücken im Bereich von 1 bis 50 bar (z.B. das FPLC-System, Pharmacia Amersham Biosciences, Uppsala, Sweden) und bei sehr hohen Drücken im Bereich bis ca. 400 bar und eventuell noch größeren Drücken (HPLC-Systeme) durchgeführt werden. Chromatographien können unter Bedingungen durchgeführt werden, die nativ und denaturierend auf die MAT-Moleküle wirken. Bei der Affinitätschromatographie können verschiedene Interaktionen zwischen Matrixmaterial und einem zu isolierenden MAT-Molekül oder Modul eines MAT-Moleküls Verwendung finden. Darunter sind zahlreiche an anderer Stelle der vorliegenden Patentanmeldung bereits genannte Tag-Moleküle, die mit bestimmten funktionellen Gruppen oder Liganden interagieren und so eine Isolierung von MAT-Molekülen oder Modulen von MAT-Molekülen erlauben. Es können jedoch prinzipiell alle Arten von Interaktion verwendet werden, wie z.B. Protein - Protein Interaktionen, Nukleinsäure - Protein Interaktionen, Nukleinsäure - Nukleinsäure Interaktionen, Zucker - Lektin Interaktionen, Zucker - Protein Interaktionen, Rezeptor - Ligand Interaktionen, Antikörper - Antigen Interaktionen (z.B. anti-FLAG-, anti-HA-, anti-myc-Tag-Antikörper), Hapten-Antikörper Interaktionen, Spiegelmer-Interaktionen (NOXXON Pharma AG, Berlin, Germany) usw.

### Affinitäts-Chromatographie

Zur Affinitätschromatographie können insbesondere Methoden herangezogen werden, die auf der selektiven Bindung eines Tag-Moduls an eine Matrix beruhen. Geeignete Kombinationen von Tag-Modulen und zugehöriger Matrix zur Isolation eines MAT-Moleküls sind u.a.: Histidin-Tag und Nickel-Chelat Matrix (Qiagen, Hilden, Germany), GST-Tag und Glutathion-Sepharose (Amersham Biosciences, Uppsala, Sweden), Maltose-Bindendes Protein-Tag und Amylose-Matrix (New England Biolabs, Beverly, MA, USA), Biotin-Tag und Streptavidin- oder Avidin-Matix (IBA GmbH, Göttingen, Germany), Chitinbindendes Protein-Tag und Chitin-Matrix (New England Biolabs, Beverly, MA, USA), Calmodulin-binding peptide tag und Calmodulin-Matrix (Stratagene, La Jolla, CA, USA), Protein A oder Protein G oder Protein A/G oder Protein L und bestimmte von den jeweiligen Proteinen erkannten Bereiche von Antikörpermolekülen, wie z.B. der Fc-Anteil von Antikörpern (Amersham Biosciences, Uppsala, Sweden), FLAG-Tag, HA-Tag, myc-Tag, Histidin-Tag, usw. und eine Matrix, an die ein Antikörper gegen das jeweilige Tag gekoppelt ist (viele verschiedene Firmen, u. a. Promega Biosciences Inc., San Louis Obispo, CA, USA, Invitrogen, Breda, The Netherlands, Qiagen, Hilden, Germany) usw.

### Protease-Erkennungssequenzen

Die so isolierten MAT-Moleküle oder Module von MAT-Molekülen können gegebenenfalls von ihrem Tag-Modul und/oder anderen Modulen getrennt werden. Dazu kann z. B. eine Protease-Erkennungssequenz an geeigneten Positionen in den jeweiligen Expressionsvektor eingefügt werden. Zahlreiche geeignete Protease-Erkennunssequenzen sind dem Fachmann bekannt, u. a. die Erkennungssequenzen von Proteasen wie z. B. Thrombin, Faktor Xa, Enterokinase oder das TAGZyme-System (Qiagen, Hilden, Germany). Die zugegebenen Proteasen können z. B. durch immobilisierte Proteaseinhibitoren wie z. B. EK-AWAY für Enterokinase (Invitrogen, Breda, The Netherlands), Xa Removal Resin (Qiagen Hilden, Germany), Benzamidin-Sepharose zur Entfernung von Thrombin, usw. wieder entfernt werden. Eine weitere Möglichkeit zur Isolierung von MAT-Molekülen oder Modulen von MAT-Molekülen ist die Verwendung von Inteinen, d. h. von Proteasen, die Bestandteil des MAT-Moleküls sind und die sich dann unter geeigneten experimentellen Bedingungen selbst proteolytisch vom Rest des MAT-Moleküls oder eines Moduls eines MAT- Moleküls abspalten (Genease", New England Biolabs, Beverly, MA, USA). Generell sind alle derzeit bekannten [29,30] und zukünftig bekannt werdenden Protease-Erkennungssequenzen geeignet, im Sinne der Erfindung Bestandteile von MAT-Molekülen zu entfernen. Die Protease-Erkennungssequenzen können dabei sowohl natürlich in der Natur vorkommen oder gezielt entworfen worden sein und können ganz oder in Teilen aus natürlichen Aminosäuren, ungewöhnlichen Aminosäuren, Peptidomimetics usw. zusammengesetzt sein.

### Inclusion Bodies

Eine weitere Ausführungsform der Erfindung ist die Verwendung von MAT-Molekülen oder von Modulen von MAT-Molekülen in Form von nicht korrekt gefalteten Protein-Aggregaten, die auch als"inclusion bodies"bezeichnet werden. Inclusion bodies können als Moleküle hergestellt werden, die Translokations-Module enthalten, die einen Transport vom extrazellulären Raum durch die Zellmembran ins Zellinnere ermöglichen. Diese Translokation führt dazu, dass die ursprünglich nicht korrekt gefalteten Moleküle korrekt gefaltet werden und dann intrazellulär so wirken wie ein von Anfang an korrekt gefaltetes MAT-Molekül. Diese Vorgehensweise hat den Vorteil, das ungefaltete

Proteine unter denaturierenden Bedingungen isoliert werden können, was oftmahls mit geringeren technischen und damit finanziellen Aufwand verbunden ist. Außerdem sind "inclusion bodies" relativ stabile Strukturen. Dieses bringt unter Umständen Vorteile bei der Lagerung und Haltbarkeit von MAT-Molekülen die zum späteren medizinischen Einsatz bereitgehalten werden.Durch dieses Verfahren können auch ungefaltete oder nicht korrekt gefaltete MAT-Moleküle oder Module von MAT-Molekülen im Sinne der Erfindung verwendet werden. Bestimmte Translokations-Module bewirken sowohl eine Translokaton vom extrazellulären Raum in die Zelle, als auch den umgekehrten Transport. Die Umwandlung eines noch nicht korrekt gefalteten MAT-Moleküls kann direkt in dem mit dem MAT-Molekül zu behandelnden Individuum in vivo stattfinden, oder die Faltung des MAT-Moleküls kann in vitro in einem Zellsystem durchgeführt werden. Außerdem kann die Translokation des ungefalteten MAT-Moleküls ins Zellinnere und die anschließende Translokation des dann korrekt gefalteten MAT-Moleküls in den extrazellulären Raum unter Umständen von dem gleichen Translokations-Modul bewirkt werden. Für einige Sequenzen, die als Translokations-Modul im Sinne dieser Erfindung geeignet sind, wie z.B. die VP22-Sequenz, ist ein solcher Mechanismus beschrieben [39].

### Modifizierung von MAT-Molekülen

MAT-Moleküle oder Module von MAT-Molekülen können über zahlreiche dem Fachmann bekannte Verfahren enzymatisch, chemisch oder über andere Verfahren modifiziert werden. Zum Beispiel können Peptide unter Verwendung von Kinasen mit Phosphorgruppen versehen werden, unter Verwendung von Phosphatasen können Phosphorgruppen entfernt werden, Zuckerstrukturen können unter Verwendung von Glykosidasen entfernt werden, usw. Entsprechende Kinasen, Phosphatasen und Glykosidasen usw. und die entsprechenden Protokolle sind von verschiedenen Herstellern, wie z.B. New England Biolabs, Beverly, MA, USA, erhältlich. Die Phosphorylierung von MAT-Molekülen oder von Modulen von MAT-Molekülen kann außerdem dazu verwendet werden, MAT-Moleküle oder Module von MAT-Molekülen mit radioaktivem Phosphor zu markieren, wodurch diese dann leicht in vitro und/oder in vivo nachweisbar werden. Auch durch chemische Reaktionen können MAT-Moleküle oder Module von MAT-Molekülen modifiziert werden. Zum Beispiel können Disulfidbrücken durch Reduktion zerstört werden, es können Thioestergruppen kovalent mit Cysteinresten reagieren, oder zwei Cysteinreste zu einer Disulfidbrücke reagieren, wodurch zirkuläre oder verzweigte MAT-Moleküle oder Module von MAT-Molekülen hergestellt werden können (z.B. "IMPACT™-TWIN Protein Fusion and Purification System", New England Biolabs, Beverly, MA, USA). Auch durch die Auswahl des Expressionssystems lassen sich Modifizierungen des MAT-Moleküls oder von Modulen von MAT-Molekülen gezielt beeinflussen. Zum Beispiel findet in Bakterien keine Glykosylierung statt und Insektenzellen synthetisieren nur bestimmte Arten von Glykosylierungen, während Zellen von Säugetieren komplette Glykosylierungen herstellen. Es können zur Expression auch Zelllinien verwendet werden, die derart modifiziert wurden oder derart ausgewählt wurden, dass sie bestimmte postranslationale Modifizierungen gezielt herstellen, bzw. gezielt nicht herstellen können. Diese vorteilhaften Eigenschaften können unter anderem eine bessere Löslichkeit in hydrophilen oder hydrophoben Lösungsmitteln, eine längere Haltbarkeit des MAT-Moleküls bei z.B. bei Raumtemperatur, bei 37°C, bei 4°C, bei -20°C, bei -70°C oder bei anderen Temperaturen, eine längere molekulare Stabilität der MAT-Moleküle, wenn sie allein oder in Gemischen mit anderen festen, flüssigen, oder gasförmigen Substanzen, z.B. in Form von Zubereitungen als Medikament oder Diagnostikum vorliegen, eine höhere Durchlässigkeit der MAT-Moleküle für Zellmembranen, für Membranen von Organellen, für die Blut-Hirn-Schranke, für die Blut-Liquor-Schranke und für andere biologische Membranen und Barrieren, usw., eine höhere oder geringere in vivo oder in vitro Halbwertszeit, eine geringere oder höhere Toxizität, eine bessere in vivo oder in vitro Nachweisbarkeit des MAT-Moleküls usw. sein.

### Protein-Ligation

Eine weitere Möglichkeit, MAT-Moleküle oder Module von MAT-Molekülen herzustellen, ist die Protein-Ligation. Darunter versteht man z.B. eine chemische Reaktion, bei der zwei Enden von Peptiden über eine oder mehrere chemische Reaktionen kovalent miteinander verknüpft werden. Ein Beispiel wäre die Reaktion eines Thiolesters mit einer Cysteinseitenkette (z.B. "IMPACT™-TWIN Protein Fusion and Purification System", New England Biolabs, Beverly, MA, USA). Auf diese Weise lassen sich z.B. zyklische Peptide herstellen. Verzweigte MAT-Moleküle lassen sich z.B. durch chemische Synthese von Polylysin-Peptiden herstellen, bei denen an jedes Lysin je zwei weitere Lysinreste (je ein Lysin an jede Aminogruppe des Lysins) angefügt werden und so ein verzweigtes Polylysin-Peptid entsteht. Anschließend kann dann an jedes endständige Lysin eine Peptidkette synthetisiert werden, bzw. durch Peptid-Ligation ein Peptid kovalent angefügt werden. Auch andere verzweigte Polymere können als Träger-Struktur für MAT-Moleküle oder Module von MAT-Moleküle im Sinne der Erfindung verwendet werden. Ein Beispiel hierfür sind z.B. "PEG star" Moleküle die durch Polymerisation von Ethylenoxid mit quervernetztem Divinylbenzene hergestellt werden können.

### Peptidomimetics

Eine weitere Möglichkeit zur Herstellung von MAT-Molekülen oder Modulen von MAT-Molekülen ist die chemische Synthese von Peptiden oder Peptidomimetics [40] oder von Kombinationen aus Peptiden und Peptidomimetics. Die Herstellung von MAT-Molekülen oder von Modulen von MAT-Molekülen durch chemische Synthese kann z.B. nach dem Merrifield-Festphasen-Syntheseprotokoll unter Verwendung von Syntheseautomaten und Synthesechemikalien, die von verschiedenen Herstellern erhältlichen sind, erfolgen. Ein Beispiel für eine Firma, die Synthesen von Peptidomimetics anbietet ist "The Peptide Laboratory^{®}", Benicia, CA, USA. Zahleiche Synthesebausteine für herkömmliche Peptide und für Peptidomimetics können z.B. von Sigma-Aldrich Co, St. Louis, MO, USA erworben werden.

### Zusammensetzung von Medikamenten, und Diagnostika

Die in den Medikamenten und Diagnostika enthaltenen Peptid-Anteile, Aminosäure-Anteile, Aminosäurederivat-Anteile, Peptidomimetika-Anteile usw. der MAT-Moleküle können auch in Form ihrer Salze vorliegen, solange es sich bei diesen Salzen um pharmakologisch akzeptable Salze handelt. Medikamente oder Diagnostika, die zur Injektion vorgesehen sind, können z.B. sterile wässrige oder ölartige Lösungen sein, die entsprechend dem Stand der Technik mit geeigneten Hilfsstoffen wie z.B. Dispersionsmitteln, Befeuchtungsmitteln und Mitteln, die Suspensionen stabilisieren, versetzt sind. Die sterilen Injektionslösungen können mit pharmakologisch akzeptablem Verdünnungs- oder Lösungsmitteln, wie z.B. 1,3-Butandiol, hergestellt werden. Unter den akzeptablen Lösungsmitteln und Puffern, die verwendet werden können, sind unter anderem Wasser, Ringerlösung, isotonische Natriumchlorid Lösungen, usw. Zusätzlich können sterile Öle, einschließlich synthetischer Mono- oder Diglyceride verwendet werden. Zusätzlich können Fettsäuren wie z.B. Ölsäure zur Präparation der Injektionslösungen heran gezogen werden. Weiterhin können Dimethylacetamid, Detergentien einschließlich ionischer und nicht-ionischer Detergentien, Polyethylenglykole, usw. benutzt werden. Gemische aus den oben genannten Substanzen sind ebenfalls möglich. Außerdem können Medikamente auch in Form von Gemischen mit biologisch abbaubaren Polymeren hergestellt werden, die die Medikamente kontinuierlich freisetzen. Ein Beispiel für ein solches System ist z.B. das Atrigel-System (Atrix Labs, Fort Collins, CO, USA).

Für rektale Applikationen können Präparationen herangezogen werden, die aus Gemischen bestehend aus MAT-Molekülen und gegebenenfalls weiterer Substanzen mit einem geeigneten, nicht-reizenden Salbengrund- oder Füllstoff wie z.B. Kakaobutter, synthetischen Mono-, Di-, oder Triglyceriden, Fettsäuren oder Polyethylenglykol bestehen. Außerdem können Farbstoffe, Konservierungsstoffe und Geruchsstoffe enthalten sein. Diese und weitere geeignete Substanzen sind bei Raumtemperatur fest und schmelzen bei Körpertemperatur, so dass sie die enthaltenden Substanzen freigesetzt werden.

Zur oralen Applikation können u.a. Kapseln, Tabletten, Pillen, Pulver, Granulate usw. verwendet werden. In solchen Darreichungsformen sind die Wirksubstanzen der Medikamente und Diagnostika oft mit Adjuvantien, die für die jeweilige Darreichungsform geeignet sind, kombiniert. Die Substanzen können mit Laktose, Saccharose, Stärkepulver, Celluloseestern, Alkansäuren, Cellulosealkylestern, Stearinsäure, Magnesium Stearat, Magnesium-Oxid, Natrium- oder Kalziumsalzen von Phosphoriger oder Schwefliger Säure, Gelatin, Gummi arabicum, Natrium-Alginat, Polyvinylpyrrolidone, Polyvinylalkohol usw. zu z.B. Tabletten, Kapseln usw. verarbeitet werden. Solche Kapseln, Tabletten, usw. können zusätzlich Substanzen enthalten, die eine kontrollierte Freisetzung der Wirksubstanzen ermöglichen oder fördern, wie z.B. Hydroxypropylmethylcellulose. Außerdem können Puffersubstanzen wie Natrium-Citrat, Magnesium- oder Kalzium-Carbonate oder -Bicarbonate, usw. enthalten sein. Weitere Bestandteile können Farb-, Duft-, Geschmacks-, Konservierungs- und Süßstoffe sein. Tabletten, Pillen, Kapseln, usw. können zusätzlich noch Beschichtungen erhalten, die sie zum einen resistent gegen Magensäure machen, die zum anderen aber bewirken, dass sie sich im alkalischen Milieu des Darms lösen.

Zur oralen Applikation können auch flüssige, pharmazeutisch akzeptable Emulsionen, Lösungen, Sirups und gelartige Präparationen verwendet werden. Diese Präparationen können Lösungsmittel enthalten, die in der Medizin verwendet werden, wie z.B. Wasser, Ethanol, usw. Diese Präparationen können auch Adjuvantien, Befeuchtungsmittel, Emulsions- und Suspensionsmittel, usw. sowie Süßstoffe, Geschmacksstoffe, Farbstoffe, Konservierungsstoffe und Geruchsstoffe enthalten.

Flüssige Präparationen, die zu Injektionszwecken vorgesehen sind, können aus sterilen Pulvern oder aus Granulat durch Lösung in wässrigen oder nicht- wässrigen Lösungsmitteln hergestellt werden. Die diesen Lösungen zugrunde liegenden Pulver und Granulate können eine oder mehrere der für oral applizierbare Medikamente genannten Substanzen enthalten. Geeignete Lösungsmittel sind u. a. Wasser, Polyethylenglykol, Polypropylenglykol, Ethanol, Maisöl, Baumwollsamenöl, Kokosöl, Benzylalkohol, Natriumchloridlösungen oder verschiedene andere Puffer. Als weitere Bestandteile sind Farbstoffe, Konservierungsstoffe usw. möglich.

Die Menge an MAT-Molekülen und weiteren Inhaltsstoffen der Medikamente hängt von der Darreichungsform, barreichungshäufigkeit, dem gewählten Applikationsweg, dem Alter, Geschlecht, Gewicht und dem gesundheitlichen Zustand des Patienten, usw. ab. Außerdem ist zu berücksichtigen, ob die Behandlung zur Diagnose, Therapie oder zur Prophylaxe durchgeführt wird und ob eine Verstärkung der Immunreaktion oder eine Dämpfung der Immunreaktion durch die Behandlung erzielt werden soll. Zur Formulierung und Dosierung von Medikamenten sind dem Fachmann zahlreiche Arbeiten bekannt [41,42].

Applikationswege für erfindungsgemäßen Medikamente und Diagnostika Die erfindungsgemäßen Medikamente und Diagnostika können über verschiede Wege dem Patienten oder einem zu immunisierenden Versuchstier verabreicht werden. Zu diesen Methoden zählen unter anderem die orale und die sublinguale Gabe, z. B. in Form von Tabletten, Dragees, Kapseln, Granulat, flüssige Lösungen, in Flüssigkeiten zu lösenden Pulvern usw., wobei z. B. Dragees, Tabletten, Granulate und Kapseln gegebenenfalls derart zusammengesetzt sein können, das die Medikamente ohne Einwirkung des sauren Milieus des Magens in den Darm gelangen und die Bestandteile der Medikamente erst dort freigesetzt werden. Außerdem können die Medikamente durch topische Anwendung auf der Haut oder auf Schleimhäuten z. B. in Form von Salben, Sprays, Pudern, Tinkturen usw., oder inhaliert als Aerosol über die Schleimhäute z. B. der Atemwege verabreicht werden. Auch die rektale Applikation in Form von Zäpfchen, Klysmen, usw. ist möglich. Bei der transdermalen Applikation der Medikamente können auch Hilfsmittel wie z. B. Dendritische Zellen, B-Lymphozyten, Makrophagen, weitere makrophagenähnliche Zellen, usw. Als weiteres Beispiel können Zellen von Versuchstieren, oder Zelllinien mit MAT-Molekülen behandelt werden. Die behandelten Zellen können dann anschließend dem Patienten oder dem Versuchstier verabreicht werden. Die Zellen können als lebende Zellen, als inaktivierte nicht mehr teilungsfähige Zellen, oder als abgetötete Zellen dem Patienten oder dem Versuchstier verabreicht werden. Inaktivierte oder abgetötete Zellen können z.B. durch Behandlung mit geeigneten Substanzen oder durch Bestrahlung, z.B. mit radioaktiver oder ultravioletter Strahlung, erhalten werden. Eine weitere Möglichkeit der Applikation der Medikamente, insbesondere der erfindungsgemäßen MAT-Moleküle ist die stabile oder transiente Transfektion von tierischen, menschlichen oder pflanzlichen Zellen mit einem Vektor, der zur Expression eines MAT-Moleküls führt. Die so modifizierten Zellen können gegebenenfalls in eine Matrix eingeschlossen werden, die sie zum einen lokal fixiert und zum anderen vor dem Immunsystem des Patienten oder des Versuchstiers schützt, die aber auf der anderen Seite jedoch die von den Zellen freigesetzten MAT-Moleküle in den Organismus des Patienten oder Versuchstiers entweichen lässt. Unter Umständen können die transfizierten Zellen auch direkt dem Patienten oder Versuchstier verabreicht werden, wobei die Zellen gegebenenfalls derart behandelt werden, dass sie sich nicht mehr teilen können, z.B. durch Bestrahlung oder durch Behandlung mit geeigneten Chemikalien. Außerdem können die Medikamente verpackt in Liposomen, oder anderen Vesikeln, wie z.B. Exosomen, oder Deoxosomen verabreicht werden, oder die Medikamente können in Form von Gemischen mit biologisch abbaubaren Polymeren verabreicht werden, die die Medikamente kontinuierlich freisetzen. Ein Beispiel für ein solches System ist z.B. das Atrigel-System (Atrix Labs, Fort Collins, CO, USA). Außerdem können über den gleichen oder über einen oder mehrere andere Applikationswege gleichzeitig oder zeitlich versetzt mit der Gabe des erfindungsgemäßen Medikaments oder Diagnostikums weitere Substanzen verabreicht werden. Diese weiteren Substanzen können unter anderem über ihre immunstimulatorischen Eigenschaften die Wirkung der erfindungsgemäßen Medikamente oder Diagnostika verbessern. Solche gleichzeitig oder zeitlich versetzt gegebenen Substanzen können unter anderem Adjuvantien, Mineralöl, Freund'sches Adjuvans, immunstimulatorische Proteine oder Mediatoren wie z.B. Zytokine, andere Impfstoffe usw. sein. Außerdem ist gegebenenfalls die gleichzeitige oder zeitlich versetzte Gabe von immunsupprimierenden Substanzen sinnvoll, um z.B. ungewollte lokale Immunreaktionen zu vermindern oder zu unterdrücken, während systemische Immunreaktionen erhalten bleiben. Die gleichzeitige oder zeitlich versetzte Gabe von immunsupprimierenden Substanzen kann aber auch umgekehrt verwendet werden, um systemische Immunreaktionen zu verhindern, während gleichzeitig die lokalen Immunreaktion erhalten bleiben.

### Möglichkeiten zur Überprüfung der Wirksamkeit von MAT-Molekülen

Zur Überprüfung der Wirksamkeit von MAT-Molekülen in Bezug auf eine Modulation der Immunantwort eines Individuums können verschiedene in vitro und in vivo Experimente durchgeführt werden.

Als in vitro Modell eignen sich z.B. periphere Blut mononukleare Zellen (PBMCs), z.B. gewonnen aus dem Blut von Patienten, die an allergischen Erkrankungen leiden. Solche Zellen haben den Vorteil, dass häufig das genaue Antigen, gegen das der jeweiligen Patient allergisch reagiert, bekannt ist. Dieses Wissen ermöglicht z.B. eine Desensibilisierung des Patienten in vitro zu simulieren, bevor klinische Studien am Patienten oder in Versuchstieren durchgeführt werden. Dazu können z.B. die PBMCs von Allergikern mit dem jeweiligen Antigen, gegen das der Allergiker reagiert, oder mit einem MAT-Molekül, das das jeweilige Allergen im Antigen-Modul beinhaltet, behandelt werden. So kann die immunologische Reaktion der primären Patientenzellen auf verschiedene Darreichungsformen (komplettes MAT-Molekül, Moleküle mit/ohne Translokations-Modul, Moleküle mit/ohne Targeting-Modul usw.) des Allergens untersucht werden. Als Messparameter bieten sich unter anderem Zytokinbestimmungen im Zellkulturüberstand an. An der Immunantwort gegen ein Antigen sind verschiedene Arten von T-Zellen beteiligt, wie z.B. T-Helfer Zellen vom Typ 1 (Th1 Zellen) oder vom Typ 2 (Th2 Zellen) oder vom Typ 0 (Th0 Zellen). Die Art der jeweils beteiligten T-Zellen hat großen Einfluss darauf, ob eine Immunantwort gegen das Antigen ausgelöst wird, die primär aus Immunglobulinen der Klasse E (IgE) oder aus Immunglobulinen der Klasse G (IgG) besteht. Aus der Literatur ist bekannt, das insbesondere IgE-Immunantworten für allergische Reaktionen und Asthma verantwortlich sind, während IgG-Immunantworten in der Regel mit einer Toleranz gegen das Antigen verbunden sind. Der jeweilige T-Zelltyp, der durch die Behandlung der PBMCs mit einem Antigen aktiviert wird kann z.B. auch durch die Bestimmung der Expression von Oberflächenantigenen auf der Zelloberfläche oder durch Bestimmung von Botenstoffe wie z.B. Zytokinen, die von den PBMCs freigesetzt werden, bestimmt werden. Als Marker für eine Th1 Immunantwort können z.B. Interferon-Gamma (INF-g) oder Interleukin-1 (IL-1) im Zellkulturmedium bestimmt werden, während IL-4, IL-5, IL-6, IL-10 und IL-13 eine Th2 Immunantwort anzeigen. Diese Zytokine können über dem Fachmann bekannte Standardmethoden wie z.B. ELISA-Bestimmungen aus z.B. den Zellkulturüberständen oder FACS-Analysen der oberflächlich oder intrazellulär vorhandenen Botenstoffe in den PBMCs, oder über Westernblots von Zellkulturüberständen oder Zelllysaten usw. nachgewiesen werden. Zahlreiche andere Methoden sind in der Literatur bekannt, die zum Nachweis dieser oder weiterer Botenstoffe geeignet sind [43, 44]. Neben den von den PBMCs freigesetzten Botenstoffen können auch intrazelluläre oder Membranständige Botenstoffe oder weitere Proteine zur immunologischen Charakterisierung der T-Zellen in PBMCs heran gezogen werden. Unter anderem bieten die Firmen Pharmingen, San Diego, CA, USA, Beckmann Coulter Inc., Fullerton, CA, USA, Santa Cruz Biotechnology Inc., Santa Cruz, CA, USA, usw. zahlreiche Antikörper an, die für solche Untersuchungen geeignet sind. Entsprechende Untersuchungen können jedoch nicht nur mit primären Zellen von Patienten durchgeführt werden, sondern auch mit Zellen, die von entsprechend behandelten Versuchstieren, z.B. Mäusen, Ratten, Meerscheinchen, usw. erhalten werden. Experimente mit Versuchstieren haben dabei den Vorteil, dass nicht nur in vitro mit den Zellen dieser Tiere gearbeitet werden kann, sondern dass das Immunsystem in vivo im Kontext eines intakten Organismus untersucht werden kann. Dadurch lassen sich auch die Auswirkung der Dosierung, Zusammensetzung und der Applikationsform der MAT-Moleküle sowie entsprechender Kontrollen wie z.B. Moleküle die nur aus einem Antigen oder die nur aus einem Translokations-Modul und einem Antigen-Modul oder die nur aus einem Targeting-Modul und einem Antigen-Modul bestehen, untersuchen. Es kann u.a.untersucht werden, ob es Unterschiede in der Art der Immunantwort gibt, wenn MAT-Moleküle oder entsprechende Kontrollen auf konventionelle Art und Weise subkutan injiziert werden oder wenn die Injektion z.B. direkt in Lymphknoten erfolgt. Auch nicht invasieve Verabreichungen, wie z.B. oral oder sublinguale Applikation der MAT-Moleküle und entsprechender Kontrollen, können auf ihren Effekt untersucht werden. Eine weitere Untersuchung, die am Versuchstier durchgeführt werden kann, ist die Applikation von MAT-Molekülen oder entsprechenden Kontrollen mit und ohne gleichzeitiger Gabe von Adjuvantien wie z.B. Mineralöl, Mycobakterienextrakte oder Freund'sches Adjuvans. Auch das wirksamste oder verträglichste Zeitschema für die durchzuführenden Immunisierungen, sowie Dosis und Anzahl der Immunisierungen können ermittelt werden. Weiterhin können verschiedene Antigen-Module auf ihre Wirksamkeit getestet werden. Auf diese Weise kann die beste Immunisierungs-Strategie für spätere Studien an humanen Patienten ermittelt werden.

Neben Botenstoffen, intrazellulären Proteinen und Oberflächenproteinen sind insbesondere auch Immunglobuline zur Charakterisierung der Immunreaktion von in vitro kultivierten Zellen oder zur Charakterisierung der Immunreaktion von Versuchstieren oder von humanen Patienten in klinischen Studien geeignet. Es kann z.B. Unter Verwendung von ELISAs untersucht und quantifiziert werden, ob die aufgrund der Verabreichung des Allergens oder Antigens freigesetzten Antikörper vom IgE- oder vom IgG-Typ sind. Diese Informationen würden Aufschluss darüber geben, um welche Art von Immunreaktion es sich handelt.

Da die von den T-Zellen freigesetzten Botenstoffe unter anderem auch einen Einfluss auf die Proliferation von Zellen des Immunsystems haben, die u.a. in PBMC-Präparationen vorhanden sind, kann auch über die Bestimmung der Proliferation von Zellen wie z.B. PBMCs die Immunantwort charakterisiert werden. Dazu können z.B. in vitro Untersuchungen durchgeführt werden, wie z.B. DNA-Einbaustudien mit tritiummarkierten Thymidin, als Nachweis für Zellwachstum. Zahlreiche andere dem Fachmann bekannte Methoden zur Bestimmung von Zellproliferation können ebenfalls bei diesen Untersuchungen angewendet werden. Auch in vivo kann die Proliferation bestimmter Zellen des Immunsystems bestimmt werden, indem z.B. FACS-Untersuchungen mit Blutproben von Versuchstieren oder humanen Patienten, die an Studien teilnehmen, durchgeführt werden. Durch die Auswahl geeigneter Antikörper können z.B. verschiedene Subpopulationen von im Blut vorhandenen Zelltypen quantifiziert werden. Auf diese Weise kann die Wirkung einer Behandlung mit MAT-Molekülen oder entsprechenden Kontrollen untersucht werden.

### Ausführungsbeispiele

Die nachfolgenden Ausführungsbeispiele sind zur exemplarischen Illustration der Erfindung gedacht, sollen aber keinesfalls den Schutzbereich der Erfindung eingrenzen.

### Beispiel 1:

### Klonierung von Expressionsvektoren für MAT-Moleküle

Alle nachfolgend beschriebenen molekularbiologischen Verfahren wurden entsprechend dem Fachmann bekannten Standard-Methoden durchgeführt [43]. Zur Klonierung eines Vektors zur Expression der MAT-Moleküle (modulare Antigen-Transport-Moleküle) wurde der Vektor pQE-30 (Qiagen, Hilden, Germany) verwendet.

In einem ersten Arbeitsschritt wurde eine Nukleinsäuresequenz, die für ein Translokations-Modul kodiert, in einen bakteriellen Expressionsvektor eingefügt. Die DNA-Sequenz, die für die Aminosäuren GYGRKKRRQRRR von HIV-Tat kodiert, wurde über synthetische Oligonukleotide in den Vektor pQE-30 eingefügt. Die Oligonukleotide enthielten zusätzlich zur HIV-Tat Sequenz am 5'-Ende eine Erkennungssequenz der Restriktionsendonuklease Bgl II und am 3'-Ende Erkennungssequenzen für BamH I, Spe I, Pst I und Hind III. Die synthetisch hergestellte HIV-Tat Sequenz wurde anschließend mit Bgl II und Hind III und der Vektor pQE30 wurde mit den Restriktionsendonukleasen Bam HI und Hind III geschnitten. Die Tat-Sequenz und der Vektor pQE-30 wurden anschließend mit NucleoSpin Extract 2 in 1 (Macherey-Nagel, Oensingen, Schweiz) isoliert, unter Verwendung von Ligase zusammengefügt, in kompetente Bakterien durch Elektroporation transformiert und auf Ampizilinhaltigen Agarplatten ausplattiert. Von den resultierenden Bakterienkolonien wurden einige ausgewählt und aus diesen Vektor-DNA isoliert. Die so erhaltenen Vektoren wurden zur Bestätigung der Nukleinsäure-Sequenz unter Verwendung von Standardmethoden sequenziert. Bakterienklone, die Vektoren mit der korrekten Sequenz enthielten, wurden für weitere Arbeiten verwendet. In einem zweiten Arbeitsschritt wurde ein Targeting-Modul in den Vektor eingefügt. Dazu wurde mRNA aus humanen peripheren mononuklearen Zellen (PBMCs) unter Verwendung von dem Fachmann bekannten Standardmethoden isoliert und über Reverse-Transkriptase PCR in komplementäre DNA (cDNA) umgeschrieben. Unter Verwendung der so gewonnenen cDNA und unter Verwendung von verschiedenen PCR-Primern, die am 5'-Ende des PCR-Produkts eine Bgl II Erkennungssequenz und am 3'-Ende der PCR-Produkte Spe I, Bam H I, Pst I und Hind III Erkennungssequenzen einfügten, wurde die Nukleinsäuresequenz der humanen Invarianten Kette des MHC II-Moleküls gewonnen, die für die Aminosäuren 1 bis 110 der Invarianten Kette von MHC II kodiert. Dieser Sequenzbereich der Invarianten Kette des MHC II wurde, entsprechend dem ersten Arbeitsschritt, hinter das 3'-Ende der bereits in den pQE-30 Vektor eingefügten Tat-Sequenz eingefügt. Die Sequenz des resultierenden Vektors wurde durch Sequenzierung bestätigt. Unter Verwendung von dem Fachmann bekannten Standardmethoden wurde durch ortsgerichtete Mutagenese an Position 292 Cytosin gegen Thymidin und an Position 318 Guanin gegen Adenin ausgetauscht. Beide Punktmutationen führen nicht zu Änderungen in der Aminosäuresequenz des korrespondierenden Proteins, sondern entfernen lediglich unerwünschte Erkennungssequenzen von Restriktionsendonukleasen. In einem dritten Arbeitsschritt wurde schließlich ein Antigen-Modul in den Vektor eingefügt. Unter Verwendung des pQE-30 Vektors, in den zuvor die kodierende Sequenz des jeweiligen Antigens eingefügt wurde und unter Verwendung von PCR-Primern, die am 5'-Ende des PCR-Produkts eine Spe I und/oder BamH I Erkennungssequenz und am 3'-Ende des PCR-Produkts ein Stop-Codon und eine Pst I oder eine Hind III Erkennungssequenz einfügen, wurden Nukleinsäuresequenzen von verschiedenen Antigenen gewonnen. Unter anderem wurde als Antigen-Modul die Nukleinsäuresequenz, die für die Aminosäuren 1 bis 222 des Antigens Der p 1 kodiert (bezogen auf die Aminosäuresequenz des maturen Der p 1 Proteins) gewonnen. Dieser Sequenzbereich des Antigens Der p 1 wurde, entsprechend den ersten beiden Arbeitsschritten, hinter das 3'-Ende der bereits in den pQE-30 Vektor eingefügten Sequenz der Invarianten Kette des MHC II eingefügt. Die korrekten Sequenzen der resultierenden Vektoren wurde durch Sequenzierungen bestätigt. Für nachfolgende Arbeiten wurden die Expressions-Vektoren unter Verwendung von QIAfilter Plasmid Midi-Kits (Qiagen, Hilden, Germany) entsprechend dem Protokoll des Herstellers gewonnen.

### Beispiel 2:

### Gewinnung der codierenden Sequenzen der Antigene Bet v 1, Asp f 1, Asp f 6 und Der p 1

Die codierenden Sequenzen der verschiedenen Antigene in den Antigen-Modulen wurden über verschiedene dem Fachmann bekannte Methoden isoliert [43]. Die Bet v 1-Sequenz wurde über synthetische Oligonukleotide erhalten, die Asp f1 und Asp f6 Sequenzen wurden in früheren Studien erhalten und aus den in diesen Studien verwendeten Vektoren gewonnen [45, 46] und anschließend in den pQE-30 Vektor eingefügt und die Der p 1-Sequenz wurde über Reverse-Transkriptase PCR unter Verwendung von mRNA, die aus Hausstaubmilben (Dermatophagoides pteronyssinus) gewonnen wurde, isoliert.

### Beispiel 3:

### Expression und Isolierung von MAT-Molekülen in Bakterien

Die Expression und Isolierung der MAT-Moleküle wurde entsprechend den Herstellerangaben (Qiagen, Hilden, Germany) durchgeführt. Im Detail wurde eine Vorkultur von E. coli M15 Bakterien, die mit dem jeweiligen Expressionsvektor (pQE-30-MAT-Molekül) transfiziert waren, in 20 ml Medium (2x YT-Medium, 100*µ*g/ml Ampicillin, 25 *µ*g/ml Kanamycin) über Nacht bei 37 °C im Bakterienschüttler angesetzt. Die Vorkultur wurde dann in 2000 ml Medium (2x YT-Medium, 100*µ*g/ml Ampicillin, 25 *µ*g/ml Kanamycin) bei 37 °C im Bakterienschüttler so lange kultiviert, bis sie eine optische Dichte von 0,6 bei einer Wellenlänge von 600 nm erreicht hatte. Nach Zugabe von IPTG in einer Endkonzentration von 1 mM und einer erneuten Wachstumsphase von 4 bis 15 h wurden die Bakterien durch Zentrifugation bei 2000 g für 20 Minuten vom Kulturmedium getrennt und das Bakterienpellet bei -20 °C gelagert. Zur Herstellung von Zelllysaten wurde das Bakterienpellet aufgetaut, in 8 M Harnstofflösung (5 ml Hanstofflösung pro Gramm Nassgewicht der Bakterienpellets) resuspendiert, 1 bis 2 h vorsichtig gerührt und anschließend 30 min. bei 48000 g zentrifugiert. Der klare Überstand wurde zur präparativen Nickel-Chelat Chromatographie der MAT-Moleküle verwendet. Zur Isolierung der MAT-Moleküle wurden 49 oder 18 ml Säulen (Bio-Rad Laboratories Inc., Hercules, CA, USA), gefüllt mit 5 bis 10 ml NI-NTA Superflow Matrix (Qiagen) und ein Chromatographiesystem der Firma Bio-Rad (Econo Pump und UV-Monitor) verwendet. Die Chromatographiesäule wird zunächst mit 5 Säulenvolumina Puffer A (100 mM NaH2PO4, 10 mM Tris/HCl, 6 M Guanidin-HCl, pH mit HCl auf 8,0 eingestellt) gewaschen und anschließend das Bakterienlysat mit einer Flussgeschwindigkeit von 1,4 ml/min. auf die Säule aufgetragen. Anschließend werden je 5 bis 10 Säulenvolumina Puffer A und Puffer B (Puffer B: 100 mM NaH2PO4, 10 mM Tris/HCl, 8 M Harnstoff, pH 8,0) mit einer Flussrate von 1,4 ml/min. auf die Säule gepumpt und die Absorption des Durchfluss bei einer Wellenlänge von 280 nm (A280) beobachtet. Sobald der Durchfluss eine stabile A280 von kleiner ca. 0,01 erreicht hat wird die Säule mit 5 bis 10 Säulenvolumina Puffer C (100 mM NaH2PO4, 10 mM Tris/HCl, 8 M Harnstoff, pH mit HCl auf 6,3 eingestellt) gespült, bis schließlich eine stabile A280 von kleiner ca. 0,01 erreicht ist. Dann wird das MAT-Molekül mit Puffer E (100 mM NaH2PO4, 10 mM Tris/HCl, 8 M Harnstoff, pH mit HCl auf 4,5 eingestellt) eluiert und gesammelt.

### Beispiel 4:

### Nachweis von MAT-Molekülen mit SDS-Polyacrylamid Gelen, Coomassie-Färbung und mit Anti-His Westernblotting

### SDS-Polyacrylamid-Gelelektrophorese:

Für die Elektrophorese wurden Puffer, NuPAGE^{®} Gele und eine Xcell SureLock™ Elektrophoresekammer von Invitrogen (Invitrogen Life Technologies, Breda, The Netherlands) entsprechend den Herstellerangaben verwendet. 5 oder 10 *µ*g der isolierten MAT-Moleküle wurden pro Spur in 12 %-igen NuPAGE^{®} Novex Bis-Tris Gelen (Invitrogen) bei einer konstanten Spannung von 200 V unter Verwendung von 1 x konzentriertem NuPAGE^{®} SDS-Probenpuffer unter reduzierenden Bedingungen innerhalb von 35 bis 50 min. elektrophoretisch getrennt. Als Laufpuffer wurde MES- oder MOPS-Puffer (MES-Puffer: 50 mM MES (Morpholinoethansulfonsäure), 50 mM Tris/HCl, 3,5 mM SDS, 1 mM EDTA, pH 7,3, MOPS-Puffer: 50 mM MOPS (3-(N-Morphilino)Propansulfonsäure), 50 mM Tris/HCl, 3,5 mM SDS, 1 mM EDTA, pH 7,7) verwendet.

### Coomassie Blue Färbung:

Zum Färben der Gele werden diese für 1 h in Färbelösung (200 ml Methanol, 50 ml Essigsäure, 250 ml Wasser, 0,5 g Coomassie blue R-250) inkubiert und anschließend unter mehrmaligem Wechseln der Entfärbelösung (200 ml Methanol, 50 ml Essigsäure, 250 ml Wasser) entfärbt, bis der Hintergrund der Gele klar ist. Zum Elektrotransfer der Proteine aus dem NuPAGE^{®} Gel auf eine Blottingmembran wird das Xcell II™ Blot Modul (Invitrogen) entsprechend den Herstellerangaben verwendet. Die Blotting-Apperatur wird entsprechend den Herstellerangaben unter Verwendung von 1 x NuPAGE^{®} Transfer Puffer mit 10 oder 20 % Methanol aufgebaut. Als Blottingmembran wurden PVDF-Membranen verwendet und der Elektrotransfer erfolgte für 1 h bei 30 V konstanter Spannung.

### Immunologischer Nachweis der MAT-Moleküle:

Der immunologische Nachweis der MAT-Moleküle erfolgte unter Verwendung von anti-His Antikörpern entsprechend den Herstellerangaben (anti-RGS(His)4 Antikörper, Qiagen, Hilden, Germany). Alle Versuchsschritte erfolgen bei Raumtemperatur. Die PVDF-Membran wird zunächst getrocknet und dann direkt, ohne vorherige Blockierung freier Proteinbindungsstellen, mit dem anti-His-Antikörper (Qiagen) in einer Verdünnung von 1:1000 bis 1:2000 in TBS (50 mM Tris/HCl, 150 mM NaCl, pH 7,5) mit 3 % BSA (Rinderserum-Albumin) für 1 h inkubiert. Dann wird die Membran 3x je 10 min. mit TBS, 0,05 % Tween 20, 0,2 % Triton X-100 gewaschen und anschließend einmal mit TBS ohne weitere Zusätze gewaschen. Als sekundärer Antikörper wird anti-Maus-Ig-HRP-Konjugat (Amersham, Buckinghamshire, England) in einer Verdünnung von 1:10000 bis 1:20000 in TBS mit 10 % Milchpulver verwendet und 1 h inkubiert. Zuletzt wird der Blot 4x für je 10 min. mit TBS, 0,05 % Tween 20, 0,2 % Triton X-100 gewaschen. Der Nachweis des Konjugates erfolgt mit dem ECL™ System (Amersham) entsprechend den Herstellerangaben. Das Chemilumineszenzsignal wird unter Verwendung von Autoradiographie-Filmen nachgewiesen, die entsprechend Standardprotokollen entwickelt werden.

### Beispiel 5:

### Translokation von MAT-Molekülen in Zelllinien und primäre humane Zellen

PBMCs (periphere Blut mononukleare Zellen) wurden entsprechend dem Fachmann bekannter Standardmethoden über Dichtegradientenzentrifugation unter Verwendung von Ficoll-Paque (Pharmacia, Uppsala, Sweden) aus frischem, heparinbehandeltem humanem Blut von freiwilligen Probanden gewonnen. Die Jurkat Zelllinie wurde von ATCC (American Type Culture Collection, Manassas, VA, USA) erhalten. Die Zellen wurden in RPMI-1640-Medium kultiviert, das folgende Zusätze enthielt: 10 % fötales Kälberserum, 200 Einheiten / ml Penicillin, 200 *µ*g/ml Streptomycin, MEM-Vitamine, MEM nicht-essentielle Aminosäuren, 1 mM Natrium Pyruvat, 2 mM L-Glutamin und 0,001 % 2-Mercaptoethanol. Zur Bestimmung der Translokation wurden die Zellen in einer Konzentration von 1 x 10Exp6 / ml RPMI Medium mit allen Zusätzen resuspendiert. Die Zellen wurden entsprechend den jeweiligen Angaben in den Erklärungen zu den Abbildungen mit Konzentrationen von 0,01 bis 5 mM der rekombinanten Moleküle, bzw. MAT-Moleküle über einen Zeitraum von 5 min. bei 4, 22 oder 37 °C inkubiert. Anschließend wurden die Zellen zentrifugiert und das Zellpellet in 40 *µ*l Lysepuffer (8 M Harnstoff, 100 mM Natriumphosphat, 10 mM Tris/HCl, 100 mM Ammoniumchlorid) für 5 min. bei Raumtemperatur inkubiert. Unlösliche Zellbestandteile wurden durch Zentrifugation (15000 g, 15 min.) abgetrennt und das klare Lysat entsprechend Beispiel 4 in einem Westernblot weiter untersucht. Zum Nachweis der Proteine wurde ein anti-His Antikörper verwendet.

### Beispiel 6:

### Stimulierung peripherer mononuklearer Zellen aus dem Blut (PBMC) von Patienten mit Allergien

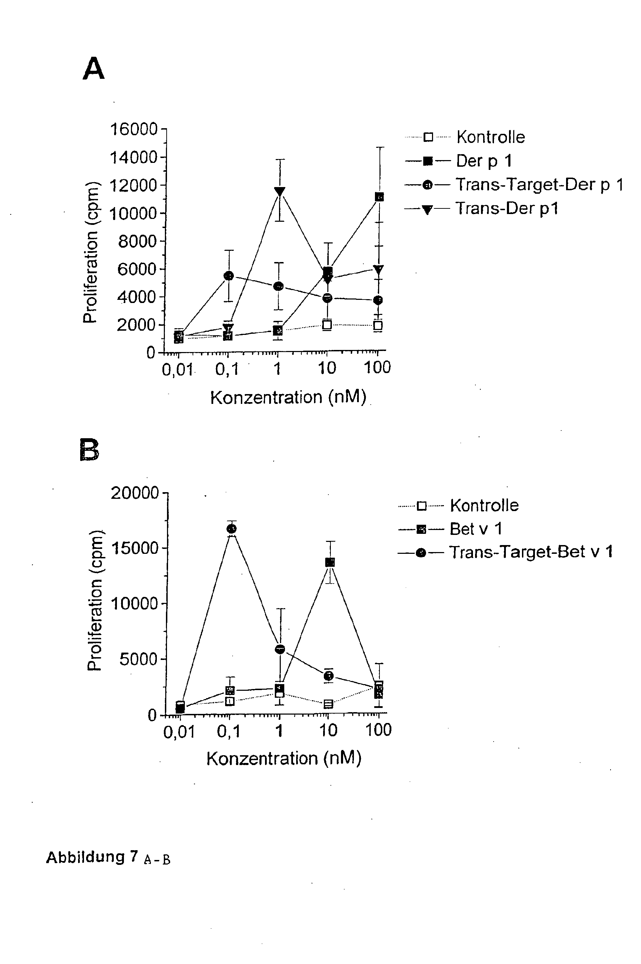

Die nachfolgend beschriebenen Methoden zur Stimulierung von PBMCs und zur Bestimmung der Proliferation der stimulierten PBMCs sind in der Literatur beschrieben [44]. PBMCs wurden durch Dichtegradientenzentrifugationen unter Verwendung von Ficoll-Paque (Pharmacia, Uppsala, Sweden) aus frischem, heparinbehandeltem Blut von freiwilligen Probanden isoliert. Bei den freiwilligen Probanden handelte es sich um Patienten mit einer Allergie gegen ein für den individuellen Patienten bekanntes Allergen. Die Patienten wurden entsprechend den schweizerischen Gesetzen über die mit ihren Blutproben durchgeführten Experimente aufgeklärt und erklärten sich damit einverstanden an dieser Studie teil zu nehmen. Nach der Gewinnung der PBMCs wurden diese in RPMI-Medium mit den Zusätzen entsprechend Beispiel 5 aufgenommen und jeweils 0,01 bis 100 nM des rekombinant hergestellten Antigens zugegeben, gegen das der jeweilige Patient allergisch reagiert. Es wurden jeweils das nicht modifizierte Antigen und das mit einem Translokations-Modul und einem Targeting-Modul gekoppelte Antigen (MAT-Molekül) untersucht. Bei einigen Experimenten wurden auch Versuchsansätze durchgeführt, bei denen kein Targeting-Modul, sondern nur ein Translokations-Modul and das Antigen-Modul gekoppelt waren (Abbildung 7 A). Als Kontrollen wurden in einigen Experimenten auch nur das Translokations-Modul oder eine Konstrukt bestehend aus Translokations-Moudul und Targeting-Modul, aber ohne Antigen-Modul verwendet. Nach einer Inkubationszeit von 5 Tagen wurde dem Medium 10 *µ*Ci/ml tritiummarkiertes Thymidin zugegeben. Die Thymidin-Incorporation der PBMCs wurde als Maß für die Effizienz der Antigenpräsentation und der damit verbundenen Proliferation der behandelten Zellen ermittelt. Dazu wurde nach 8 bis 10 h das radioaktive Zellkulturmedium entfernt, die Zellen gewaschen und die Menge an eingebautem radioaktivem Thymidin über Messung der Radioaktivität bestimmt. Dazu wurde ein 1205 Betaplate Liquid scintillation counter der Firma Wallac ADL AG, Hünenberg, Switzerland, verwendet. Als Kontrolle wurden nicht mit Antigen stimulierte Zellen ebenfalls mit tritiummarkiertem Thymidin inkubiert und anschließend in gleicher Art und Weise analysiert. Als Ergebnis erhält man einen Messwert für den Einbau an radioaktivem Thymidin als Maß für die Proliferation und damit als Maß für die Effizienz der Antigenpräsentation. Je höher der gemessene Thymidineinbau ist, desto effizienter war die Antigenpräsentation. Da in jedem Versuch Antigenkonzentrationen von 0,01 nM bis 100 nM untersucht wurden, lässt sich außerdem bestimmen, bei welcher Antigen-Konzentration eine maximale Antigenpräsentation erfolgt. Je geringer diese Konzentration ist, desto wirksamer ist das Antigen als Modulator einer Immunantwort. Als weitere Kontrolle wurden Zellen mit je 0,5 *µ*g/ml eines anti-CD3 und eines anti-CD28 Antikörpers behandelt, was einen sehr starken Proliferationsstimulus darstellt. Dadurch konnte für jeden Versuch der durch die Proliferation der PBMCs maximal mögliche Thymidineinbau bestimmt werden.

### Beispiel 7:

### MAT-Molekül stimulierte Zytokinfreisetzung

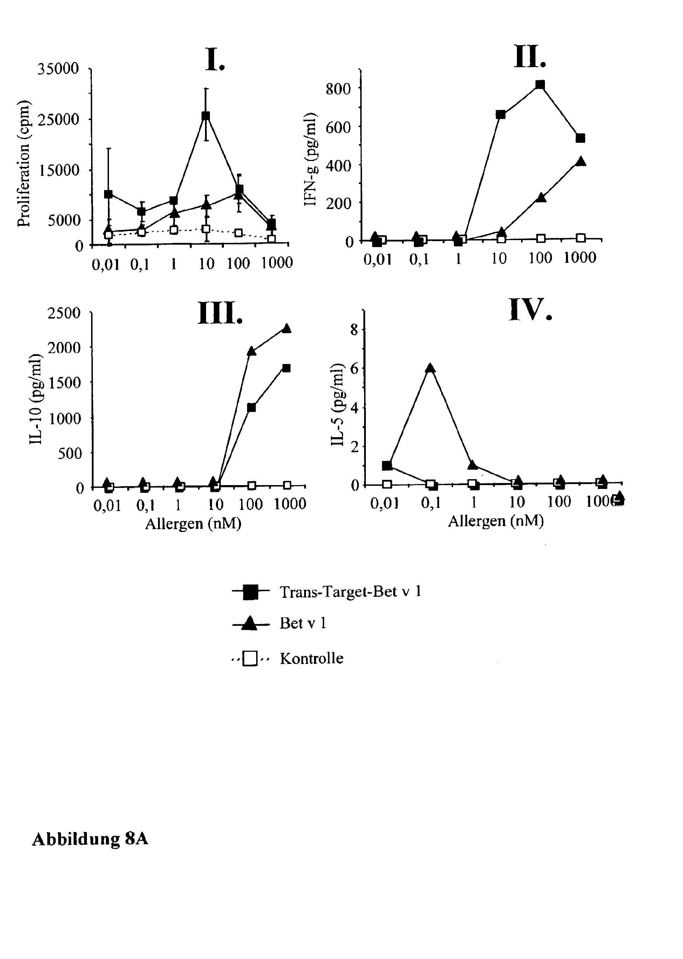

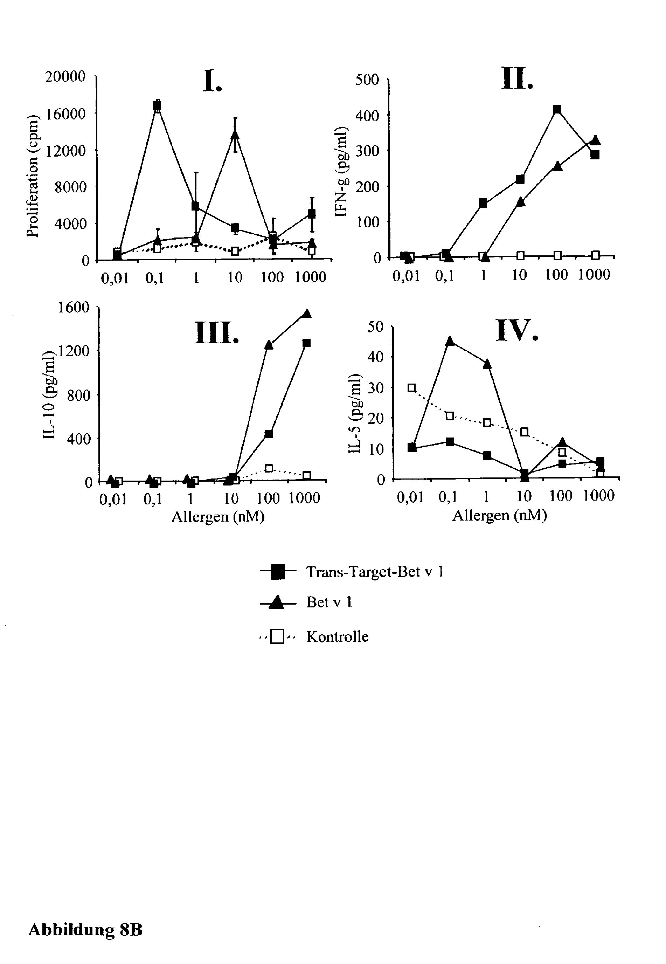

PBMCs wurden wie in Beispiel 5 beschrieben aus dem Blut von Allergikern isoliert und 10Exp6 / ml Medium verdünnt. Es wurden je 100 *µ*l dieser Zellsuspension in 96-Loch Platten ausgesät und mit 0,01 bis 1000 nM der isolierten Antigene für 5 Tage behandelt. In dieser Zeit wurde das Zellkulturmedium nicht ausgetauscht. Nach Zentrifugation der 96-Loch Platten wurden die Überstände abgenommen und bis zur Zytokin-Analyse bei -20 °C gelagert. Die PBMCs wurden mit jeweils dem Antigen behandelt, gegen das der Patient, von dem die PBMCs isoliert wurden, allergisch reagiert. Folgende Zytokine wurden in den so gewonnenen Überständen untersucht: Interferon gamma (INFg), Interleukin-10 (IL-10) und Interleukin-5 (IL-5). Abbildung 8A und 8B zeigen Ergebnisse, die mit PBMCs von Patienten, die gegen Bet v 1 allergisch reagieren, erhalten wurden. Die INFg, IL-10 und IL-5 Immunoassays (ELISAs = Enzyme-linked Immuno Sorbent Assays) wurden entsprechend dem Fachmann bekannten Methoden unter Verwendung von "DuoSet^{®} ELISA Development Systemen" der Firma R & D Systems Inc., Minneapolis, USA durchgeführt.

Parallel zur Gewinnung der Überstände für die Zytokinbestimmungen wurden mit PBMCs der gleichen Spender auch Stimulierungs-Experimente entsprechend Beispiel 6 durchgeführt. Die Ergebnisse dieser Zellproliferationsassays sind ebenfalls in Abbildung 8A und 8B dargestellt.

### Beispiel 8:

### In vivo Wirkung von nicht modifizierten Antigenen und Antigenen, die sich im Antigen-Modul von MAT-Molekülen befinden

### Immunisierung der Mäuse

Um die Wirksamkeit von MAT-Molekülen zu testen wurden CBA/2 Mäuse 3x in einem Abstand von je 2 Wochen mit rekombinant hergestellten MAT-Molekülen, gemeinsam mit Aluminiumhydroxid als Adjuvans in einer dem Fachmann bekannten Art und Weise immunisiert. Die rekombinanten MAT-Moleküle wurden wie in Beispiel 3 beschrieben hergestellt. Es wurden 3 verschiede Wege zur Immunisierung verwendet. Je eine Versuchsreihe mit subkutaner, intraperitonealer und intranodaler Injektion der Antigene bzw. der Kontrollen wurde durchgeführt. Bei der intranodalen Injektion wurde das Gewebe operativ freigelegt, so dass direkt injiziert werden konnte. Als MAT-Molekül wurde ein Protein verwendet, das aus der HIV-Tat-Sequenz als Translokations-Modul, der humanen Invarianten Kette als Targeting-Modul und dem PLA2- (Phospholipase A2 aus dem Bienengift) Peptid als Antigen-Modul bestand (Bezeichnung: Trans-Target-PLA2). Als Kontrolle wurde PLA2 (Bezeichnung: PLA2) und als weitere Kontrolle 0,9 %-ige Kochsalzlösung (Bezeichnung: Kontrolle) verwendet. Die intranodale Immunisierung erfolgte mit 0,1 *µ*g, die subkutane und intraperitoneale Immunisierung erfolgte mit 10 *µ*g MAT-Molekül bzw. entsprechende Mengen an Kontroll-Protein oder Kontroll-Puffer gemeinsam mit Aluminiumhydroxid als Adjuvans. Pro Versuchsgruppe wurden 3 Tiere immunisiert, so dass jeweils 9 Tiere pro Versuch verwendet wurden. Es wurden 3 Versuchsreihen durchgeführt und bei jeder Versuchsreihe ein anderer Immunisierungs-Weg (subkutan, intraperitoneal, intranodal) erprobt. Vor der ersten Immunisierung, und anschließend nach 2, 4 und 6 Wochen wurde jedem Versuchstier über die Schwanzvene Blut entnommen. Die Gerinnung des Blutes erfolgte bei Raumtemperatur und anschließend wurde nach Zentrifugation das Serum gewonnen und bis zur Analyse bei -20 °C gelagert. Die so hergestellten Seren wurden zur Bestimmung der PLA2-spezifischen IgG- und IgE-Titer verwendet.

### Bestimmung der PLA2-spezifischen IgG2a Titer in den Maus-Seren

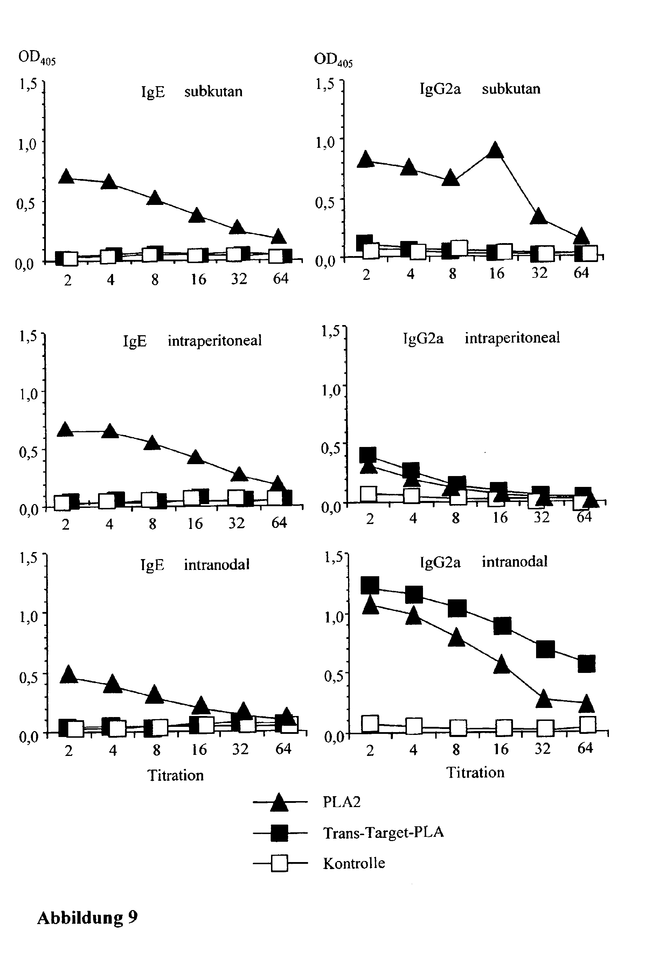

Zur Bestimmung des PLA2-spezifischen IgG2a Titers wurden Mikrotiterplatten (96 Kavitäten) über Nacht mit 100 *µ*l/Kavität einer Lösung von 5*µ*g/ml PLA2 (Sigma-Aldrich, Buchs DG, Schweiz) in Carbonatpuffer bei 4 °C beschichtet. Nach 2x waschen mit phosphatgepufferter Natriumchloridlösung (PBS), 0,05 % Tween wurden freie Proteinbindungsstellen durch Inkubation mit Blockierungspuffer (PBS, 2,5 % Magermilchpulver), 200 *µ*l/Kavität, bei Raumtemperatur für 1 bis 2 h blockiert. Es wurde erneut 2x gewaschen und anschließend serielle 1:2 Verdünnungsreihen in Blockierungspuffer (50 *µ*l/Kavität) der zu testenden Serumproben (1:2 bis 1:64 Verdünnungen) für 3 h bei Raumtemperatur oder über Nacht bei 4 °C inkubiert. Als Negativkontrollen wurden Inkubationen ohne Serum oder mit Serum von unbehandelten Tieren durchgeführt. Danach wurde 5x gewaschen und für 2 h bei Raumtemperatur (100*µ*l/Kavität) mit einer 1:500 Verdünnung in Blockierungspuffer eines Biotinmarkierten anti-Maus IgG2a (PharMingen GmbH, Hamburg, Deutschland) inkubiert und erneut 5x gewaschen. Zuletzt wurden 100 *µ*l/Kavität einer 1:1000 in Blockierungspuffer verdünnten Meerrettichperoxidase für 1 h bei Raumtemperatur inkubiert und anschließend 6x gewaschen. Die Farbreaktion wurde mit 100 *µ*l/Kavität einer Lösung von ABTS (2,2'-azino-di-(3 ethylbenzthiazoline sulfonic acid) in ABTS-Puffer entsprechend den Herstellerangaben mit 0,1 % (v/v) einer 30-%igen Wasserstoffperoxidlösung, durchgeführt. Nach ca. 30 Minuten wurde die Absorption bei einer Wellenlänge von 405 nm (Referenzfilter: 595 nm) gemessen. Die Ergebnisse dieser Tests sind in Abbildung 9 dargestellt.

### Bestimmung der PLA2-spezifischen IgE Titer in Maus-Seren

Der PLA2-IgE ELISA wird entsprechend dem Protokoll für den PLA2-IgG2a ELISA durchgeführt. Folgende Abweichungen von dem oben beschriebenen Protokoll treten auf: die Mikrotiterplatten werden mit 5 *µ*g/ml eines anti-Maus IgE Antikörpers beschichtet. Nach Inkubation der Serumproben und Waschen der Platten wird eine 1:333 Verdünnung von Biotin-markiertem PLA2 (Pierce Biotechnology Inc., Rockford, USA) inkubiert. Die Farbentwicklung mit ABTS dauert etwa 1 h. Die Ergebnisse dieser Tests sind in Abbildung 9 dargestellt.

### Erklärungen zu den Abbildungen

### Abbildung 1

### Theoretischer Aufbau von MAT-Molekülen

Abbildung 1 zeigt exemplarisch, wie die einzelnen Module eines erfindungsgemäßen MAT-Moleküls schematisch aufgebaut sein können. Dabei steht "Trans" für ein Translokations-Modul, "Target" für ein Targeting-Modul, "AG" für ein Antigen-Modul, "Tag" für ein Tag-Modul und ein Strich (-) steht für ein Linker-Modul. Das Linker-Modul kann die anderen Module durch kovalente und/oder nicht kovalente Bindungen miteinander verbinden. Abbildung 1A stellt eine Reihe von exemplarischen linearen Anordnungen der verschiedenen Module dar. Abbildung 1B stellt verschiedene exemplarische Anordnungen von verzweigten MAT-Molekülen dar, wobei auch dendrimere Strukturen eingeschlossen sind und Abbildung 1C stellt einige exemplarische Anordnungen von zirkulären MAT-Molekülen dar, wobei zirkuläre Anordnungen mit linearen und/oder verzweigten Anordnungen kombiniert sein können. Generell sind alle dargestellten Anordnungen nur Beispiele, die verdeutlichen sollen, dass verschiedenste Anordnungen möglich sind. Die schematisch dargestellten MAT-Molekül-Beispiele sind keinesfalls als limitierend für den Schutzumfang der vorliegenden Erfindung zu verstehen.

### Abbildung 2

### Expression, Aufreinigung und Nachweis von Fusionsproteinen

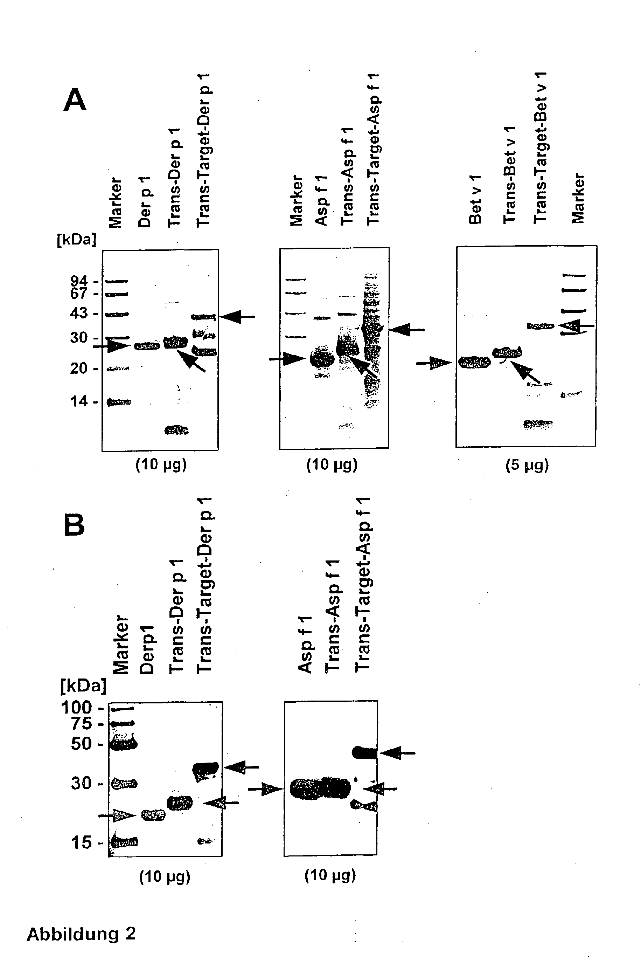

Verschiedene MAT-Moleküle bestehend aus Tag-Modul (His6-Tag), Translokations-Modul (Tat-Sequenz), Targeting-Modul (humane invariante Kette des MHC II) und Antigen-Modul (Asp f1 = Aspergillus fumigatus Antigen 1) wurden in E. coli exprimiert und unter denaturierenden Bedingungen unter Verwendung von immobilisierter Metall-lonenaffinitäts-Chromatographie isoliert. Die Arbeitsschritte wurden entsprechend den Angaben des Herstellers des Expressions-Systems (Qiagen, Hilden, Germany) durchgeführt [47, 48]. Je 10 *µ*g, bzw. 5 *µ*g der isolierten Proteine wurden in SDS-Polyacrylamid Gelen elektrophoretisch aufgetrennt und anschließend entweder mit Coomassie Blue gefärbt (Abb. 2 A), oder in einem Westernblot unter Verwendung von Antikörpern, die das Tag-Modul erkennen, analysiert (Abb. 2 B). Die Positionen der exprimierten Proteine sind durch Pfeile markiert.

### Abbildung 3

Temperaturunabhängige Translokation von Proteinen und MAT-Molekülen Primäre humane periphere mononukleare Zellen (PBMCs) wurden für 5 Minuten bei 4, 22 oder 37 °C mit den jeweiligen Proteinen oder MAT-Molekülen in einer Konzentration von 1 *µ*M inkubiert, gewaschen, in harnstoffhaltigem Probenpuffer aufgenommen und lysiert. Die Lysate wurden anschließend in SDS-Polyacrylamidgelen elektrophoretisch aufgetrennt, auf PVDF-Membranen elektrotransferiert und die Proteine, bzw. MAT-Moleküle mit einem spezifischen Antikörper (anti-RGS(His)4 Antikörper) detektiert. Die Pfeile zeigen die Position der Proteine, bzw. MAT-Moleküle an. Bei allen Temperaturen konnten die Fusionsproteine in den Lysaten der Zellen nachgewiesen werden, was eine erfolgreiche Translokation ins Zellinnere anzeigt.

### Abbildung 4

### Translokation von MAT-Molekülen in Zelllinien und primären Zellen

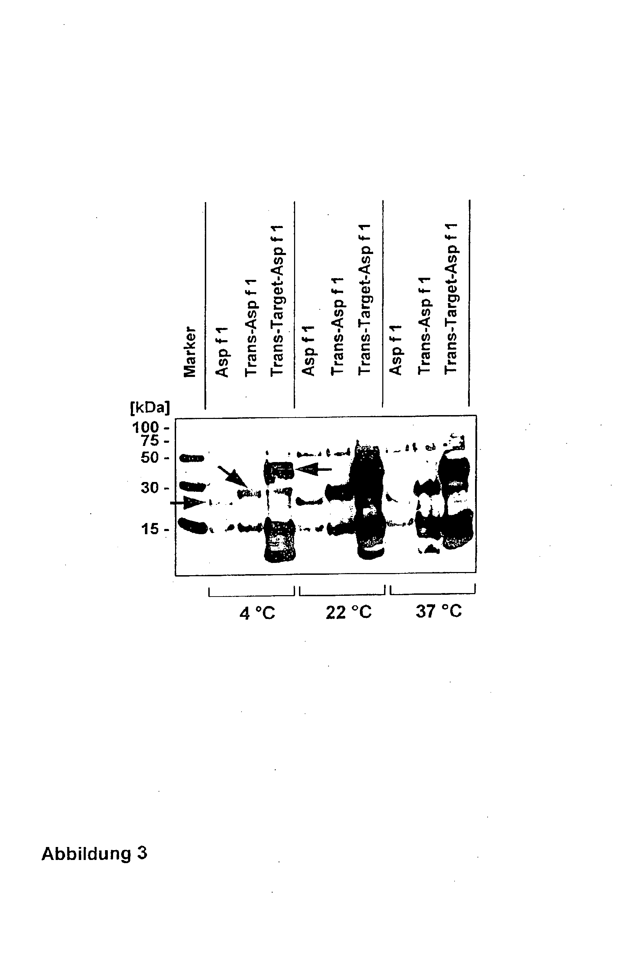

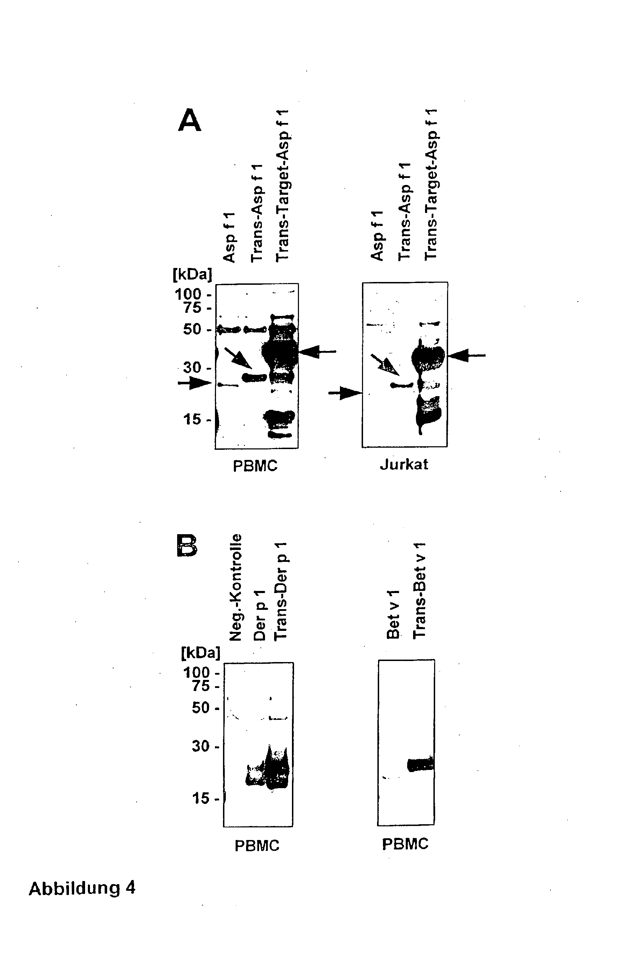

Das in Abbildung 4 dargestellte Ergebnis wurde mit nahezu der gleichen Methodik wie der Versuch in Abbildung 3 durchgeführt, jedoch mit zwei Abweichungen: Abbildung 4A zeigt, dass sowohl in primären humanen Zellen (PBMCs), als auch in humanen Tumorzelllinien (Jurkat Zellen) die Translokation der MAT-Moleküle erfolgreich war. Die Pfeile zeigen die Position auf dem Westernblot, an der das Protein, bzw. das MAT-Molekül detektiert wurde. Abbildung 4A und 4 B zeigen außerdem, dass verschiedene Antigen-Module (Asp f 1 = Aspergillus fumigatus Allergen, Der p 1 = Hausstaub-Milben Allergen, Bet v 1 = Birkenpollen Allergen) über Translokation in primäre humane PBMC Zellen gelangen können.

### Abbildung 5

### Dosiskinetik der Translokation von MAT-Molekülen

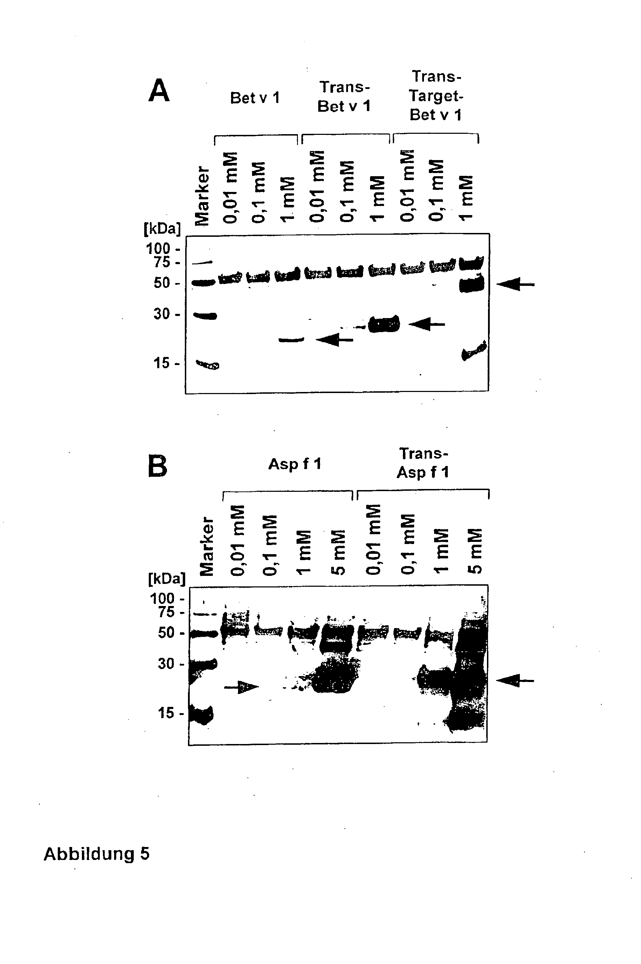

Das in Abbildung 5 dargestellte Ergebnis wurde mit nahezu der gleichen Methodik wie der Versuch in Abbildung 3 erhalten, wobei jedoch drei, bzw. vier verschiedene Konzentrationen von Proteinen, bzw. MAT-Molekülen verwendet wurden (0,01 mM, 0,1 mM, 1 mM und 5 mM). Es konnte eine klare Dosisabhängigkeit für die Translokationsreaktion gezeigt werden. Mit zunehmender Konzentration an zugegebenen MAT-Molekülen wurden größere Mengen des jeweiligen Moleküls im Zellinneren detektiert. Die Positionen der jeweiligen Moleküle sind mit Pfeilen markiert.

### Abbildung 6

### Verschiedene funktionale Strukturen von MAT-Molekülen

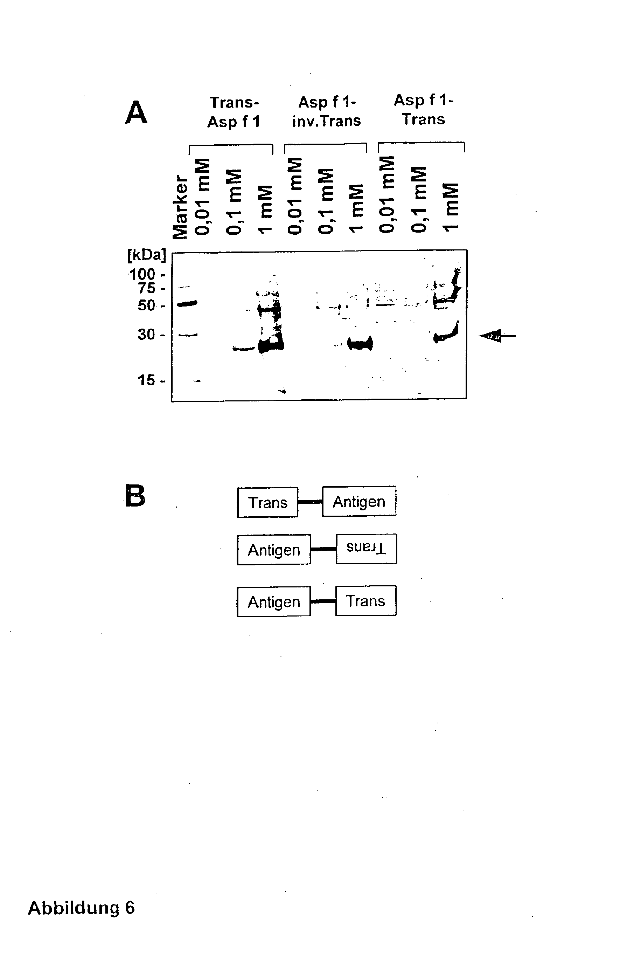

Das in Abbildung 6 dargestellte Ergebnis wurde mit nahezu der gleichen Methodik wie der Versuch in Abbildung 3 erhalten, wobei jedoch drei verschiedene Anordnungen von Translokations-Modul (Tat-Sequenz) und Antigen-Modul (Asp f 1 = Aspergillus fumigatus Allergen) untersucht wurden. Der Pfeil in Abbildung 6A gibt die Position an, an der das Fusionsprotein in dem Westernblot detektiert wurde. Abbildung 6B stellt den Aufbau der drei verschiedenen Fusionsproteine dar. Es wurden Fusionsproteine mit N-terminalem, invertiertem C-terminalem und mit C-terminalem Translokations-Modul untersucht. Alle drei Fusionsproteine wurden durch Translokation erfolgreich ins Zellinnere transportiert.

### Abbildung 7

### Antigen/MAT-Molekül vermitteltein vitro Zellproliferation von PBMCs von Alergikern

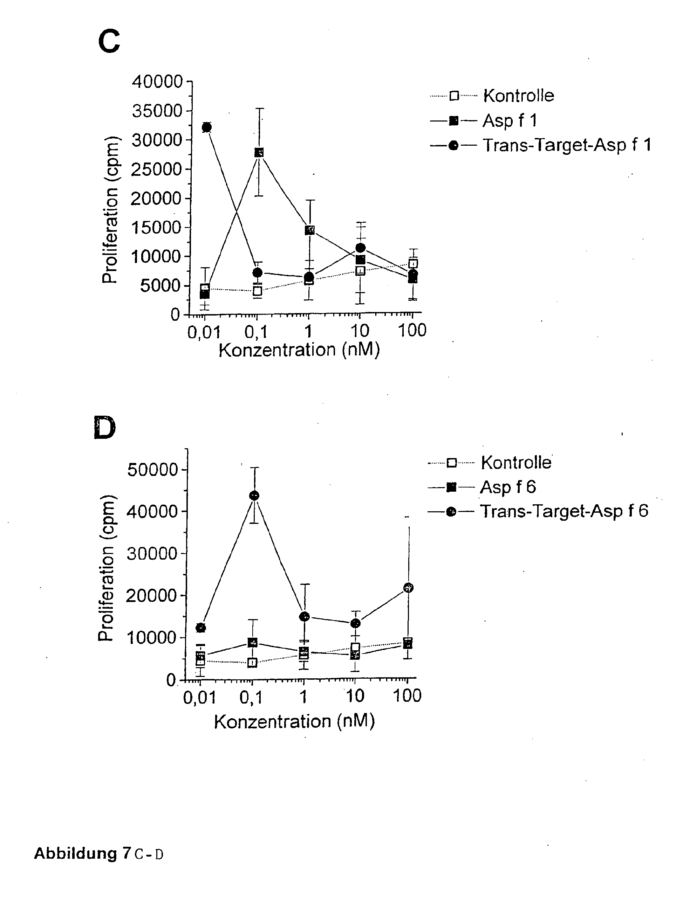

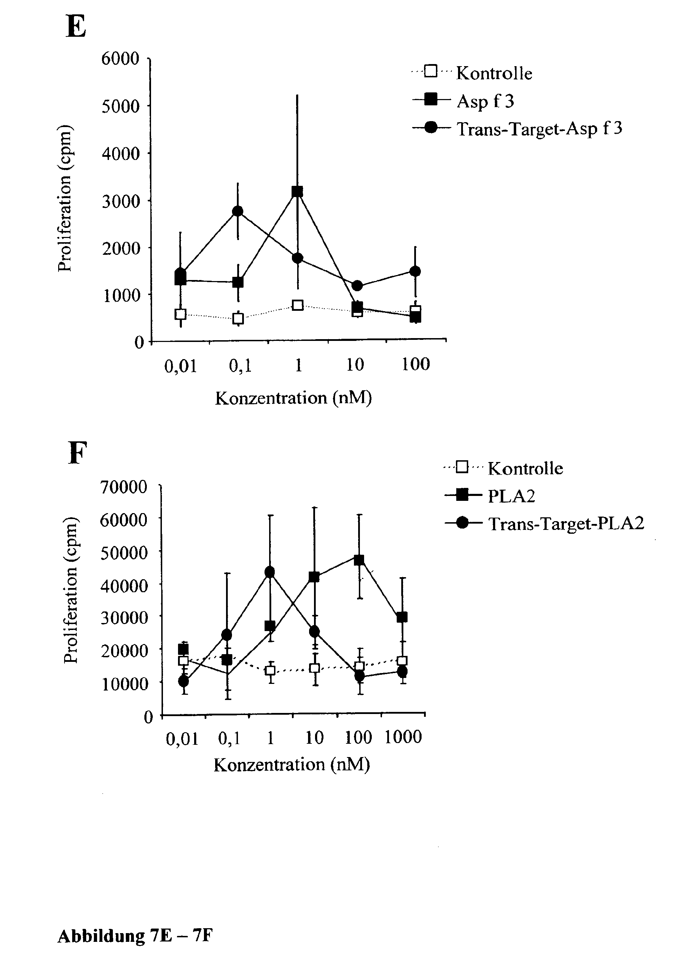

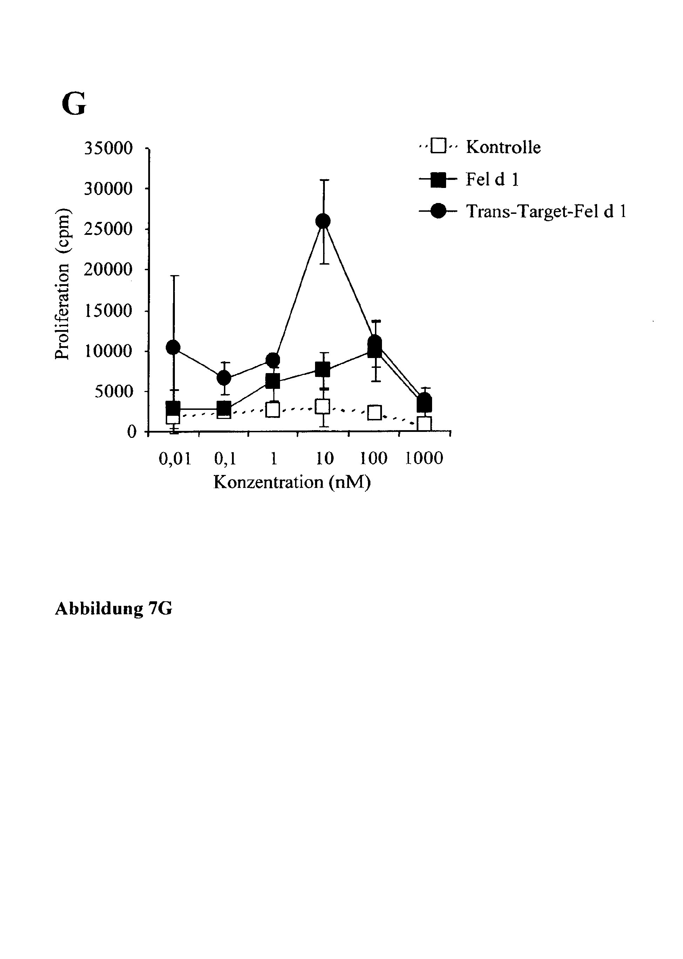

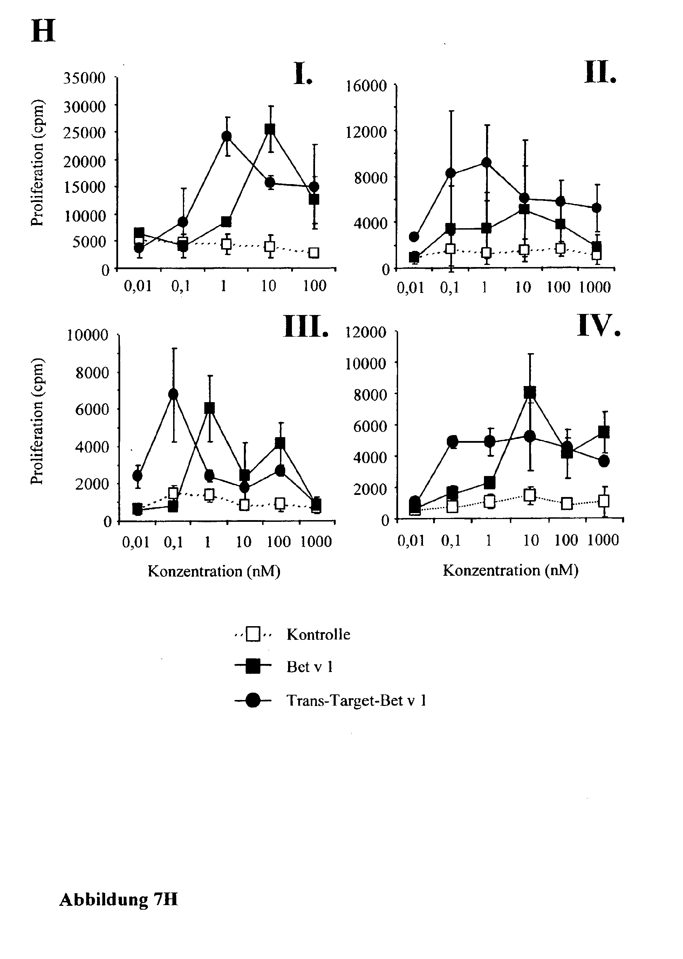

In Abbildung 7 sind Untersuchungen an PBMCs (periphere Blut mononukleäre Zellen) von Allergikern dargestellt, die in vitro mit einem MAT-Molekül zusammen gegeben wurden. Das MAT-Molekül enthielt als Antigen-Modul das jeweilige Allergen, gegen das der Allergie-Patient allergisch reagiert. Eine aufgrund der Zugabe des MAT-Moleküls effizientere Antigen-Präsentation der Antigen-präsentierenden Zellen des Allergikers führt zu einer Wachstumsstimulation der PBMCs. Die daraus resultierende Zellproliferation wurde über den Einbau von radioaktiv markierten DNA-Bausteinen (Thymidin) quantifiziert (y-Achse). Die x-Achse gibt die in dem jeweiligen Zell-Stimulations-Test verwendete MAT-Molekül-, bzw. Antigen-Molekül-Konzentration in nM an. In allen untersuchten Patienten mit allen untersuchten Antigen-Modulen (als Teil des MAT-Moleküls) kommt es bei Verwendung von MAT-Molekülen bei geringeren Konzentrationen zu einer Zellproliferation (= Immunstimulation = Zunahme der Thymidineinbaus = Anstieg der Radioaktivität). Als Kontrolle wurden jeweils die gleichen PBMCs nur mit Wasser behandelt, das keine MAT-Moleküle oder Antigene enthielt (= Kontrolle in der Legende). Außerdem wurde für jeden Versuch als Positivkontrolle der Thymidineinbau von Zellen, die mit einem starken Wachstumsstimulus (anti-CD3 und anti-CD28 Antikörper, je 0,5 *µ*g/ml) für 5 Tage behandelt wurden, gemessen. Das durch diesen Stimulus erhaltene Wachstum stellt eine Kontrolle für die Qualität der präparierten PBMCs in Bezug auf ihre Proliferationsfähigkeit dar. Für den Versuch in Abbilung 7A ergab sich daraus ein Wert von 77864 ± 5347 cpm, für den Versuch in Abbildung 7B ergab sich ein Wert von 100374 ± 11678 cpm und für die Versuche in den Abbildungen 7C und D ergab sich ein Wert von 112205 ± 5958 cpm.

Die Abbildungen 7 A bis 7 G zeigen, dass bei allen 7 getesteten Antigenen der Einbau des Antigens in das Antigen-Modul eines MAT-Moleküls dazu führt, dass schon bei geringeren Konzentrationen eine Proliferation der PBMCs des Allergikers einsetzt. Getestet wurden die Antigene Der p 1 (Hausstaub-Milben Allergen; Abb. 7 A), Bet v 1 (Birkenpollen Allergen, Abb. 7 B), Asp f 1 (Aspergillus fumigatus Allergen 1, Abb. 7 C), Asp f 6 (Aspergillus fumigatus Allergen 6, Abb. 7 D), Asp f 3 (Aspergillus fumigatus Allergen 3, Abb. 7 E), PLA2 (Phospholipase A2 Allergen aus Bienengift, Abb. 7F) und Fel d 1 (Felis domesticus, Abb. 7G). Abbildung 7H zeigt zusätzlich für das Antigen Bet v 1, dass bei 4 verschiedenen Patienten (I. bis IV.) mit einer Allergie gegen Bet v 1 in allen 4 Fällen, bei Verwendung von MAT-Molekülen mit Bet v1 im Antigen-Modul, schon bei geringeren Konzentrationen eine Proliferation der PBMCs der individuellen Patienten auftritt, als bei Verwendung von Bet v 1 als Antigen.

### Abbildung 8

### Antigen / MAT-Molekül vermittelte in vitro Zytokin-Sezernierung durch PBMCs von Allergiker

Die Abbildungen 8A und 8B zeigen für zwei unabhängige Patienten, die gegen Bet v 1 allergisch reagieren, dass in vitro kultivierte, mit dem Bet v 1 Antigen (Bet v 1 allein oder als Modul eines MAT-Moleküls) stimulierte, PBMCs ein definiertes Zytokin-Sezernierungs-Muster aufweisen. I. zeigt jeweils das Ergebnis eines Zellproliferationstests (wie in Abb. 7), II. zeigt die Interferon gamma (INFg) Werte, III. zeigt die Interleukin-10 (IL-10) Werte und IV. zeigt die Interleukin-5 (IL-5) Werte in den Zellkultur-Überständen an. Bei einer desensibilisierend wirkenden PBMC-Stimulation sollte die PBMC-Proliferation und ein Anstieg der INFg-Freisetzung schon bei geringeren Antigen-Dosen auftreten. Zusätzlich sollte die IL-5 Produktion niedrig sein. Dieses Zytokin-Sezernierungs-Muster deutet auf eine Desensibilisierung der Immunzellen der Allergiker hin (Th1- statt Th2-Immunantwort). Der Anstieg der IL-10 Konzentration erklärt, warum die Zellproliferation (Abb. I.) bei höheren Antigen-Dosen sinkt. Dieses Zytokin-Sezernierungs-Muster tritt in beiden Fällen (Abb. 8A und Abb. 8B) auf.

### Abbildung 9

### Antigen / MAT-Molekül vermittelte in vivo Immunantwort in Mäusen

CBA/2 Mäuse wurden mit isoliertem PLA2, mit MAT-Molekülen mit PLA2 im Antigen-Modul oder mit Kontroll-Puffer 3x in 2-wöchigem Abstand immunisiert und anschließend die PLA2-spezifischen Serum-Titer an IgG2a und IgE Antikörpern gemessen. Bei einer Desensibilisierung sollten nur PLA2-spezifische IgG, aber keine PLA2-spezifischen IgE-Antikörper auftreten. IgE-Antikörper sind für allergische Reaktionen verantwortlich. Es wurden 3 verschiedene Immunisierungs-Wege untersucht, subkutane, intraperitoneale und intranodale Injektion der Antigene. Bei allen drei Immunisierungs-Wegen zeigt sich, dass eine Immunisierung mit PLA2 zu einer deutlichen IgE Immunantwort (Allergie) führt (Abb. 9, linke Spalte). Bei einer Immunisierung mit einem MAT-Molekül, das PLA2 als Antigen-Modul enthält, kommt es jedoch zu keiner IgE-Immunantwort (keine Allergie). Hingegen führen sowohl eine Immunisierung mit PLA2, als auch mit MAT-Molekülen, die PLA2 im Antigen-Modul enthalten, zu einer erwünschten, nicht allergische Reaktionen auslösenden, IgG Immunantwort.

Alle Beispiele und Aufzählungen in der vorliegenden Patentanmeldung sind grundsätzlich zur Verdeutlichung des Sachverhalts, nicht aber zur Einschränkung der Ansprüche gedacht. Insbesondere die Beispiele für Translokations-Module, Targeting-Module, Antigen-Module, Spacer-Module und Tag-Module sind nur als Beispiele, nicht jedoch als erschöpfende Auflistung aller möglichen Bestandteile des MAT-Moleküls zu verstehen. Die Grundidee der Erfindung besteht nicht in einer bestimmten Kombination bestimmter Translokations-Module, bestimmter Targeting-Module und bestimmter Antigen-Module. Die Grundidee der vorliegenden Erfindung ist vielmehr, eine Kombination zumindest dieser drei Module zu einem MAT-Molekül zur Immunisierung zu verwenden. Daher spielt das speziell verwendete Beispiel in jedem einzelnen der Module keine Rolle für den Erfindungsgedanken und es können folglich auch andere, auch derzeit noch nicht bekannte Beispiele, für die jeweiligen Module im Sinne der Erfindung verwendet werden.

Falls in dieser Patenschrift ein Begriff nicht eindeutig definiert ist, oder dem Fachmann auf dem jeweiligem Fachgebiet nicht bekannt sein sollte, oder ein Begriff aus dem Textzusammenhang nicht eindeutig definiert werden kann, so gilt die jeweils in den nachfolgenden Standardwerken genannte Definition des jeweiligen Begriffs. Falls ein Begriff in mehreren der nachfolgend zitierten Werke mit verschiedenen Definitionen aufgeführt wird, so gilt jeweils die Definition, die in dem zuerst in der nachfolgenden Liste aufgeführten Werk genannt wird. Folgende Publikationen werden zu diesem Zweck zitiert:
- The Merck Mannual [49]
- Molecular Cloning - A Laboratory Manual [43]
- Current Protocols in Immunology [44]
- Current Protocols in Protein Science [50]
- Current Protocols in Pharmacology [51]
- Current Protocols in Cell Biology [52]

### Literaturzitate

1. Pieters, J. 2000. MHC class II-restricted antigen processing and presentation. Adv Immunol. 75:159-208.
2. Marks, M.S., P.A. Roche, E. van Donselaar, L. Woodruff, P.J. Peters, and J.S. Bonifacino. 1995. A lysosomal targeting signal in the cytoplasmic tail of the beta chain directs HLA-DM to MHC class II compartments. J Cell Biol. 131:351-69.
3. Karlsson, K., and S.R. Carlsson. 1998. Sorting of lysosomal membrane glycoproteins lamp-1 and lamp-2 into vesicles distinct from mannose 6-phosphate receptor/gamma-adaptin vesicles at the trans-Golgi network. J Biol Chem. 273:18966-73.
4. Obermüller, S., C. Kiecke, K. von Figura, and S. Honing. 2002. The tyrosine motifs of Lamp 1 and LAP determine their direct and indirect targetting to lysosomes. J Cell Sci 115:185-94.
5. Chervonsky, A.V., L. Gordon, and A.J. Sant. 1994. A segment of the MHC class II beta chain plays a critical role in targeting class II molecules to the endocytic pathway. Int Immunol. 6:973-82.
6. Frankel, A., C. Pabo, J.G. Barsoum, S.E. Fawell, and R.B. Pepinsky. 1995. Tat-derived transport polypeptides and fusions proteins. US-Patent 5, 804, 604*.*
7. Prochiantz, A. 2000. Messenger proteins: homeoproteins, TAT and others. Curr Opin Cell Biol. 12:400-6.
8. Futaki, S., T. Suzuki, W. Ohashi, T. Yagami, S. Tanaka, K. Ueda, and Y. Sugiura. 2001. Arginine-rich peptides. An abundant source of membrane-permeable peptides having potential as carriers for intracellular protein delivery. J Biol Chem. 276:5836-40.
9. Schwartz, J.J., and S. Zhang. 2000. Peptide-mediated cellular delivery. Curr Opin Mol Ther. 2:162-7.
10. Wender, P.A., D.J. Mitchell, K. Pattabiraman, E.T. Pelkey, L. Steinman, and J.B. Rothbard. 2000. The design, synthesis, and evaluation of molecules that enable or enhance cellular uptake: peptoid molecular transporters. Proc Natl Acad Sci U S A. 97:13003-8.
11. Dorange, F., S. EI Mehdaoui, C. Pichon, P. Coursaget, and J.F. Vautherot. 2000. Marek's disease virus (MDV) homologues of herpes simplex virus type 1 UL49 (VP22) and UL48 (VP16) genes: high-level expression and characterization of MDV-1 VP22 and VP16. J Gen Virol. 81:2219-30.
12. Banerjee-Basu, S., D.W. Sink, and A.D. Baxevanis. 2001. The Homeodomain Resource: sequences, structures, DNA binding sites and genomic information. Nucleic Acids Res. 29:291-3.
13. Park, J., J. Ryu, K.A. Kim, H.J. Lee, J.H. Bahn, K. Han, E.Y. Choi, K.S. Lee, H.Y. Kwon, and S.Y. Choi. 2002. Mutational analysis of a human immunodeficiency virus type 1 Tat protein transduction domain which is required for delivery of an exogenous protein into mammalian cells. J Gen Virol. 83:1173-81.
14. Rothbard, J.B., and P.A. Wender. 2001. Composistions and methods for enhancing drug delivery across biological membranes and tissues. *In* US patent US 09779693.
15. Florkiewicz, R.Z., and A. Baird. 1998. US 6083706: Inhibitors of leaderless protein export. *In* US patent US 6083706. Ciblex Corporation (San Diego, CA).
16. Anderson, D.C., C.A. Morgan, A. P.G., E.J. Nichols, and A.R. Fritzberg. 1988. Covalently-linked complexes and methods for enhanced cytotoxicity and imaging. *In* European patent EP 0359347. Neorx corporation.
17. Schwarze, S.R., and S.F. Dowdy. 2000. In vivo protein transduction: intracellular delivery of biologically active proteins, compounds and DNA. Trends Pharmacol Sci. 21:45-8.
18. Schwarze, S.R., A. Ho, A. Vocero-Akbani, and S.F. Dowdy. 1999. In vivo protein transduction: delivery of a biologically active protein into the mouse. Science. 285:1569-72.
19. Zhong, G., P. Romagnoli, and R.N. Germain. 1997. Related leucinebased cytoplasmic targeting signals in invariant chain and major histocompatibility complex class II molecules control endocytic presentation of distinct determinants in a single protein. J Exp Med. 185:429-38.
20. Escola, J.M., M.J. Kleijmeer, W. Stoorvogel, J.M. Griffith, O. Yoshie, and H.J. Geuze. 1998. Selective enrichment of tetraspan proteins on the internal vesicles of multivesicular endosomes and on exosomes secreted by human B-lymphocytes. J Biol Chem. 273:20121-7.
21. August, T.J., D.M. Pardoll, and F.G. Guarnieri. 1993. Lysosomal targeting of immunogens. *In* US patent US 5633234. The Johns Hopkins University, Baltimore, MD, USA.
22. Sugita, M., R.M. Jackman, E. van Donselaar, S.M. Behar, R.A. Rogers, P.J. Peters, M.B. Brenner, and S.A. Porcelli. 1996. Cytoplasmic taildependent localization of CD1 b antigen-presenting molecules to MIICs. Science. 273:349-52.
23. Pieters, J., 0. Bakke, and B. Dobberstein. 1993. The MHC class IIassociated invariant chain contains two endosomal targeting signals within its cytoplasmic tail. J Cell Sci. 106:831-46.
24. Kornfeld, S., and I. Mellman. 1989. The biogenesis of lysosomes. Annu Rev Cell Biol. 5:483-525.
25. Schluesener, H.J. 1996. Protection against experimental nervous system autoimmune diseases by a human immunodeficiency virus-1 Tat peptide-based polyvalent vaccine. J Neurosci Res. 46:258-62.
26. Larsen, J.N., and H. Lowenstein. 2000. Offical list of allergens, IUIS Allergen Nomenclature Subcommittee. ftp://biobase.dk/pub/who-iuis/allergen. list.
27. Larsen, J.N., and H. Lowenstein. 2001. List of isoallergens and variants, IUIS Allergen Nomenclature Subcommittee. ftp://biobase.dk/pub/whoiuis/isoallergen. list.
28. Xenarios, I., L. Salwinski, X.J. Duan, P. Higney, S.M. Kim, and D. Eisenberg. 2002. DIP, the Database of Interacting Proteins: a research tool for studying cellular networks of protein interactions. Nucleic Acids Res. 30:303-5.
29. Barrett, A.J., J.D. Rawlings, and J.F. Woessner. 1998. Handbook of Proteolytic Enzymes. Academic Press, ISBN 0-12-079371-7*.*
30. Rawlings, N.D., E. O'Brien, and A.J. Barrett. 2002. MEROPS: the protease database. Nucleic Acids Res. 30:343-6.
31. Vlahovicek, K., J. Murvai, E. Barta, and S. Pongor. 2002. The SBASE protein domain library, release 9.0: an online resource for protein domain identification. Nucleic Acids Res. 30:273-5.
32. Sheldon, K., D. Liu, J. Ferguson, and J. Gariepy. 1995. Loligomers: design of de novo peptide-based intracellular vehicles. Proc Natl Acad Sci U S A. 92:2056-60.
33. Dawson, P.E., and S.B. Kent. 2000. Synthesis of native proteins by chemical ligation. Annu Rev Biochem. 69:923-60.
34. Yan, L.Z., and P.E. Dawson. 2001. Synthesis of peptides and proteins without cysteine residues by native chemical ligation combined with desulfurization. J Am Chem Soc. 123:526-33.
35. Garavelli, J.S., Z. Hou, N. Pattabiraman, and R.M. Stephens. 2001. The RESID Database of protein structure modifications and the NRL-3D Sequence-Structure Database. Nucleic Acids Res. 29:199-201.
36. Hruby, V.J., J.M. Ahn, and S. Liao. 1997. Synthesis of oligopeptide and peptidomimetic libraries. Curr Opin Chem Biol. 1:114-9.
37. Senderowitz, H., and R. Rosenfeld. 2001. Design of structural combinatorial libraries that mimic biologic motifs. J Recept Signal Transduct Res. 21:489-506.
38. Sulyok, G.A., C. Gibson, S.L. Goodman, G. Holzemann, M. Wiesner, and H. Kessler. 2001. Solid-phase synthesis of a nonpeptide RGD mimetic library: new selective alphavbeta3 integrin antagonists. J Med Chem. 44:1938-50.
39. Invitrogen. VoyagerTM NES Protein Produktion Kits: Rapid cloning and expression of VP22 fusion proteins in E. coli for translocation of purified recombinant protein into the cytoplasma of mammalian cells. Invitrogen life technologies. Version C 050302 25-0377.
40. Goodman, M., A. Felix, L. Moroder, and C. Toniolo. 2002. Houben-Weyl: Synthesis of Peptides and Peptidomimetics. Thieme Medical and Scientific Publishers. Vol. 22.
41. Hover, J.E. 1975. Remington's Pharmaceutical Sciences. Mack Publishing Co., Easton, PA, USA*.*
42. Libermann, H.A., and L. Lachman. 1980. Pharmaceutical Dosage Forms. Marcel Decker Inc., New York, NY, USA*.*
43. Sambrook, J., and D.W. Russell. 2001. Molecular Cloning - A Laboratory Manual. Cold Spring Harbor Laboratory Press, Cold Spring Harbor, NY, USA. 3rd Edition.
44. Coligan, J.E., A.M. Kruisbeek, D.H. Margulies, E.M. Shevach, W. Strober, and (Editors). 2002. Current Protocols in Immunology. John Wiley & Son, Inc., Hoboken, NJ, USA*.*
45. Moser, M., R. Crameri, G. Menz, T. Schneider, T. Dudler, C. Virchow, M. Gmachl, K. Blaser, and M. Suter. 1992. Cloning and expression of recombinant Aspergillus fumigatus allergen I/a (rAsp f I/a) with IgE binding and type I skin test activity. J Immunol. 149:454-60.
46. Mayer, C., S. Hemmann, A. Faith, K. Blaser, and R. Crameri. 1997. Cloning, production, characterization and IgE cross-reactivity of different manganese superoxide dismutases in individuals sensitized to Aspergillus fumigatus. Int Arch Allergy Immunol. 113:213-5.
47. Qiagen. 2001. The QIAexpressionist: A handbook for high-level expression and purification of 6xHis-tagged proteins. Qiagen GmbH, Hilden, Germany. Fifth Edition: 1-128.
48. Qiagen. 2001. QIAexpress Detection and Assay Handbook. Qiagen GmbH, Hilden Germany. Third Edition:1-100.
49. Beers, M.H., R. Berkow, and (Editors). 1999. The Merck Manual of Diagnosis and Therapy. Merck Research Laboratories, Whitehous Station, NJ, USA. 17th Edition.
50. Coligan, J.E., B.M. Dunn, H.L. Ploegh, D.W. Speicher, P.T. Wingfield, and (Editors). 2002. Current Protocols in Protein Science. John Wiley & Son, Inc., Hoboken, NJ, USA*.*
51. Enna, S.J., M. Williams, J.W. Ferkany, T. Kenakin, R.D. Porsolt, J.P. Sullivan, and (Editors). 2002. Current Protocols in Pharmacology. John Wiley & Son, Inc., Hoboken, NJ, USA*.*
52. Bonifacino, J.S., M. Dasso, J. Lippincott-Schwartz, J.B. Harford, K.M. Yamada, and (Editors). 2002. Current Protocols in Cell Biology. John Wiley & Son, Inc., Hoboken, NJ, USA*.*

## Patentansprüche

1. Verwendung eines modularen Antigen-Transport-Moleküls (MAT-Molekül), enthaltende mindestens ein Translokations-Modul, das den Transport des MAT-Moleküls vom extrazellulären Raum ins Zellinnere bewirkt, mindestens ein Targeting-Modul, das bewirkt, dass das MAT-Molekül intrazellulär zu den Organellen transportiert wird, die an der Prozessierung von Antigenen oder der Beladung von MHC-II Molekülen mit Antigen beteiligt sind und mindestens ein Antigen-Modul, das wenigstens eine aus einem Antigen bestehende Moleküleinheit ist und das die Spezifität einer durch das MAT-Molekül in einem Individuum modulierten Immunantwort bestimmt, zur Herstellung eines Medikaments zur Prophylaxe oder Behandlung von einer Infektionserkrankung, einer Autoimmunerkrankung, Allergie, einer rheumatischen Erkrankung, einer Abstoßreaktion gegen ein transplantiertes Organ und/oder einer malignen Erkrankung.

2. Verwendung nach Anspruch 1, bei dem das Translokationsmodul eine Aminosäuresequenz ist, das Targeting-Modul eine Aminosäuresequenz ist und das Antigen-Modul ein Protein oder Peptid ist.

3. Verwendung nach einem der Ansprüche 1 oder 2, bei dem die Einzelmodule über kovalente Bindungen aneinander gekoppelt sind.

4. Verwendung nach einem der Ansprüche 1 bis 3, bei dem das MAT-Molekül eine lineare Struktur aufweist.

5. Verwendung nach einem der Ansprüche 1 bis 4, bei dem das MAT-Molekül bis zu zwei Antigen-Module, bis zu zwei Targeting-Module und bis zu zwei Translokationsmodule enthält.

6. Verwendung nach einem der Ansprüche 1 bis 5, bei dem zumindest ein Translokations-Modul zumindest eine der nachfolgenden Sequenzen oder zumindest einen funktionellen Sequenzabschnitt dieser Sequenzen für den Transport des MAT-Moleküls vom extrazellulären Raum ins Zellinneren beinhaltet: HIV-tat, Antennapedia, VP22, Polyarginin- oder Polylysin-Sequenzen mit einer Länge von 4 bis 20 Aminosäuren oder zu den vorgenannten invertierte Sequenzen.

7. Verwendung nach einem der Ansprüche 1 oder 6, bei dem zumindest ein Targeting-Modul eine der nachfolgenden Sequenzen oder Strukturen, oder zumindest eine funktionellen Teilsequenz dieser Sequenz beinhaltet: MHC invariante Kette (liP33, liP4I, liP35, liP43), MHC II-beta-Kette, Mannose-6-Phosphatrezeptor, membrangebundenes Immunglobulin (mlg), HLA-DM beta-Kette, LAMP-1, LAMP-2, LAMP-3, Limp 1, CD63, CA82, CD1b oder zu den vorgenannten invertierte Sequenzen.

8. Verwendung nach einem der Ansprüche 1 bis 7, bei dem zumindest ein Antigen-Modul ein Protein, eine Nukleinsäure, eine Zuckerstruktur oder ein Lipid, ist.

9. Verwendung nach Anspruch 8, wobei das Antigen unter Verwendung kombinatorischer Methoden hergestellt werden kann.

10. Verwendung nach einem der Ansprüche 1 bis 9, in dem zusätzlich wenigstens ein Tag-Modul vorhanden ist.

11. Verwendung nach Anspruch 10, wobei zumindest ein Tag-Modul aus der Gruppe His-tag mit 4 bis 6 aufeinander folgenden Histidinresten, Myc-tag. FLAG-tag, HA-tag, Strep-tag, Xpress-tag, S-tag, CBD-tag, GST-tag, HSV-tag, T7-tag, Protein A-tag, V5-tag, Chitin-bindendes tag, Maltose-bindendes tag, Calmodulin-bindendes tag oder eine Biotin-Gruppe ausgewählt wird.

12. Verwendung nach einem der Ansprüche 1 bis 11, bei dem die Module über Spacer-Module miteinander verbunden sind.

13. Verwendung nach einem der Ansprüche 1 bis 12 zur Prophylaxe oder Therapie von Allergien.

14. Verwendung nach Anspruch 13, wobei das Antigen-Modul zumindest ein Allergen ist, das ausgewählt ist aus der Gruppe Feld 1, Bet v 1, Bet v 2, Der p 1, PLA 2.

15. Verwendung nach einem der vorherigen Ansprüche, wobei das Medikament zusätzlich zumindest einen der nachfolgenden Bestandteile und/oder andere pharmazeutisch akzeptable Hilfs- und Zusatzstoffe enthält:
a) eine physiologische Natriumchloridlösung
b) ein pharmazeutisch akzeptables Adjuvans
c) eine pharmazeutisch akzeptable Puffersubstanz
d) ein pharmazeutisch akzeptables Trägerprotein
e) ein pharmazeutisch akzeptabler Konservierungsstoff
f) ein pharmazeutisch akzeptabler Farbstoff.

16. Verwendung einer (i) für das MAT-Molekül nach Anspruch 1 kodierenden Nukleinsäuresequenz, ii) eines Vektors, der mindestens eine Nukleinsäuresequenz, die für ein MAT-Molekül nach einem der vorherigen Ansprüche kodiert, enthält, iii) einer primären Zelle oder Zelllinie, die wenigstens einen Vektor und/oder eine Nukleinsäuresequenz nach i) öder ii) enthält, oder iv) von Exosomen, Deoxosomen oder Liposomen, die wenigstens ein MAT-Molekül nach einem der Ansprüche 1 bis 14, eine dafür kodierende Nukleinsäure oder ein diese Nukleinsäure enthaltenen Vektor enthält, zur Herstellung eines Medikamentes zur Prophylaxe oder Behandlung einher Infektionserkrankung, einer Autoimmunerkrankung, Allergie, einer rheumatischen Erkrankung, einer Abstoßreaktion gegen ein transplantiertes Organ und/oder einer malignen Erkrankung.

17. Verwendung nach einem der vorliegenden Ansprüche, wobei das Medikament ein Impfstoff ist.

18. Verwendung nach einem der vorherigen Ansprüche, **dadurch gekennzeichnet, dass** das Präparat für die Injektion in einen Lymphknoten oder für die Applikation über die Schleimhäute ausgelegt ist.

## Claims

1. The use of a modular antigen-transport-molecule (MAT-molecule) containing at least one translocation module which brings about the transport of the MAT-molecule from the extracellular space into the interior of the cells, at least one targeting module which brings about the transport of the MAT-molecule intracellularly to the organelles which are involved in the processing of antigens or the loading of MHC-molecules with antigens and at least one antigen molecule unit consisting of the antigen and which determines the specificity of an immune response modulated by the MAT-molecule in an individual for the preparation of a medicament for the prophylaxis or treatment of an infectious disease, an autoimmune disease, an allergy, a rheumatic disorder, a rejection reaction against a transplanted organ and/or a malignant disease.

2. The use according to claim 1 in which the translocation module being an amino acid sequence and the targeting module being an amino acid sequence and the antigen module is a protein or peptide.

3. The use according to any one of claims 1 or 2 in which the individual modules are coupled to one another by covalent linkage.

4. The use according to any one of claims 1 to 3 wherein the MAT-molecule has a linear structure.

5. The use according to any one of claims 1 to 4 wherein the MAT-molecule contains up to two antigen modules, up to two targeting modules and up to two translocation modules.

6. The use according to any one of claims 1 to 5, in which the at least one of the translocation modules comprises at least one of the following sequences or at least one partial functional sequence of these sequences for the transport of the MAT-molecule from the extracellular space into the interior of the cells: HIV-tat, antennapedia, VP22, polyarginine or polylysine sequences having a length of from 4 to 20 amino acids or sequences, which are inverted relative to the afore-mentioned.

7. The use according to any one of claims 1 to 6 in which the at least one of the targeting modules comprises at least one of the following sequences or structures or at least a functional partial sequence of these sequences: MHC invariant chain (IiP33, IiP41, IiP35, IiP43), MHC II beta chain, mannose 6-phosphate receptor, membrane-bound immunoglobulin (mIg), HLA-DM beta chain, LAMP-1, LAMP-2, LAMP-3, Limp 1, CD63, CD82, CD1b or sequences which are inverted relative to the afore-mentioned.

8. The use according to any one of claims 1 to 7 wherein at least one antigen module is a protein, nucleic acid, a sugar structure or a lipid.

9. The use according to claim 8 wherein the antigen is produced by combinatorial methods.

10. The use according to any one of claims 1 to 9 in which in addition at least one Tag-module is present.

11. The use according to claim 10 in which the at least Tag module is selected from the group of His-Tag having 4 to 6 consecutive histidine residues, Myc-tag, FLAG-tag, HA-tag, Strep-tag, Xpress-tag, Stag, CBD-tag, GST-tag, HSV-tag, T7-tag, protein A-tag, V5-tag, chitin binding tag, maltose binding tag, calmodulin binding tag or a biotin group.

12. The use according to any one of claims 1 to 11 in which the modules are covalently connected via spacer modules.

13. The use according to any one of claims 1 to 12 for the prophylaxis or therapy of allergies.

14. The use according to claim 13 wherein the antigen module is at least one allergen selected from the group of FeL d1, Bet v 1, Bet v2, Der p 1, PLA 2.

15. The use according to any one of the preceding claims wherein the medicament contains in addition any one of the following constituents and/or other pharmaceutically acceptable excipients and additives:
a) a physiological sodium chloride solution
b) a pharmaceutically acceptable adjuvant
c) a pharmaceutically acceptable buffer substance
d) a pharmaceutically acceptable carrier protein
e) a pharmaceutically acceptable preservative
f) a pharmaceutical colorant

16. The use of i) a nucleic acid sequence encoding the MAT-molecule according to claim 1, ii) a vector containing at least a nucleic acid sequence encoding a MAT-molecule according to any one of the preceding claims, iii) a primary cell or cell line containing at least a vector and/or a nucleic acid sequence according to i) or ii), or iv) exosomes, deoxosomes or liposomes, containing at least one MAT-molecule according to any one of claims 1 to 14, a nucleic acid encoding the same or a vector containing said nucleic acid, for the production of a medicament for the prophylaxis or treatment of infectious disease, autoimmune disease, allergy, rheumatic disorder, a rejection reaction against a transplanted organ and/or a malignant disease.

17. The use according to any one of the preceding claims wherein the medicament is a vaccine.

18. The use according to any one of the preceding claims **characterized in that** the preparation is designed for injection into a lymph node or for the administration via the mucous membrane.

## Revendications

1. Utilisation d'une molécule modulaire de transport d'antigènes (molécule MAT) contenant au moins un module de translocation, qui assure le transport de la molécule MAT de l'espace extracellulaire à l'intérieur de la cellule, au moins un module de ciblage, qui provoque le transport intracellulaire de la molécule MAT vers les organelles qui participent à la maturation d'antigènes ou au chargement de molécules MHC-II par un antigène, et au moins un module antigène, qui est au moins une unité moléculaire consistant en un antigène, et qui détermine la spécificité d'une réponse immune modulée par la molécule MAT chez un individu, pour la fabrication d'un médicament destiné à la prophylaxie ou au traitement d'une maladie infectieuse, d'une maladie auto-immune, d'une allergie, d'une maladie rhumatismale, d'une réaction de rejet d'un organe transplanté et/ou d'une maladie maligne.

2. Utilisation selon la revendication 1, pour laquelle le module de translocation est une séquence d'acides aminés, le module de ciblage est une séquence d'acides aminés, et le module antigène est une protéine ou un peptide.

3. Utilisation selon l'une des revendications 1 ou 2, pour laquelle les modules individuels sont couplés les uns aux autres par l'intermédiaire de liaisons covalentes.

4. Utilisation selon l'une des revendications 1 à 3, pour laquelle la molécule MAT présente une structure linéaire.

5. Utilisation selon l'une des revendications 1 à 4, pour laquelle la molécule MAT contient jusqu'à deux modules antigènes, jusqu'à deux modules de ciblage et jusqu'à deux modules de translocation.

6. Utilisation selon l'une des revendications 1 à 5, pour laquelle au moins un module de translocation contient au moins l'une des séquences suivantes ou au moins un segment de séquence fonctionnel de ces séquences pour le transport de la molécule MAT de l'espace extracellulaire à l'intérieur de la cellule : VIH-tat, Antennapedia, VP22, les séquences de polyarginine ou de polylysine ayant une longueur de 4 à 20 acides aminés, ou les séquences inversées par rapport aux séquences mentionnées ci-dessus.

7. Utilisation selon l'une des revendications 1 à 6, pour laquelle au moins une séquence de ciblage contient l'une des séquences ou structures suivantes, ou au moins une séquence partielle fonctionnelle de cette séquence : la chaîne invariante du CMH (liP33, liP41, liP35, liP43), la chaîne bêta-II du CMH, le récepteur du mannose-6-phosphate, l'immunoglobuline liée à la membrane (mig), la chaîne bêta de HLA-DM, LAMP-1, LAMP-2, LAMP-3, Limp 1, CD63, CD82, CD1b, ou les séquences inversées par rapport aux séquences mentionnées ci-dessus.

8. Utilisation selon l'une des revendications 1 à 7, pour laquelle au moins un module antigène est une protéine, un acide nucléique, une structure sucre ou un lipide.

9. Utilisation selon la revendication 8, pour laquelle l'antigène peut être fabriqué par utilisation de méthodes combinatoires.

10. Utilisation selon l'une des revendications 1 à 9, dans laquelle au moins un module étiquette est en outre présent.

11. Utilisation selon la revendication 10, pour laquelle au moins un module d'étiquette est choisi dans le groupe consistant en l'étiquette His ayant 4 à 6 résidus d'histidine successifs, l'étiquette Myc, l'étiquette FLAG, l'étiquette HA, l'étiquette Strep, l'étiquette X-press, l'étiquette S, l'étiquette CBD, l'étiquette GST, l'étiquette HSV, l'étiquette T7, l'étiquette protéine A, l'étiquette V5, l'étiquette de liaison à la chitine, l'étiquette de liaison au maltose, l'étiquette de liaison à la calmoduline ou un groupe biotine.

12. Utilisation selon l'une des revendications 1 à 11, pour laquelle les modules sont reliés les uns aux autres par l'intermédiaire de modules espaceurs.

13. Utilisation selon l'une des revendications 1 à 12, pour la prophylaxie ou le traitement d'allergies.

14. Utilisation selon la revendication 13, pour laquelle le module antigène est au moins un allergène qui est choisi dans le groupe consistant en Feld 1, Bet v 1, Bet v 2, Der p 1, PLA 2.

15. Utilisation selon l'une des revendications précédentes, pour laquelle le médicament contient en outre au moins l'un des constituants suivants et/ou d'autres adjuvants et additifs pharmaceutiquement acceptables :
a) une solution physiologique de chlorure de sodium,
b) un adjuvant pharmaceutiquement acceptable,
c) une substance tampon pharmaceutiquement acceptable,
d) une protéine support pharmaceutiquement acceptable,
e) un conservateur pharmaceutiquement acceptable,
f) un colorant pharmaceutiquement acceptable.

16. Utilisation (i) d'une séquence d'acide nucléique codant pour la molécule MAT selon la revendication 1, (ii) d'un vecteur qui contient au moins une séquence d'acide nucléique qui code pour une molécule MAT selon l'une des revendications précédentes, (iii) d'une cellule ou d'une lignée cellulaire primaire qui contient au moins un vecteur et/ou une séquence d'acide nucléique selon (i) ou (ii), et (iv) d'exosomes, de désoxosomes ou de liposomes, qui contiennent au moins une molécule MAT selon l'une des revendications 1 à 14, un acide nucléique codant pour cette dernière ou un vecteur contenant cet acide nucléique, pour la fabrication d'un médicament destiné à la prophylaxie ou au traitement d'une maladie infectieuse, d'une maladie auto-immune, une allergie, d'une maladie rhumatismale, d'une réaction de rejet d'un organe transplanté et/ou d'une maladie maligne.

17. Utilisation selon l'une des revendications précédentes, pour laquelle le médicament est un vaccin.

18. Utilisation selon l'une des revendications précédentes, **caractérisée en ce que** la préparation est conçue pour injection dans un ganglion lymphatique, ou pour administration à travers les muqueuses.


### SEQUENCE LISTING

<110> BioVisioN AG
<120> Moduläre Antigen-Transporter Molekule (MAT-Moleküle) zur
   Modulierung von Immunreaktionen
<130> MAT
<150> EP02022774.0
<151> 2002-10-11
<160> 20
<170> Patent In version 3.1
<210> 1
   <211> 870
   <212> DNA
   <213> Human immunodeficiency virus + Homo sapiens + Aspergillus fumigatus
<400> 1
   atgagaggat cgcatcacca tcaccatcac ggatctggtt acggtcgtaa aaagcgtcgc 60
   cagcgtcgcc gtggatctat ggatgaccag cacgacctta tctccaacaa tgagcaactg 120
   cccatgctgg gccggcgccc tggggccccg gagagcaagt gcagccgcgg agccctgtac 180
   acaggctttt ccatcctggt gactctgctc ctcgctggcc aggccaccac cgcctacttc 240
   ctgtaccagc agcagggccg gctggacaaa ctgacagtca cctcccagaa cttgcagctg 300
   gagaacctgc gcatgaaact tcccaagcct cccaagcctg tgagcaagat gcgcatggcc 360
   accccgctgc tgatgcaggc gctgcccatg ggagccctgc cccaggggac tagtggatcc 420
   gcgacctgga catgcatcaa ccaacagctg aatcccaaga caaacaaatg ggaagacaag 480
   cggcttctat acagtcaagc caaagccgaa agcaactccc accacgcacc tctttccgac 540
   ggcaagaccg gtagcagcta cccgcactgg ttcactaacg gctacgacgg gaatggcaag 600
   ctcatcaagg gtcgcacgcc catcaaattc ggaaaagccg actgtgaccg tcccccgaag 660
   cgcagccaga acggcatggg caaggatgac cactacctgc tggagttccc gacttttcca 720
   gatggccacg actataagtt tgactcgaag aaacccaagg aagacccggg cccagcgagg 780
   gtcatctata cttatcccaa caaggtgttt tgcggcattg tggcccatca gcgggggaat 840
   cagggagact tgagactgtg ttctcattag 870
<210> 2
   <211> 289
   <212> PRT
   <213> Human immunodeficiency virus + Homo sapiens + Aspergillus fumigatus
<400> 2
   Met Arg Gly Ser His His His His His His Gly Ser Gly Tyr Gly Arg
   1               5                   10                  15
   Lys Lys Arg Arg Gln Arg Arg Arg Gly Ser Met Asp Asp Gln His Asp
               20                  25                  30
   Leu Ile Ser Asn Asn Glu Gln Leu Pro Met Leu Gly Arg Arg Pro Gly
           35                  40                  45
   Ala Pro Glu Ser Lys Cys Ser Arg Gly Ala Leu Tyr Thr Gly Phe Ser
       50                  55                  60
   Ile Leu Val Thr Leu Leu Leu Ala Gly Gln Ala Thr Thr Ala Tyr Phe
   65                  70                  75                  80
   Leu Tyr Gln Gln Gln Gly Arg Leu Asp Lys Leu Thr Val Thr Ser Gln
                   85                  90                  95
   Asn Leu Gln Leu Glu Asn Leu Arg Met Lys Leu Pro Lys Pro Pro Lys
               100                 105                 110
   Pro Val Ser Lys Met Arg Met Ala Thr Pro Leu Leu Met Gln Ala Leu
           115                 120                 125
   Pro Met Gly Ala Leu Pro Gln Gly Thr Ser Gly Ser Ala Thr Trp Thr
       130                 135                 140
   Cys Ile Asn Gln Gln Leu Asn Pro Lys Thr Asn Lys Trp Glu Asp Lys
   145                 150                 155                 160
   Arg Leu Leu Tyr Ser Gln Ala Lys Ala Glu Ser Asn Ser His His Ala
                   165                 170                 175
   Pro Leu Ser Asp Gly Lys Thr Gly Ser Ser Tyr Pro His Trp Phe Thr
               180                 185                 190
   Asn Gly Tyr Asp Gly Asn Gly Lys Leu Ile Lys Gly Arg Thr Pro Ile
           195                 200                 205
   Lys Phe Gly Lys Ala Asp Cys Asp Arg Pro Pro Lys Arg Ser Gln Asn
       210                 215                 220
   Gly Met Gly Lys Asp Asp His Tyr Leu Leu Glu Phe Pro Thr Phe Pro
   225                 230                 235                 240
   Asp Gly His Asp Tyr Lys Phe Asp Ser Lys Lys Pro Lys Glu Asp Pro
                   245                 250                 255
   Gly Pro Ala Arg Val Ile Tyr Thr Tyr Pro Asn Lys Val Phe Cys Gly
               260                 265                 270
   Ile Val Ala His Gln Arg Gly Asn Gln Gly Asp Leu Arg Leu Cys Ser
           275                 280                 285
   His
<210> 3
   <211> 1044
   <212> DNA
   <213> Human immunodeficiency virus + Homo sapiens + Aspergillus fumigatus
<400> 3
   atgagaggat cgcatcacca tcaccatcac ggatctggtt acggtcgtaa aaagcgtcgc     60
   cagcgtcgcc gtggatctat ggatgaccag cacgacctta tctccaacaa tgagcaactg    120
   cccatgctgg gccggcgccc tggggccccg gagagcaagt gcagccgcgg agccctgtac    180
   acaggctttt ccatcctggt gactctgctc ctcgctggcc aggccaccac cgcctacttc    240
   ctgtaccagc agcagggccg gctggacaaa ctgacagtca cctcccagaa cttgcagctg    300
   gagaacctgc gcatgaaact tcccaagcct cccaagcctg tgagcaagat gcgcatggcc    360
   accccgctgc tgatgcaggc gctgcccatg ggagccctgc cccaggggac tagtggatct    420
   caatacacgc tcccacccct cccctacccc tacgatgccc tccaacccta catctcccaa    480
   cagatcatgg agctgcacca caaaaagcac catcaaacct acgtcaatgg cctgaatgcc    540
   gcactcgagg cgcagaagaa agcggcggaa gccaccgacg tccccaagct cgtctccgtg    600
   cagcaagcga tcaaattcaa cggcgggggg cacatcaacc attccctctt ctggaagaat    660
   ctggccccgg agaaatccgg gggtggcaag atcgatcagg caccggtcct caaagcagcc    720
   atcgagcagc gttggggatc cttcgataag ttcaaggatg ctttcaacac gaccctgctg    780
   ggcattcagg gcagcggatg gggttggtta gtgaccgacg gacccaaggg aaagctagac     840
   attaccacaa cccacgacca ggatccggtg accggggcgg cccccgtctt tggggtggat    900
   atgtgggagc atgcttacta ccttcagtac ttgaacgaca aagcctcgta tgccaagggc    960
   atctggaacg tgatcaactg ggctgaagcg gagaatcggt acatagcggg tgacaagggt   1020
   ggacacccat tcatgaagct gtag                                          1044
<210> 4
   <211> 347
   <212> PRT
   <213> Human immunodeficiency virus + Homo sapiens + Aspergillus fumigatus
<400> 4
   Met Arg Gly Ser His His His His His His Gly Ser Gly Tyr Gly Arg
   1 5 10 15
   Lys Lys Arg Arg Gln Arg Arg Arg Gly Ser Met Asp Asp Gln His Asp
   20 25 30
   Leu Ile Ser Asn Asn Glu Gln Leu Pro Met Leu Gly Arg Arg Pro Gly
   35 40 45
   Ala Pro Glu Ser Lys Cys Ser Arg Gly Ala Leu Tyr Thr Gly Phe Ser
   50 55 60
   Ile Leu Val Thr Leu Leu Leu Ala Gly Gln Ala Thr Thr Ala Tyr Phe
   65 70 75 80
   Leu Tyr Gln Gln Gln Gly Arg Leu Asp Lys Leu Thr Val Thr Ser Gln
   85 90 95
   Asn Leu Gln Leu Glu Asn Leu Arg Met Lys Leu Pro Lys Pro Pro Lys
   100 105 110
   Pro Val Ser Lys Met Arg Met Ala Thr Pro Leu Leu Met Gln Ala Leu
   115 120 125
   Pro Met Gly Ala Leu Pro Gln Gly Thr Ser Gly Ser Gln Tyr Thr Leu
   130 135 140
   Pro Pro Leu Pro Tyr Pro Tyr Asp Ala Leu Gln Pro Tyr Ile Ser Gln
   145 150 155 160
   Gln Ile Met Glu Leu His His Lys Lys His His Gln Thr Tyr Val Asn
   165 170 175
   Gly Leu Asn Ala Ala Leu Glu Ala Gln Lys Lys Ala Ala Glu Ala Thr
   180 185 190
   Asp Val Pro Lys Leu Val Ser Val Gln Gln Ala Ile Lys Phe Asn Gly
   195 200 205
   Gly Gly His Ile Asn His Ser Leu Phe Trp Lys Asn Leu Ala Pro Glu
   210 215 220
   Lys Ser Gly Gly Gly Lys Ile Asp Gln Ala Pro Val Leu Lys Ala Ala
   225 230 235 240
   Ile Glu Gln Arg Trp Gly Ser Phe Asp Lys Phe Lys Asp Ala Phe Asn
   245 250 255
   Thr Thr Leu Leu Gly Ile Gln Gly Ser Gly Trp Gly Trp Leu Val Thr
   260 265 270
   Asp Gly Pro Lys Gly Lys Leu Asp Ile Thr Thr Thr His Asp Gln Asp
   275 280 285
   Pro Val Thr Gly Ala Ala Pro Val Phe Gly Val Asp Met Trp Glu His
   290 295 300
   Ala Tyr Tyr Leu Gln Tyr Leu Asn Asp Lys Ala Ser Tyr Ala Lys Gly
   305 310 315 320
   Ile Trp Asn Val Ile Asn Trp Ala Glu Ala Glu Asn Arg Tyr Ile Ala
   325 330 335
   Gly Asp Lys Gly Gly His Pro Phe Met Lys Leu
   340 345
<210> 5
   <211> 903
   <212> DNA
   <213> Human immunodeficience virus + Homo sapiens + Betula verrucosa
<400> 5
   atgagaggat cgcatcacca tcaccatcac ggatctggtt acggtcgtaa aaagcgtcgc 60
   cagcgtcgcc gtggatctat ggatgaccag cacgacctta tctccaacaa tgagcaactg 120
   cccatgctgg gccggcgccc tggggccccg gagagcaagt gcagccgcgg agccctgtac 180
   acaggctttt ccatcctggt gactctgctc ctcgctggcc aggccaccac cgcctacttc 240
   ctgtaccagc agcagggccg gctggacaaa ctgacagtca cctcccagaa cttgcagctg 300
   gagaacctgc gcatgaaact tcccaagcct cccaagcctg tgagcaagat gcgcatggcc 360
   accccgctgc tgatgcaggc gctgcccatg ggagccctgc cccaggggac tagtggatcc 420
   atgggtgttt tcaactacga aaccgaaacc acctccgtta tcccggctgc tcgtctgttc 480
   aaggccttca tcctggacgg tgacaacctg ttccctaagg ttgctccgca ggctatctcc 540
   tccgttgaaa acatcgaagg taacggtggc ccgggtacca tcaagaaaat ctccttcccg 600
   gaaggtttcc catttaaata cgtaaaagac cgtgttgacg aagttgacca caccaacttc 660
   aaatacaact actccgttat cgaaggtggt ccaattggtg acaccctgga aaaaatctcc    720
   aacgaaatca aaatcgtggc aaccccggac ggtggttcca tccttaagat ctccaacaaa    780
   taccacacca aaggtgacca cgaagttaaa gctgaacagg ttaaagcttc gaaagaaatg    840
   ggtgaaaccc tgctgcgtgc tgttgaatcc tacctgctgg ctcactccga tgcatacaac    900
   taa                                                                   903
<210> 6
   <211> 300
   <212> PRT
   <213> Human immunodeficiency virus + Homo sapiens + Betula verrucosa
<400> 6
<210> 7
   <211> 1089
   <212> DNA
   <213> Human immunodeficiency virus + Homo sapiens + Dermatophagoides
   pteronyssinus
<400> 7
   atgagaggat cgcatcacca tcaccatcac ggatctggtt acggtcgtaa aaagcgtcgc 60
   cagcgtcgcc gtggatctat ggatgaccag cacgacctta tctccaacaa tgagcaactg 120
   cccatgctgg gccggcgccc tggggccccg gagagcaagt gcagccgcgg agccctgtac 180
   acaggctttt ccatcctggt gactctgctc ctcgctggcc aggccaccac cgcctacttc 240
   ctgtaccagc agcagggccg gctggacaaa ctgacagtca cctcccagaa cttgcagctg 300
   gagaacctgc gcatgaaact tcccaagcct cccaagcctg tgagcaagat gcgcatggcc 360
   accccgctgc tgatgcaggc gctgcccatg ggagccctgc cccaggggac tagtggatcc 420
   actaacgcat gtagtatcaa tggaaatgct ccagctgaaa tcgatttgcg acaaatgcga 480
   actgtcactc ccattcgtat gcaaggaggc tgtggttcat gttgggcttt ctctggtggt 540
   gccgcaactg aatcagctta tttggctcac cgtaatcaat cattggatct tgctgaacaa 600
   gaattagtcg attgtgcttc ccaacacggt tgtcatggtg ataccattcc acgtggtatt     660
   gaatacatcc aacataatgg tgtcgtccaa gaaagctact atcgatacgt tgcacgagaa     720
   caatcatgcc gacgaccaaa tgcacaacgt ttcggtatct caaactattg ccaaatttac     780
   ccaccaaatg caaacaaaat tcgtgaagct ttggctcaaa cccacagcgc tattgccgtc     840
   attattggca tcaaagattt agacgcattc cgtcattatg atggccgaac aatcattcaa     900
   cgcgataatg gttaccaacc aaactatcac gctgtcaaca ttgttggtta cagtaacgca     960
   caaggtgtcg attattggat cgtacgaaac agttgggata ccaattgggg tgataatggt    1020
   tacggttatt ttgctgccaa catcgatttg atgatgattg aagaatatcc atatgttgtc    1080
   attctctaa                                                            1089
<210> 8
   <211> 362
   <212> PRT
   <213> Human immunodeficiency virus + Homo sapiens + Dermatophagoides
   pteronyssinus
<400> 8
<210> 9
   <211> 39
   <212> DNA
   <213> Human immunodeficiency virus
<400> 9
   ggttacggtc gtaaaaagcg tcgccagcgt cgccgtgga 39
<210> 10
   <211> 13
   <212> PRT
   <213> Human immunodeficiency virus
<400> 10
   Gly Tyr Gly Arg Lys Lys Arg Arg Gln Arg Arg Arg Gly
1 5 10
<210> 11
   <211> 429
   <212> DNA
   <213> Homo sapiens
<400> 11
   atgagaggat cgcatcacca tcaccatcac ggatctggtt acggtcgtaa aaagcgtcgc 60
   cagcgtcgcc gtggatctat ggatgaccag cacgacctta tctccaacaa tgagcaactg 120
   cccatgctgg gccggcgccc tggggccccg gagagcaagt gcagccgcgg agccctgtac 180
   acaggctttt ccatcctggt gactctgctc ctcgctggcc aggccaccac cgcctacttc 240
   ctgtaccagc agcagggccg gctggacaaa ctgacagtca cctcccagaa cttgcagctg 300
   gagaacctgc gcatgaaact tcccaagcct cccaagcctg tgagcaagat gcgcatggcc 360
   accccgctgc tgatgcaggc gctgcccatg ggagccctgc cccaggggac tagtctgcag 420
   aagcttaat 429
<210> 12
   <211> 143
   <212> PRT
   <213> Homo sapiens
<400> 12
   Met Arg Gly Ser His His His His His His Gly Ser Gly Tyr Gly Arg
   1               5                   10                  15
   Lys Lys Arg Arg Gln Arg Arg Arg Gly Ser Met Asp Asp Gln His Asp
               20                  25                  30
   Leu Ile Ser Asn Asn Glu Gln Leu Pro Met Leu Gly Arg Arg Pro Gly
           35                  40                  45
   Ala Pro Glu Ser Lys Cys Ser Arg Gly Ala Leu Tyr Thr Gly Phe Ser
       50                  55                  60
   Ile Leu Val Thr Leu Leu Leu Ala Gly Gln Ala Thr Thr Ala Tyr Phe
   65                  70                  75                  80
   Leu Tyr Gln Gln Gln Gly Arg Leu Asp Lys Leu Thr Val Thr Ser Gln
                   85                  90                  95
   Asn Leu Gln Leu Glu Asn Leu Arg Met Lys Leu Pro Lys Pro Pro Lys
               100                 105                 110
   Pro Val Ser Lys Met Arg Met Ala Thr Pro Leu Leu Met Gln Ala Leu
           115                 120                 125
   Pro Met Gly Ala Leu Pro Gln Gly Thr Ser Leu Gln Lys Leu Asn
       130                 135                 140
<210> 13
   <211> 450
   <212> DNA
   <213> Aspergillus fumigatus
<400> 13
   gcgacctgga catgcatcaa ccaacagctg aatcccaaga caaacaaatg ggaagacaag     60
   cggcttctat acagtcaagc caaagccgaa agcaactccc accacgcacc tctttccgac    120
   ggcaagaccg gtagcagcta cccgcactgg ttcactaacg gctacgacgg gaatggcaag    180
   ctcatcaagg gtcgcacgcc catcaaattc ggaaaagccg actgtgaccg tcccccgaag    240
   cacagccaga acggcatggg caaggatgac cactacctgc tggagttccc gacttttcca    300
   gatggccacg actataagtt tgactcgaag aaacccaagg aagacccggg cccagcgagg    360
   gtcatctata cttatcccaa caaggtgttt tgcggcattg tggcccatca gcgggggaat    420
   cagggagact tgagactgtg ttctcattag                                     450
<210> 14
   <211> 149
   <212> PRT
   <213> Aspergillus fumigatus
<400> 14
   Ala Thr Trp Thr Cys Ile Asn Gln Gln Leu Asn Pro Lys Thr Asn Lys
   1               5                   10                  15
   Trp Glu Asp Lys Arg Leu Leu Tyr Ser Gln Ala Lys Ala Glu Ser Asn
               20                  25                  30
   Ser His His Ala Pro Leu Ser Asp Gly Lys Thr Gly Ser Ser Tyr Pro
           35                  40                  45
   His Trp Phe Thr Asn Gly Tyr Asp Gly Asn Gly Lys Leu Ile Lys Gly
       50                  55                  60
   Arg Thr Pro Ile Lys Phe Gly Lys Ala Asp Cys Asp Arg Pro Pro Lys
   65                  70                  75                  80
   His Ser Gln Asn Gly Met Gly Lys Asp Asp His Tyr Leu Leu Glu Phe
                   85                  90                  95
   Pro Thr Phe Pro Asp Gly His Asp Tyr Lys Phe Asp Ser Lys Lys Pro
               100                 105                 110
   Lys Glu Asp Pro Gly Pro Ala Arg Val Ile Tyr Thr Tyr Pro Asn Lys
           115                 120                 125
   Val Phe Cys Gly Ile Val Ala His Gln Arg Gly Asn Gln Gly Asp Leu
       130                 135                 140
   Arg Leu Cys Ser His
   145
<210> 15
   <211> 633
   <212> DNA
   <213> Aspergillus fumigatus
<400> 15
   atgtcacagc aatacacgct cccacccctc ccctacccct acgatgccct ccaaccctac      60
   atctcccaac agatcatgga gctgcaccac aaaaagcacc atcaaaccta cgtcaatggc     120
   ctgaatgccg cactcgaggc gcagaagaaa gcggcggaag ccaccgacgt ccccaagctc     180
   gtctccgtgc agcaagcgat caaattcaac ggcggggggc acatcaacca ttccctcttc     240
   tggaagaatc tggccccgga gaaatccggg ggtggcaaga tcgatcaggc accggtcctc     300
   aaagcagcca tcgagcagcg ttggggatcc ttcgataagt tcaaggatgc tttcaacacg     360
   accctgctgg gcattcaggg cagcggatgg ggttggttag tgaccgacgg acccaaggga     420
   aagctagaca ttaccacaac ccacgaccag gatccggtga ccggggcggc ccccgtcttt     480
   ggggtggata tgtgggagca tgcttactac cttcagtact tgaacgacaa agcctcgtat     540
   gccaagggca tctggaacgt gatcaactgg gctgaagcgg agaatcggta catagcgggt     600
   gacaagggtg gacacccatt catgaagctg tag                                  633
<210> 16
   <211> 210
   <212> PRT
   <213> Aspergillus fumigatus
<400> 16
   Met Ser Gln Gln Tyr Thr Leu Pro Pro Leu Pro Tyr Pro Tyr Asp Ala
   1               5                   10                  15
   Leu Gln Pro Tyr Ile Ser Gln Gln Ile Met Glu Leu His His Lys Lys
               20                  25                  30
   His His Gln Thr Tyr Val Asn Gly Leu Asn Ala Ala Leu Glu Ala Gln
           35                  40                  45
   Lys Lys Ala Ala Glu Ala Thr Asp Val Pro Lys Leu Val Ser Val Gln
       50                  55                  60
   Gln Ala Ile Lys Phe Asn Gly Gly Gly His Ile Asn His Ser Leu Phe
   65                  70                  75                  80
   Trp Lys Asn Leu Ala Pro Glu Lys Ser Gly Gly Gly Lys Ile Asp Gln
                   85                  90                  95
   Ala Pro Val Leu Lys Ala Ala Ile Glu Gln Arg Trp Gly Ser Phe Asp
               100                 105                 110
   Lys Phe Lys Asp Ala Phe Asn Thr Thr Leu Leu Gly Ile Gln Gly Ser
           115                 120                 125
   Gly Trp Gly Trp Leu Val Thr Asp Gly Pro Lys Gly Lys Leu Asp Ile
       130                 135                 140
   Thr Thr Thr His Asp Gln Asp Pro Val Thr Gly Ala Ala Pro Val Phe
   145                 150                 155                 160
   Gly Val Asp Met Trp Glu His Ala Tyr Tyr Leu Gln Tyr Leu Asn Asp
   165 170 175
   Lys Ala Ser Tyr Ala Lys Gly Ile Trp Asn Val Ile Asn Trp Ala Glu
   180 185 190
   Ala Glu Asn Arg Tyr Ile Ala Gly Asp Lys Gly Gly His Pro Phe Met
   195 200 205
   Lys Leu
   210
<210> 17
   <211> 519
   <212> DNA
   <213> Betula verrucosa
<400> 17
   atgagaggat cgcatcacca tcaccatcac ggatccatgg gtgttttcaa ctacgaaacc     60
   gaaaccacct ccgttatccc ggctgctcgt ctgttcaagg ccttcatcct ggacggtgac    120
   aacctgttcc ctaaggttgc tccgcaggct atctcctccg ttgaaaacat cgaaggtaac    180
   ggtggcccgg gtaccatcaa gaaaatctcc ttcccggaag gtttcccatt taaatacgta    240
   aaagaccgtg ttgacgaagt tgaccacacc aacttcaaat acaactactc cgttatcgaa    300
   ggtggtccaa ttggtgacac cctggaaaaa atctccaacg aaatcaaaat cgtggcaacc    360
   ccggacggtg gttccatcct taagatctcc aacaaatacc acaccaaagg tgaccacgaa    420
   gttaaagctg aacaggttaa agcttcgaaa gaaatgggtg aaaccctgct gcgtgctgtt    480
   gaatcctacc tgctggctca ctccgatgca tacaactaa                           519
<210> 18
   <211> 172
   <212> PRT
   <213> Betula verrucosa
<400> 18
   Met Arg Gly Ser His His His His His His Gly Ser Met Gly Val Phe
   1 5 10 15
   Asn Tyr Glu Thr Glu Thr Thr Ser Val Ile Pro Ala Ala Arg Leu Phe
   20 25 30
   Lys Ala Phe Ile Leu Asp Gly Asp Asn Leu Phe Pro Lys Val Ala Pro
   35 40 45
   Gln Ala Ile Ser Ser Val Glu Asn Ile Glu Gly Asn Gly Gly Pro Gly
   50 55 60
   Thr Ile Lys Lys Ile Ser Phe Pro Glu Gly Phe Pro Phe Lys Tyr Val
   65 70 75 80
   Lys Asp Arg Val Asp Glu Val Asp His Thr Asn Phe Lys Tyr Asn Tyr
   85 90 95
   Ser Val Ile Glu Gly Gly Pro Ile Gly Asp Thr Leu Glu Lys Ile Ser
   100 105 110
   Asn Glu Ile Lys Ile Val Ala Thr Pro Asp Gly Gly Ser Ile Leu Lys
   115 120 125
   Ile Ser Asn Lys Tyr His Thr Lys Gly Asp His Glu Val Lys Ala Glu
   130 135 140
   Gln Val Lys Ala Ser Lys Glu Met Gly Glu Thr Leu Leu Arg Ala Val
   145 150 155 160
   Glu Ser Tyr Leu Leu Ala His Ser Asp Ala Tyr Asn
   165 170
<210> 19
   <211> 1099
   <212> DNA
   <213> Dermatophagoides pteronyssinus
<400> 19
   gaattccttt tttttctttt ctctctctaa aatctaaaat ccatccaaca tgaaaattgt      60
   tttggccatc gcctcattgt tggcattgag cgctgtttat gctcgtccat catcgatcaa     120
   aacttttgaa gaatacaaaa aagccttcaa caaaagttat gctaccttcg aagatgaaga     180
   agctgcccgt aaaaactttt tggaatcagt aaaatatgtt caatcaaatg gaggtgccat     240
   caaccatttg tccgatttgt cgttggatga attcaaaaac cgatttttga tgagtgcaga     300
   agcttttgaa cacctcaaaa ctcaattcga tttgaatgct gaaactaacg cctgcagtat     360
   caatggaaat gctccagctg aaatcgattt gcgacaaatg cgaactgtca ctcccattcg     420
   tatgcaagga ggctgtggtt catgttgggc tttctctggt gttgccgcaa ctgaatcagc     480
   ttatttggct taccgtaatc aatcattgga tcttgctgaa caagaattag tcgattgtgc     540
   ttcccaacac ggttgtcatg gtgataccat tccacgtggt attgaataca tccaacataa     600
   tggtgtcgtc caagaaagct actatcgata cgttgcacga gaacaatcat gccgacgacc     660
   aaatgcacaa cgtttcggta tctcaaacta ttgccaaatt tacccaccaa atgtaaacaa     720
   aattcgtgaa gctttggctc aaacccacag cgctattgcc gtcattattg gcatcaaaga     780
   tttagacgca ttccgtcatt atgatggccg aacaatcatt caacgcgata atggttacca     840
   accaaactat cacgctgtca acattgttgg ttacagtaac gcacaaggtg tcgattattg     900
   gatcgtacga aacagttggg ataccaattg gggtgataat ggttacggtt attttgctgc     960
   caacatcgat ttgatgatga ttgaagaata tccatatgtt gtcattctct aaacaaaaag    1020
   acaatttctt atatgattgt cactaattta tttaaaatca aaatttttag aaaatgaata    1080
   aattcattca caaaaatta                                                 1099
<210> 20
   <211> 320
   <212> PRT
   <213> Dermatophagoides pteronyssinus
<400> 20
   Met Lys Ile Val Leu Ala Ile Ala Ser Leu Leu Ala Leu Ser Ala Val
   1               5                   10                  15
   Tyr Ala Arg Pro Ser Ser Ile Lys Thr Phe Glu Glu Tyr Lys Lys Ala
               20                  25                  30
   Phe Asn Lys Ser Tyr Ala Thr Phe Glu Asp Glu Glu Ala Ala Arg Lys
           35                  40                  45
   Asn Phe Leu Glu Ser Val Lys Tyr Val Gln Ser Asn Gly Gly Ala Ile
       50                  55                  60
   Asn His Leu Ser Asp Leu Ser Leu Asp Glu Phe Lys Asn Arg Phe Leu
   65                  70                  75                  80
   Met Ser Ala Glu Ala Phe Glu His Leu Lys Thr Gln Phe Asp Leu Asn
                   85                  90                  95
   Ala Glu Thr Asn Ala Cys Ser Ile Asn Gly Asn Ala Pro Ala Glu Ile
               100                 105                 110
   Asp Leu Arg Gln Met Arg Thr Val Thr Pro Ile Arg Met Gln Gly Gly
           115                 120                 125
   Cys Gly Ser Cys Trp Ala Phe Ser Gly Val Ala Ala Thr Glu Ser Ala
       130                 135                 140
   Tyr Leu Ala Tyr Arg Asn Gln Ser Leu Asp Leu Ala Glu Gln Glu Leu
   145                 150                 155                 160
   Val Asp Cys Ala Ser Gln His Gly Cys His Gly Asp Thr Ile Pro Arg
                   165                 170                 175
   Gly Ile Glu Tyr Ile Gln His Asn Gly Val Val Gln Glu Ser Tyr Tyr
               180                 185                 190
   Arg Tyr Val Ala Arg Glu Gln Ser Cys Arg Arg Pro Asn Ala Gln Arg
           195                 200                 205
   Phe Gly Ile Ser Asn Tyr Cys Gln Ile Tyr Pro Pro Asn Val Asn Lys
       210                 215                 220
   Ile Arg Glu Ala Leu Ala Gln Thr His Ser Ala Ile Ala Val Ile Ile
   225                 230                 235                 240
   Gly Ile Lys Asp Leu Asp Ala Phe Arg His Tyr Asp Gly Arg Thr Ile
                   245                 250                 255
   Ile Gln Arg Asp Asn Gly Tyr Gln Pro Asn Tyr His Ala Val Asn Ile
               260                 265                 270
   Val Gly Tyr Ser Asn Ala Gln Gly Val Asp Tyr Trp Ile Val Arg Asn
           275                 280                 285
   Ser Trp Asp Thr Asn Trp Gly Asp Asn Gly Tyr Gly Tyr Phe Ala Ala
       290                 295                 300
   Asn Ile Asp Leu Met Met Ile Glu Glu Tyr Pro Tyr Val Val Ile Leu
   305                 310                 315                 320